(19) Europäisches Patentamt
European Patent Office
Office européen des brevets

(11) **EP 3 646 782 A1**

(12) **EUROPEAN PATENT APPLICATION**

(43) Date of publication:
06.05.2020  Bulletin 2020/19

(51) Int Cl.:
**A61B 5/04** (2006.01)     **A61B 5/11** (2006.01)

(21) Application number: **19201512.1**

(22) Date of filing: **04.10.2019**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN**<br><br>(30) Priority: 02.11.2018  JP 2018207703<br>14.12.2018  JP 2018234815<br>14.12.2018  JP 2018234813<br><br>(71) Applicant: **Ricoh Company, Ltd.**<br>**Tokyo 143-8555 (JP)**<br><br>(72) Inventors:<br>• **ISHII, Toshihiro**<br>**Tokyo, 143-8555 (JP)**<br>• **OBA, Yoshihiro**<br>**Tokyo, 143-8555 (JP)** | • **OSAKA, Takanobu**<br>**Tokyo, 143-8555 (JP)**<br>• **MIFUNE, Hironobu**<br>**Tokyo, 143-8555 (JP)**<br>• **MITANI, Yuki**<br>**Tokyo, 143-8555 (JP)**<br>• **SHIBATA, Shinsuke**<br>**Tokyo, 143-8555 (JP)**<br>• **KUDO, Kiwamu**<br>**Tokyo, 143-8555 (JP)**<br>• **SATO, Shinji**<br>**Tokyo, 143-8555 (JP)**<br>• **KOU, Futoyoshi**<br>**Tokyo, 143-8555 (JP)**<br><br>(74) Representative: **White, Duncan Rohan**<br>**Marks & Clerk LLP**<br>**Fletcher House**<br>**Heatley Road**<br>**The Oxford Science Park**<br>**Oxford OX4 4GE (GB)** |

(54) **BIOMAGNETIC-FIELD MEASUREMENT APPARATUS, BIOMAGNETIC-FIELD MEASUREMENT METHOD, AND MAGNETIC SHIELD BOX**

(57)  A biomagnetic-field measurement apparatus (100), a biomagnetic-field measurement method, and a magnetic shield box (110). The biomagnetic-field measurement apparatus (100) measures a magnetic field of a site of a live subject, and the biomagnetic-field measurement apparatus includes sensor modules (510) disposed at a measurement position of the site. Each of the sensor modules (510) includes an optically pumped atomic magnetometer. The biomagnetic-field measurement method includes measuring magnetic field waveforms due to an eliciting stimulus multiple times by using the sensor modules (510), integrating the magnetic field waveforms measured multiple times with regard to the sensor modules (510), calculating a time gap T of the integrated magnetic field waveforms with regard to each adjacent pair of the sensor modules (510), measuring a spontaneous magnetic field waveform at a position identical to a position where the magnetic field waveform due to the eliciting stimulus is measured by using the sensor modules (510), and shifting the spontaneous magnetic field waveform measured by one of the adjacent pair of the sensor modules (510) on a time axis by the time gap T relative to the spontaneous magnetic field waveform measured by another one of the adjacent pair of the sensor modules (510) and combining the spontaneous magnetic field waveforms.

FIG. 4

**Description**

BACKGROUND

Technical Field

Embodiments of the present disclosure relate to a biomagnetic-field measurement apparatus, a biomagnetic-field measurement method, and a magnetic shield box.

Background Art

To diagnose amyotrophic lateral sclerosis (ALS) or muscular dystrophy, the needle electromyograph is used as a diagnostic device. However, with techniques using the needle electromyograph according to a related art, a needle electrode is inserted into a muscle of the subject, which causes pain to the subject. For this reason, consideration has been given to various techniques that use no needle.

One of the examples of the method that uses no needle is a method using a surface electrode. However, this method is not applicable because, generally, the spatial resolution is not desirable and the potentials from a plurality of motor units are measured. Another example of the method that uses no needle is the method for detecting a magnetic field of a live subject by using an optically pumped atomic magnetometer (OPM) (for example, see WO 2015/083242).

It is, however, considered that the limit of a high-speed response of the OPM is approximately 200 Hz as the response speed is determined by relaxation times T1 and T2 of an alkaline metal (Rb or Cs) that is sealed in an internal gas cell.

As the frequency of the magnetic field (muscle magnetic field) generated by a muscle is approximately 500 Hz, the detection of a muscle magnetic field waveform of approximately 10 msec is desirable. The response speed of the OPM is not sufficient to accurately detect a muscle magnetic field waveform of approximately 10 msec. Without improvements in the response speed, a difficulty arises in accurately measuring a multiphase waveform of the ALS or a spiky and small-amplitude waveform of the muscular dystrophy, and therefore an accurate diagnosis is not achieved.

Conventionally, it is difficult to detect a muscle magnetic field by using an optically pumped atomic magnetometer with sufficient accuracy.

SUMMARY

Embodiments of the present disclosure described herein provide a biomagnetic-field measurement apparatus, a biomagnetic-field measurement method, and a magnetic shield box. The biomagnetic-field measurement apparatus measures a magnetic field of a site of a live subject, and the biomagnetic-field measurement apparatus includes a plurality of sensor modules disposed at a measurement position of the site. Each of the sensor modules including an optically pumped atomic magnetometer. The biomagnetic-field measurement method includes measuring magnetic field waveforms due to an eliciting stimulus multiple times by using the sensor modules, integrating the magnetic field waveforms measured multiple times with regard to the sensor modules, calculating a time gap T of the integrated magnetic field waveforms with regard to each adjacent pair of the sensor modules, measuring a spontaneous magnetic field waveform at a position identical to a position where the magnetic field waveform due to the eliciting stimulus is measured by using the sensor modules, and shifting the spontaneous magnetic field waveform measured by one of the adjacent pair of the sensor modules on a time axis by the time gap T relative to the spontaneous magnetic field waveform measured by another one of the adjacent pair of the sensor modules and combining the spontaneous magnetic field waveforms. The magnetic shield box includes a hollow shielding member to shield an external magnetic field, the shielding member provided with an opening for allowing insertion of a site of a live subject for which a magnetic field is measured, a first magnetic sensor disposed at a measurement position of the site inside the shielding member, a second magnetic sensor disposed between the opening and the measurement position inside the shielding member, and a coil disposed between the opening and the second magnetic sensor inside the shielding member, the coil to be supplied with a current determined based on a value measured by the second magnetic sensor from outside the shielding member to reduce a magnetic field inside the shielding member.

According to the present disclosure, a biomagnetic-field measurement apparatus using an optically pumped atomic magnetometer may improve the accuracy at which a muscle magnetic field is detected.

BRIEF DESCRIPTION OF THE DRAWINGS

A more complete appreciation of embodiments and the many attendant advantages thereof will be readily obtained as the same becomes better understood by reference to the following detailed description when considered in connection with the accompanying drawings.

FIG. 1 is a diagram illustrating an external configuration of a biomagnetic-field measurement apparatus according to a first embodiment of a first group of embodiments of the present invention;
FIG. 2 is a first diagram illustrating the flow of an operation to diagnose a muscle magnetic field of a skeletal muscle according to the first embodiment of the first group of embodiments;
FIGS. 3A and 3B are second diagrams illustrating the flow of an operation to diagnose a muscle magnetic field of a skeletal muscle according to the first embodiment of the first group of embodiments of the present invention;

FIG. 4 is a diagram illustrating an example of the system configuration of the biomagnetic-field measurement apparatus illustrated in FIG. 1;

FIG. 5 is a diagram illustrating an internal configuration of a magnetic shield box according to the first embodiment of the first group of embodiments;

FIG. 6 is a diagram illustrating a situation where a palm is placed inside a shielding member illustrated in FIG. 5;

FIG. 7 is a first diagram illustrating a situation where an arm is inserted into the shielding member illustrated in FIG. 5;

FIG. 8 is a second diagram illustrating a situation where the arm is inserted into the shielding member illustrated in FIG. 5;

FIG. 9 is a third diagram illustrating a situation where the arm is inserted into the shielding member illustrated in FIG. 5;

FIG. 10 is a diagram illustrating a detailed configuration of a holder illustrated in FIG. 5;

FIG. 11 is a diagram illustrating a schematic configuration of an optically pumped atomic magnetometer according to the first embodiment of the first group of embodiments;

FIG. 12 is a diagram illustrating a potential propagation direction of a muscle fiber and a light propagation direction of the sensor module according to the first embodiment of the first group of embodiments;

FIG. 13 is a diagram illustrating an example of the arrangement of sensor modules in the holder illustrated in FIG. 5;

FIG. 14 is a graph illustrating an example of magnetic field shield characteristics of a flexible film illustrated in FIG. 5;

FIGS. 15A and 15B are first diagrams illustrating a function of an opening/closing mechanism illustrated in FIG. 5;

FIGS. 16A and 16B are second diagrams illustrating a function of the opening/closing mechanism illustrated in FIG. 5;

FIG. 17 is a table illustrating types of muscles in humans according to the first embodiment of the first group of embodiments;

FIG. 18 is a diagram that schematically illustrates an abductor pollicis brevis that is an example of a skeletal muscle according to the first embodiment of the first group of embodiments;

FIG. 19 is a first table illustrating waveforms (comparative example) of needle electromyography according to the first embodiment of the first group of embodiments;

FIG. 20 is a second table illustrating waveforms (comparative example) of needle electromyography according to the first embodiment of the first group of embodiments;

FIGS. 21A to 21D are diagrams for examining the response speed of a typical sensor module according to the first embodiment of the first group of embodiments;

FIG. 22 is a first graph illustrating an improvement in the accuracy of sensor modules illustrated in FIG. 13;

FIG. 23 is a second graph illustrating an improvement in the accuracy of the sensor modules illustrated in FIG. 13;

FIG. 24 is a third graph illustrating an improvement in the accuracy of the sensor modules illustrated in FIG. 13;

FIG. 25 is a fourth graph illustrating an improvement in the accuracy of the sensor modules illustrated in FIG. 13;

FIG. 26 is a fifth graph illustrating an improvement in the accuracy of the sensor modules illustrated in FIG. 13;

FIG. 27 is a first diagram illustrating another example of the arrangement of the two sensor modules illustrated in FIG. 13;

FIG. 28 is a second diagram illustrating another example of the arrangement of the two sensor modules illustrated in FIG. 13;

FIG. 29 is a diagram illustrating an example of the arrangement of the three sensor modules illustrated in FIG. 13;

FIG. 30 is a diagram illustrating a specific method for quantifying a depth direction according to the first embodiment of the first group of embodiments;

FIG. 31 is a diagram illustrating an example of the hardware configuration of an information processing device illustrated in FIG. 1;

FIG. 32 is a diagram illustrating details of a functional configuration of the information processing device illustrated in FIG. 1;

FIG. 33 is a diagram illustrating the positional relationship between the position where electrical stimulation is applied and the position where a magnetic field is detected according to the first embodiment of the first group of embodiments;

FIG. 34 is a diagram illustrating the flow of an operation to make a diagnosis based on the muscle magnetic field of a skeletal muscle according to the first embodiment of the first group of embodiments;

FIG. 35 is a flowchart illustrating the flow of positional alignment according to the first embodiment of the first group of embodiments;

FIGS. 36A to 36C are diagrams illustrating the positional alignment according to the first embodiment of the first group of embodiments;

FIG. 37 is a flowchart illustrating the flow of a magnetic-field detection process according to the first embodiment of the first group of embodiments;

FIG. 38 is a flowchart illustrating an example of the flow of data analysis according to the first embodiment of the first group of embodiments;

FIGS. 39A and 39B are first graphs illustrating pattern recognition of a waveform according to the first embodiment of the first group of embodiments;

FIGS. 40A and 40B are second graphs illustrating the pattern recognition of a waveform according to the first embodiment of the first group of embodiments;

FIGS. 41A and 41B are third graphs illustrating the pattern recognition of a waveform according to the first embodiment of the first group of embodiments;

FIG. 42 is a fourth table illustrating the pattern recognition of a waveform according to the first embodiment of the first group of embodiments;

FIG. 43 is a diagram illustrating an internal configuration of a magnetic shield box according to a second embodiment of the first group of embodiments;

FIG. 44 is a diagram illustrating a detailed configuration of the holder illustrated in FIG. 43;

FIG. 45 is a diagram illustrating an example of the arrangement of sensor modules in the holder illustrated in FIG. 44;

FIG. 46 is a graph illustrating an example of crosstalk according to the second embodiment of the first group of embodiments;

FIG. 47 is a diagram illustrating an internal configuration of a magnetic shield box according to a modification of the second embodiment of the first group of embodiments;

FIGS. 48A and 48B are diagrams illustrating an auxiliary member according to a third embodiment of the first group of embodiments;

FIGS. 49A and 49B are diagrams illustrating a magnetic shield box according to a fourth embodiment of the first group of embodiments;

FIG. 50 is a first diagram illustrating an external configuration of a biomagnetic-field detection apparatus according to a first embodiment of a second group of embodiments of the present invention;

FIG. 51 is a first diagram illustrating the flow of an operation to diagnose carpal tunnel syndrome according to the first embodiment of the second group of embodiments;

FIGS. 52A and 52B are second diagrams illustrating the flow of an operation to diagnose carpal tunnel syndrome according to the first embodiment of the second group of embodiments;

FIG. 53 is a third diagram illustrating the flow of an operation to diagnose carpal tunnel syndrome according to the first embodiment of the second group of embodiments;

FIG. 54 is a diagram illustrating an example of the system configuration of a biomagnetic-field detection apparatus illustrated in FIG. 50;

FIG. 55 is a first diagram illustrating an internal configuration of a measuring unit and a drive illustrated in FIG. 50;

FIG. 56 is a diagram illustrating a situation where an object under examination is placed in the measuring unit illustrated in FIG. 50;

FIG. 57 is a first diagram illustrating a detailed configuration of the holder illustrated in FIG. 55;

FIGS. 58A and 58B are second diagrams illustrating a function of the opening/closing mechanism illustrated in FIG. 55;

FIG. 59 is a diagram illustrating an example of capturing with the camera illustrated in FIG. 54;

FIG. 60 is a diagram illustrating an example of the hardware configuration of an information processing device illustrated in FIG. 50;

FIG. 61 is a diagram illustrating an example of the functional configuration of the information processing device illustrated in FIG. 50;

FIG. 62 is a diagram illustrating a specific example of ultrasonic data according to the first embodiment of the second group of embodiments;

FIG. 63 is a diagram illustrating the positional relationship between the position where the electrical stimulation is applied and the position where the magnetic field is detected according to the first embodiment of the second group of embodiments;

FIG. 64 is a diagram illustrating the flow of an operation to diagnose carpal tunnel syndrome according to the first embodiment of the second group of embodiments;

FIG. 65 is a flowchart illustrating the flow of a magnetic-field detection process according to the first embodiment of the second group of embodiments;

FIGS. 66A, 66B, and 66C are graphs illustrating specific examples of magnetic-field data according to the first embodiment of the second group of embodiments;

FIG. 67 is a flowchart illustrating the flow of a current-distribution calculation process according to the first embodiment of the second group of embodiments;

FIGS. 68A and 68B are first diagrams illustrating examples of the display of current distribution according to the first embodiment of the second group of embodiments;

FIG. 69 is a second diagram illustrating internal configurations of the measuring unit and the drive according to a second embodiment of the second group of embodiments;

FIG. 70 is a second diagram illustrating a detailed configuration of a holder illustrated in FIG. 69;

FIG. 71 is a diagram illustrating an example of the arrangement of sensor modules in the holder illustrated in FIG. 69;

FIGS. 72A and 72B are diagrams illustrating an auxiliary member according to a third embodiment of the second group of embodiments;

FIG. 73 is a second diagram illustrating an external configuration of a biomagnetic-field detection apparatus according to a fourth embodiment of the second group of embodiments;

FIG. 74 is a diagram illustrating part of the flow of an operation to diagnose the neurological disorder of a leg according to the fourth embodiment of the second group of embodiments;

FIG. 75 is a second diagram illustrating a display example of a current distribution according to the fourth embodiment of the second group of embodiments;

FIG. 76 is a diagram illustrating a part of the flow of an operation to diagnose the neurological disorder of an elbow according to a fifth embodiment of the second group of embodiments;

FIG. 77 is a third diagram illustrating an internal configuration of a measuring unit and a drive illustrated in FIG. 76;

FIG. 78 is a third diagram illustrating a detailed structure of a holder illustrated in FIG. 77;

FIG. 79 is a diagram illustrating an external configuration of a biomagnetic-field measurement apparatus according to a first embodiment of a third group of embodiments of the present invention;

FIG. 80 is a diagram illustrating a system configuration of the biomagnetic-field measurement apparatus illustrated in FIG. 79;

FIG. 81 is a diagram illustrating an internal configuration of a magnetic shield box according to the first embodiment of the third group of embodiments;

FIGS. 82A and 82B are first diagrams illustrating a gradient magnetic field according to the first embodiment of the third group of embodiments;

FIG. 83 is a second graph illustrating a gradient magnetic field according to the first embodiment of the third group of embodiments;

FIGS. 84A and 84B are third diagrams illustrating a gradient magnetic field according to the first embodiment of the third group of embodiments;

FIG. 85 is a fourth graph illustrating a gradient magnetic field according to the first embodiment of the third group of embodiments;

FIG. 86 is a first diagram illustrating a situation where the arm is inserted into the shielding member illustrated in FIG. 81;

FIG. 87 is a second diagram illustrating a situation where the arm is inserted into the shielding member illustrated in FIG. 81;

FIG. 88 is a third diagram illustrating a situation where the arm is inserted into the shielding member illustrated in FIG. 81;

FIG. 89 is a diagram illustrating details of a functional configuration of the information processing device illustrated in FIG. 79;

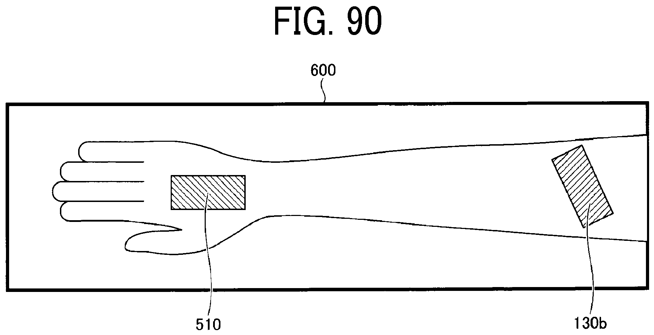

FIG. 90 is a diagram illustrating the positional relationship between the position where electrical stimulation is applied and the position where a magnetic field is detected;

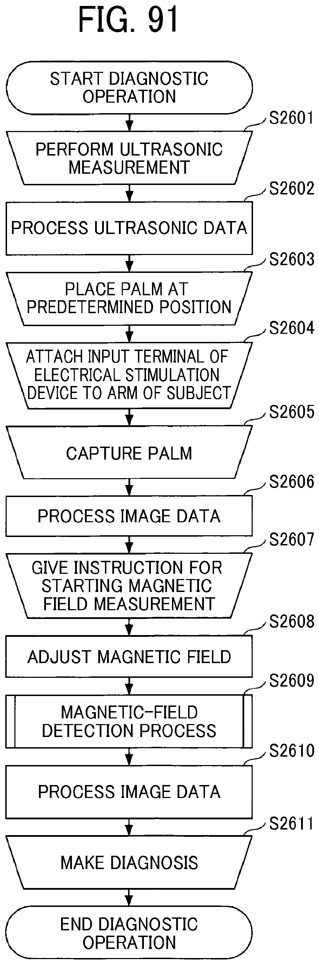

FIG. 91 is a diagram illustrating the flow of an operation to make a diagnosis based on a muscle magnetic field of a skeletal muscle;

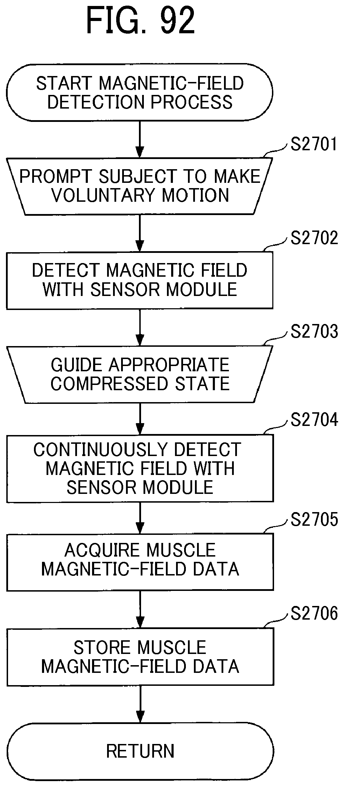

FIG. 92 is a flowchart illustrating the flow of a magnetic-field detection process;

FIG. 93 is a flowchart illustrating the flow of data analysis;

FIG. 94 is a perspective view illustrating a magnetic shield box according to a second embodiment of the third group of embodiments;

FIGS. 95A and 95B are diagrams illustrating a diameter of an insertion hole of a chimney structure according to the second embodiment of the third group of embodiments;

FIG. 96 is a diagram illustrating the chimney structure before processing according to the second embodiment of the third group of embodiments;

FIG. 97 is a first diagram illustrating another example of the chimney structure according to the second embodiment of the third group of embodiments;

FIG. 98 is a second diagram illustrating another example of the chimney structure according to the second embodiment of the third group of embodiments;

FIG. 99 illustrates a first variation of the shape of the shielding member according to a third embodiment of the third group of embodiments;

FIG. 100 illustrates a second variation of the shape of the shielding member according to the third embodiment of the third group of embodiments;

FIG. 101 illustrates a third variation of the shape of the shielding member according to the third embodiment of the third group of embodiments;

FIG. 102 illustrates a fourth variation of the shape of the shielding member according to the third embodiment of the third group of embodiments;

FIG. 103 is a diagram illustrating a structure with which a shielding member and an auxiliary shielding member, illustrated in FIG. 102, are attached to or detached from each other;

FIG. 104 illustrates a fifth variation of the shape of the shielding member according to the third embodiment of the third group of embodiments;

FIG. 105 is a schematic diagram illustrating an example of the internal configuration of a magnetic shield box according to a fourth embodiment of the third group of embodiments;

FIG. 106 is a schematic diagram illustrating another example of the internal configuration of a magnetic shield box according to the fourth embodiment of the third group of embodiments;

FIGS. 107A and 107B are diagrams illustrating a magnetic shield box according to a sixth embodiment of the third group of embodiments;

FIGS. 108A and 108B are diagrams illustrating a magnetic shield box according to a seventh embodiment of the third group of embodiments;

FIG. 109 is a diagram illustrating an internal configuration of a magnetic shield box according to an eighth embodiment of the third group of embodiments; and

FIGS. 110A and 110B are diagrams illustrating the coupling between a magnetic shield box and a module support device according to the eighth embodiment of the third group of embodiments.

The accompanying drawings are intended to depict embodiments of the present disclosure and should not be interpreted to limit the scope thereof. The accompanying drawings are not to be considered as drawn to scale unless explicitly noted.

DETAILED DESCRIPTION

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "includes" and/or "including", when used in this specification, specify the presence of stated features, integers, steps, operations, elements, and/or components, but do not preclude the presence or addition of one or more other features, integers, steps, operations, elements, components, and/or groups thereof.

In describing example embodiments shown in the drawings, specific terminology is employed for the sake of clarity. However, the present disclosure is not intended to be limited to the specific terminology so selected and it is to be understood that each specific element includes all technical equivalents that have the same structure, operate in a similar manner, and achieve a similar result.

Referring to the drawings, embodiments for carrying out the present invention are described. In each drawing, the same components are denoted by the same reference numeral, and duplicate descriptions may be omitted.

FIRST GROUP OF EMBODIMENTS

FIRST EMBODIMENT OF FIRST GROUP OF EMBODIMENTS

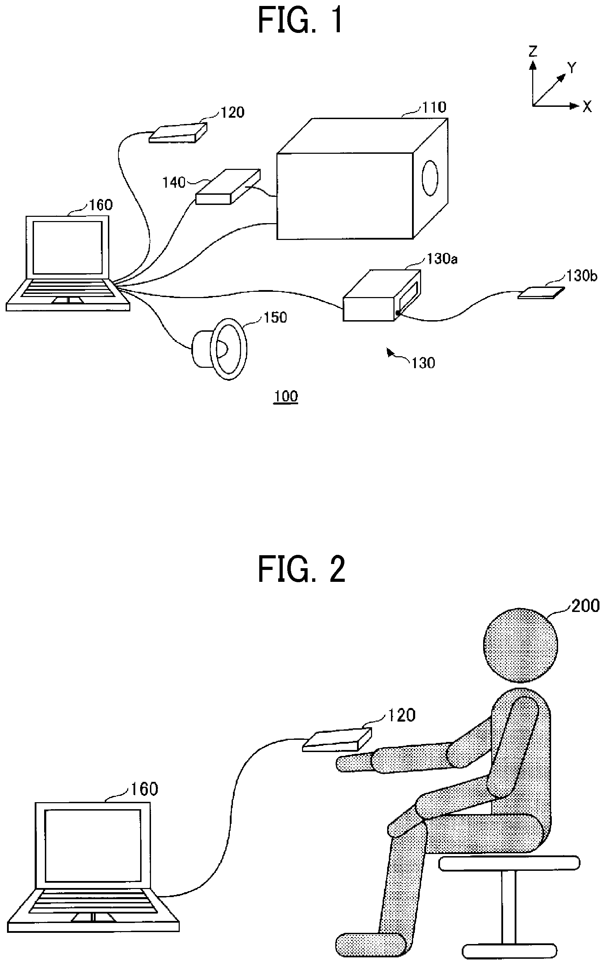

First, an external configuration of a biomagnetic-field measurement apparatus according to a first embodiment of a first group of embodiments of the present invention is described. FIG. 1 is a diagram illustrating an external configuration of a biomagnetic-field measurement apparatus 100. As illustrated in FIG. 1, the biomagnetic-field measurement apparatus 100 includes a magnetic shield box 110, an ultrasonic measuring device 120, an electrical stimulation device 130 including a generator 130a and an input terminal 130b, a current generating device 140, a sound generating device 150, and an information processing device 160. The ultrasonic measuring device 120, the electrical stimulation device 130, and the sound generating device 150 may be omitted from or included in the biomagnetic-field measurement apparatus 100 as appropriate.

A description is primarily provided below of an example in which the measurement target of the biomagnetic-field measurement apparatus 100 is a "skeletal muscle of a hand," which is a part of a limb of the subject, more specifically, "abductor pollicis brevis of the hand." However, this is merely an example, and the measurement target of the biomagnetic-field measurement apparatus 100 is not limited to the "skeletal muscle of the hand." The measurement target of the biomagnetic-field measurement apparatus 100 may be a leg, head, etc.

The magnetic shield box 110 is a device that detects a magnetic field generated in the subject. The magnetic shield box 110 detects the magnetic field generated in the hand in a state where the subject's arm is inserted and the hand is placed at a predetermined position. The magnetic shield box 110 transmits magnetic-field data (data group on the magnetic flux density at each time) to the information processing device 160.

According to the first embodiment of the first group of embodiments, the longitudinal direction of the magnetic shield box 110 illustrated in FIG. 1 is the X-axis direction, the depth direction thereof is the Y-axis direction, and the height direction thereof is the Z-axis direction.

The ultrasonic measuring device 120 is a device that transmits and receives ultrasonic waves to measure the thickness of the fat of the hand, etc. The ultrasonic measuring device 120 transmits the measured ultrasonic data to the information processing device 160.

The measurement by the ultrasonic measuring device 120 is used to, for example, quantify the thickness of the fat on the surface layer of the abductor pollicis brevis of the hand. The ultrasonic measuring device 120 may measure the position and the depth of the fat in three dimensions. As the thickness of the fat may be estimated from the physical size, the weight, or the like, of the subject, the measurement by the ultrasonic measuring device 120 may be conducted as appropriate.

The electrical stimulation device 130 is a device that applies electrical stimulation to a predetermined site of the subject in a state where the subject's arm is inserted into the magnetic shield box 110 and the hand is placed at a predetermined position. As the electrical stimulation device 130, for example, "electromyography/evoked potential testing device MEB-9400 series Neuropack S1" manufactured by Nihon Kohden may be used.

The generator 130a of the electrical stimulation device 130 generates a voltage to be applied to an electrode of the input terminal 130b in response to an instruction from the information processing device 160. The input terminal 130b of the electrical stimulation device 130 is attached to a predetermined site of the subject to apply the voltage to the site so as to give electrical stimulation.

The information processing device 160 may process ultrasonic data transmitted from the ultrasonic measuring device 120 and calculate the thickness of the fat of a predetermined part of the hand. The information processing device 160 may also transmit an instruction to the electrical stimulation device 130 at predetermined timing to drive the electrical stimulation device 130.

The information processing device 160 transmits an instruction for driving each unit of the magnetic shield box 110 to the magnetic shield box 110. The information processing device 160 receives magnetic-field data transmitted from the magnetic shield box 110. The information processing device 160 calculates the muscle magnetic field of the skeletal muscle of the hand by using the data on the fat of the hand and the received magnetic-field data and then displays the waveform of the muscle magnetic field and numerical data calculated from the waveform.

The biomagnetic-field measurement apparatus 100 may calculate the spontaneous muscle magnetic field of the subject's skeletal muscle and display the waveform of the muscle magnetic field and the numerical data calculated from the waveform. The biomagnetic-field measurement apparatus 100 may calculate the muscle magnetic field (evoked muscle magnetic field), which is evoked when an electrical stimulus is given to the subject, of the subject's skeletal muscle and display the waveform of the muscle magnetic field and the numerical data calculated from the waveform. Thus, with the use of the biomagnetic-field measurement apparatus 100, the doctor, or the like, may properly diagnose ALS and muscular dystrophy.

For example, the ALS tends to show the sign of disability in a muscle of the hand. Therefore, with the use of the biomagnetic-field measurement apparatus 100, the doctor, or the like, detects the spontaneous muscle magnetic field or the evoked muscle magnetic field of, for example, the "abductor pollicis brevis" of the hand so as to estimate the degree of progress of the ALS.

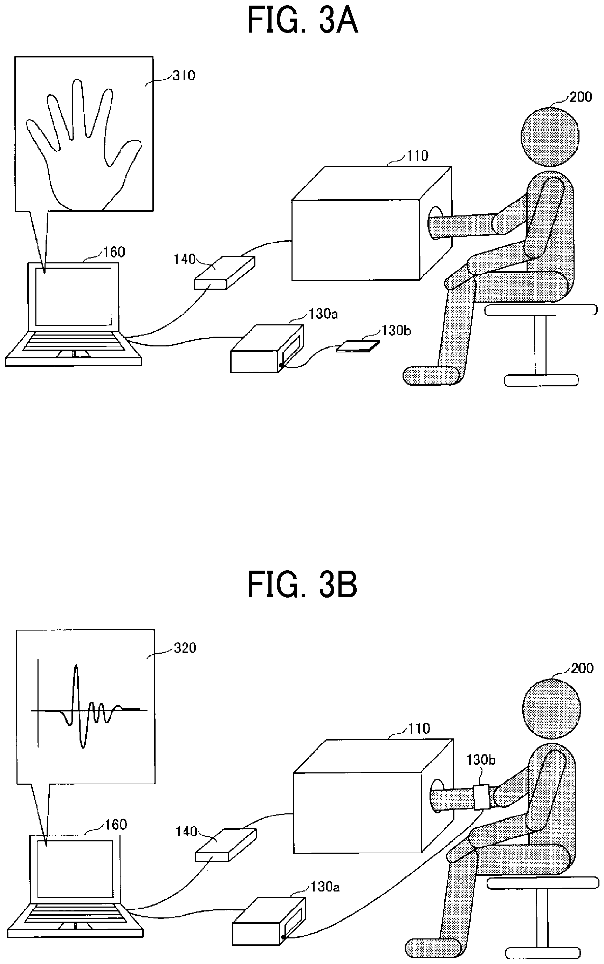

Next, the flow of an operation to diagnose the muscle magnetic field of the skeletal muscle of the hand using the biomagnetic-field measurement apparatus 100 is described. FIGS. 2 and 3 are diagrams illustrating the flow of an operation to diagnose the muscle magnetic field of the skeletal muscle. In the example illustrated according to the present embodiment, the magnetic field of the abductor pollicis brevis is detected.

FIG. 2 illustrates a situation where the doctor, or the like, measures the thickness of the fat of the hand of a subject 200 using the ultrasonic measuring device 120. As illustrated in FIG. 2, the hand of the subject 200 is measured by using the ultrasonic measuring device 120 so that the information processing device 160 calculates the thickness of the fat of the part of the abductor pollicis brevis of the hand of the subject 200.

FIG. 3A illustrates a situation where the doctor, or the like, guides the subject 200 so as to insert the arm into the magnetic shield box 110 and place the palm at a predetermined position within the magnetic shield box 110.

Furthermore, FIG. 3A illustrates a situation where the doctor, or the like, operates the information processing device 160 so that a camera provided in the magnetic shield box 110 captures the palm. As illustrated in FIG. 3A, after the palm of the subject 200 is captured, the information processing device 160 displays image data 310.

FIG. 3B illustrates a situation where the doctor, or the like, attaches the input terminal 130b to the elbow (a site different from the palm that is the object under examination) of the subject 200. When the doctor, or the like, operates the information processing device 160 in a state where the input terminal 130b is attached, the generator 130a is driven to apply electrical stimulation to the subject 200. The magnetic shield box 110 detects the evoked muscle magnetic field generated in the abductor pollicis brevis of the hand and transmits the magnetic-field data to the information processing device 160. The stimulation to the median nerve of the elbow allows the abductor pollicis brevis to be efficiently evoked.

To detect the spontaneous muscle magnetic field, the doctor or the like prompts the subject 200 to perform a weak compression action. In this case, as no electrical stimulation is applied, the input terminal 130b is not attached in FIG. 3B. The magnetic shield box 110 detects the spontaneous muscle magnetic field generated in the abductor pollicis brevis of the hand in response to a weak compression action and transmits the magnetic-field data to the information processing device 160.

In a case where the biomagnetic-field measurement apparatus 100 exclusively detects the spontaneous muscle magnetic field, the biomagnetic-field measurement apparatus 100 may omit the electrical stimulation device 130.

In the case of both the detection of the evoked muscle magnetic field and the detection of the spontaneous muscle magnetic field, the information processing device 160 uses the thickness of the fat of the hand and the magnetic-field data to generate waveform data 320 on the muscle magnetic field of the abductor pollicis brevis of the hand and displays the waveform data 320 on the information processing device 160. The information processing device 160 may display the numerical data calculated from the waveform data 320 on the muscle magnetic field together with the waveform data 320 or in place of the waveform data 320.

The detection of the muscle magnetic field by the magnetic shield box 110 and the generation and the display of the waveform data 320, or the like, by the information processing device 160 continue for a predetermined time period. The doctor or the like monitors the waveform data 320 or the numerical data calculated from the waveform data 320 so as to properly diagnose ALS or muscular dystrophy of the subject 200.

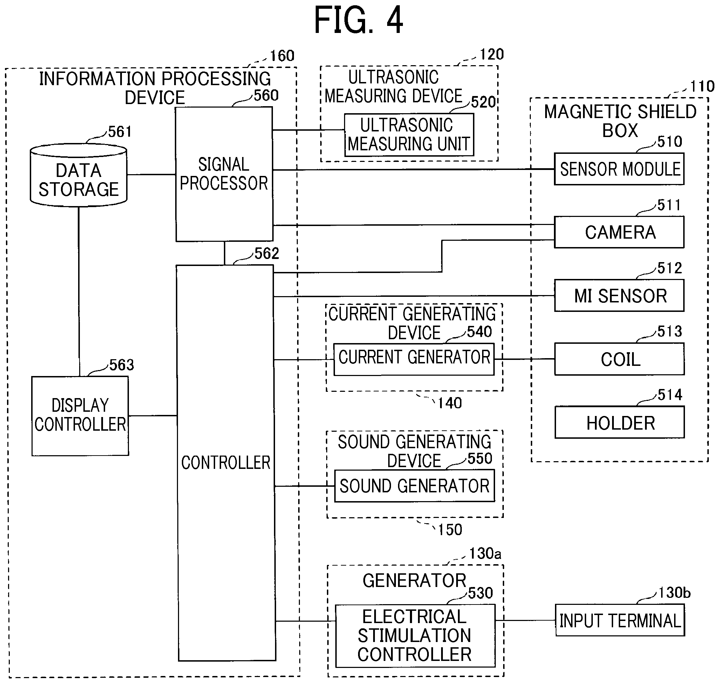

Next, a system configuration of the biomagnetic-field measurement apparatus 100 is described. FIG. 4 is a diagram illustrating an example of the system configuration of the biomagnetic-field measurement apparatus 100.

As illustrated in FIG. 4, in the biomagnetic-field measurement apparatus 100 according to the first embodiment of the first group of embodiments, the magnetic shield box 110 includes a sensor module 510, a camera 511, an MI sensor 512, a coil 513, and a holder 514.

The sensor module 510 has an optically pumped atomic magnetometer, which is a room-temperature magnetic sensor, and a position sensor built therein. The sensor module 510 detects the magnetic field (the muscle magnetic field of the abductor pollicis brevis) generated in the palm in a state where the end of the sensor module 510 is pressed against and is brought into contact with the abductor pollicis brevis of the hand placed at a predetermined position. The magnetic field measured by the sensor module 510 may be a spontaneous muscle magnetic field or an evoked biomagnetic field.

In the sensor module 510, the built-in position sensor detects the position of the sensor module 510 when a magnetic field is detected. The sensor module 510 transmits the detected magnetic-field data and the detected position data to a signal processor 560 of the information processing device 160.

Although FIG. 4 illustrates the single sensor module 510, a plurality of sensor modules are arranged in the Y-axis direction.

The camera 511 captures the palm placed at a predetermined position and transmits the image data to the signal processor 560 of the information processing device 160.

The MI sensor 512 is a solid-state magnetic sensor using a magneto-impedance element to measure a magnetic field in the magnetic shield box 110. The MI sensor 512 has a sensitivity of sub nT, a response speed of 1 kHz or more, and a size of approximately several centimeters. As the MI sensor 512, for example, MI-CB-1DH (Aich Micro Intelligent Corporation) may be used. The MI sensor 512 transmits the measured internal magnetic-field data to a controller 562 of the information processing device 160.

The coil 513 is supplied with the current determined by a current generator 540 of the current generating device 140 based on the measured value of the MI sensor 512. In other words, the current generator 540 acquires the current value calculated by the controller 562 of the information processing device 160 based on the internal magnetic-field data measured by the MI sensor 512 and controls the current flowing through the coil 513 based on the acquired current value. Accordingly, the magnetic field inside the magnetic shield box 110 is reduced so that a magnetic field allowing the detection of a muscle magnetic field may be formed inside the magnetic shield box 110.

The holder 514 is a member that holds the sensor module 510. The holder 514 holds the sensor module 510 such that the end of the sensor module 510 is pressed against and is brought into contact with the palm placed at a predetermined position.

The holder 514 is attached so as to be movable in a predetermined direction. This makes it possible to easily align the sensor module 510 and the measurement site of the subject 200 at appropriate positions.

As illustrated in FIG. 4, the ultrasonic measuring device 120 includes an ultrasonic measuring unit 520. The ultrasonic measuring unit 520 starts the measurement in response to an input instruction for starting ultrasonic measurement from the doctor, or the like, and transmits ultrasonic data to the signal processor 560 of the information processing device 160.

As illustrated in FIG. 4, the generator 130a includes an electrical stimulation controller 530. The electrical stimulation controller 530 generates the voltage to be applied to an electrode provided in the input terminal 130b in response to an instruction from the controller 562 of the information processing device 160.

As illustrated in FIG. 4, the current generating device 140 includes the current generator 540. The current generator 540 acquires the current value calculated by the controller 562 of the information processing device 160 based on the internal magnetic-field data measured by the MI sensor 512 and controls the current flowing through the coil 513 based on the acquired current value.

As illustrated in FIG. 4, the sound generating device 150 includes a sound generator 550. The sound generator 550 generates sound in response to an instruction from the controller 562 of the information processing device 160.

As illustrated in FIG. 4, the information processing device 160 includes the signal processor 560, a data storage 561, the controller 562, and a display controller 563.

The signal processor 560 calculates the thickness of the fat of the hand of the subject 200 based on the ultrasonic data transmitted from the ultrasonic measuring unit 520 and stores the thickness of the fat in the data storage 561. The signal processor 560 stores the image data transmitted from the camera 511 in the data storage 561. The signal processor 560 uses the thickness of the fat of the hand stored in the data storage 561 and the magnetic-field data and the position data transmitted from the sensor module 510 to generate the waveform data on the muscle magnetic field of the abductor pollicis brevis of the hand and stores the waveform data in the data storage 561.

The controller 562 receives a capturing instruction input by the doctor, or the like, via the display controller 563 and transmits an instruction to the camera 511. The controller 562 calculates the current value based on the internal magnetic field data measured by the MI sensor 512 and transmits the current value to the current generator 540. The controller 562 receives an instruction for starting the magnetic field measurement input by the doctor, or the like, via the display controller 563 and transmits an instruction to the electrical stimulation controller 530 and the current generator 540.

The display controller 563 notifies the controller 562 of an instruction for starting the magnetic field measurement input by the doctor or the like. The display controller 563 displays the waveform data stored in the data storage 561 in response to the notification of the instruction for starting the magnetic field measurement.

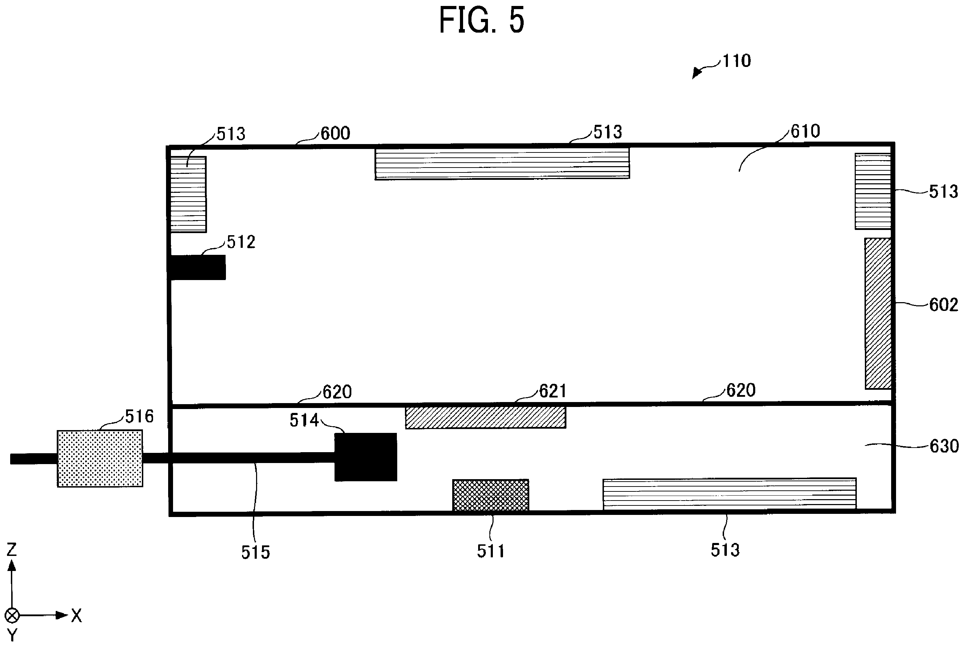

Next, an internal configuration of the magnetic shield box 110 is described. FIG. 5 is a diagram illustrating the internal configuration of the magnetic shield box 110 according to the first embodiment of the first group of embodiments. As illustrated in FIG. 5, the magnetic shield box 110 is covered with a hollow shielding member 600 that shields the external magnetic field.

The shielding member 600 may be formed by, for example, bending a permalloy flat plate (a thickness of approximately 2 mm). In order to reduce the residual magnetic field inside the shielding member 600 to such a degree that the muscle magnetic field is measurable, it is preferable that the permalloy used for the shielding member 600 has a multi-layer structure (e.g., a three-layer structure). The residual magnetic field inside the shielding member 600 may be 50 [nT] or less.

For the shielding member 600, permalloy plates may be bonded by, for example, welding so that a magnetic field is prevented from leaking, except for the holes for passing the wires of the sensor module 510 and the MI sensor 512 and an opening 601 described later.

The internal space of the magnetic shield box 110 is divided into a first space 610 in which the palm of the inserted arm is placed and a second space 630 in which the holder 514 holding the sensor module 510 is provided.

A boundary member 620 is provided between the first space 610 and the second space 630. The boundary member 620 includes, for example, permalloy.

The boundary member 620 is a support member that supports the live subject inserted through the opening 601 and is provided between the opening 601 and at least the measurement position. As the boundary member 620, which is a support member, is located close to the site where the magnetic field is generated, the boundary member 620 may absorb the noise magnetic field (artifact) effectively due to the direct contact with the live subject. Providing the boundary member 620 allows the removal of the electromagnetic field reflected and diffused in the second space 630 and allows a reduction in the noise in the first space 610.

As illustrated in FIG. 5, the coils 513 are provided in the first space 610. The MI sensor 512 is disposed in the first space 610. The opening 601, through which the subject 200 inserts the arm into the first space 610, is provided in the first space 610, and an opening/closing mechanism 602 is disposed around the opening 601.

The camera 511, the coil 513, and the holder 514 are provided in the second space 630. The holder 514 including the sensor module 510 is coupled to a moving mechanism 516 via a lever 515. The moving mechanism 516 is configured to move the holder 514 in a tilt direction ($\theta$) due to the rotation of the lever 515 and in a front-back direction (the X-axis direction) due to the insertion and the removal of the lever 515.

The boundary member 620 includes, for example, permalloy. An opening is provided near the center position of the boundary member 620, and a flexible film 621 is secured to the opening. The opening to which the flexible film 621 is secured is in the position where the palm is placed in the first space 610, and the end of the sensor module 510 held by the holder 514 is in contact with the palm placed in the first space 610 via the flexible film 621.

The flexible film 621 is pressed by the sensor module 510 in the holder 514 and is therefore deformed along the surface shape of the object under examination. The flexible film 621 includes a Teflon (registered trademark) film, or the like, having high smoothness so that the end of the sensor module 510 may smoothly move in contact with the object under examination via the flexible film 621 in accordance with the movement of the holder 514 in the X-axis direction. Alternatively, the flexible film 621 may include such as a magnetic shield film in which the front and back surfaces of an amorphous metallic foil are processed with a PET film (a magnetic shield film having an amorphous metallic foil sandwiched therein). For example, a KOYO MS sheet manufactured by Koyo Sangyo Co., Ltd. may be used as the flexible film 621.

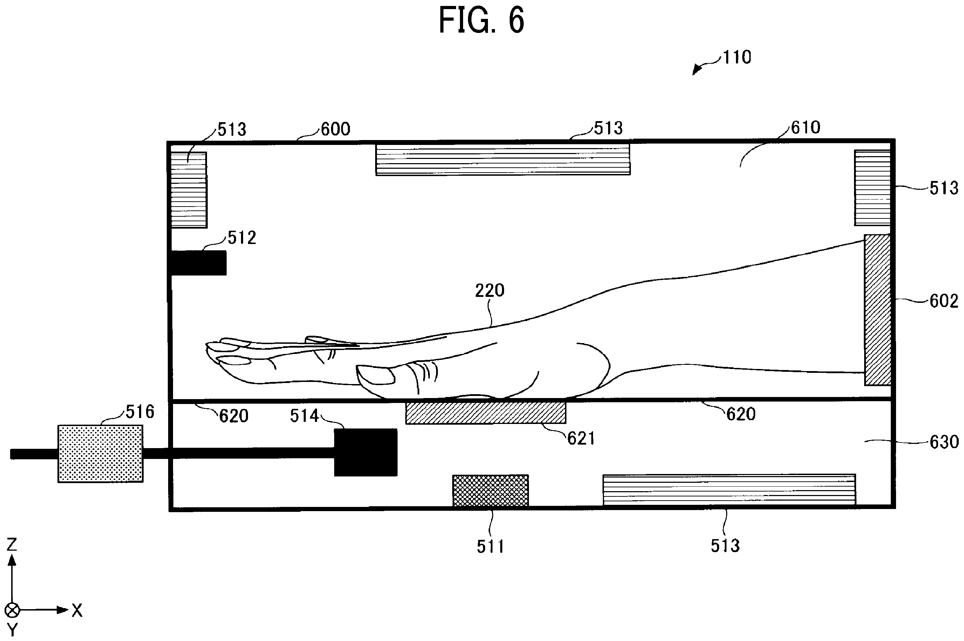

Next, the positional relationship with each unit in a case where the palm is placed inside the shielding member 600 is described. FIG. 6 is a diagram illustrating a situation where the palm is placed inside the shielding member. As illustrated in FIG. 6, the subject 200 inserts the arm through the opening 601 provided in the first space 610 to place a palm 220 at the position where the flexible film 621 is fixedly mounted.

As described later, as the opening/closing mechanism 602 provided around the opening 601 is closed in a state where the palm 220 is placed, the first space 610 is sealed. Thus, the shielding member 600 is configured such that the palm 220 of the subject 200 may be placed (may be held) inside the shielding member 600. The camera 511 may capture the palm 220 in a state where the palm 220 is placed. As the holder 514 is driven in the X-axis direction in a state where the palm 220 is placed, the sensor module 510 inside the holder 514 may detect the magnetic field generated in the palm 220 at each position of the palm 220 in the X-axis direction.

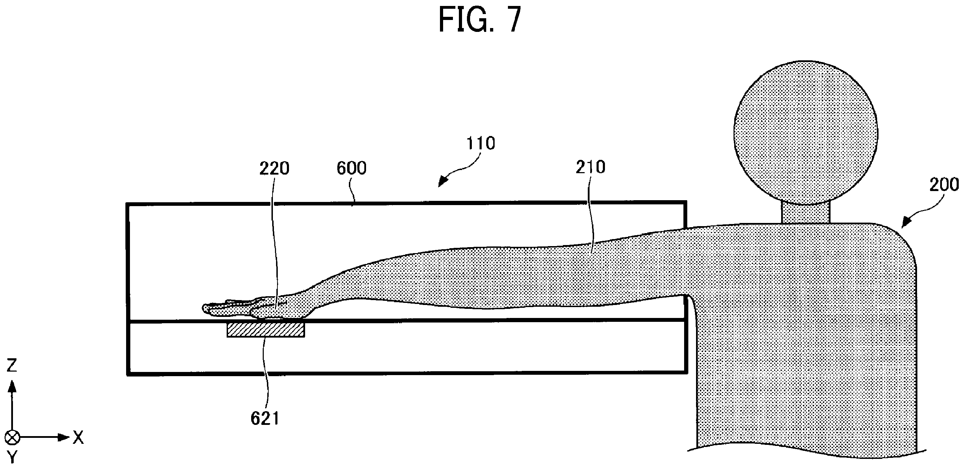

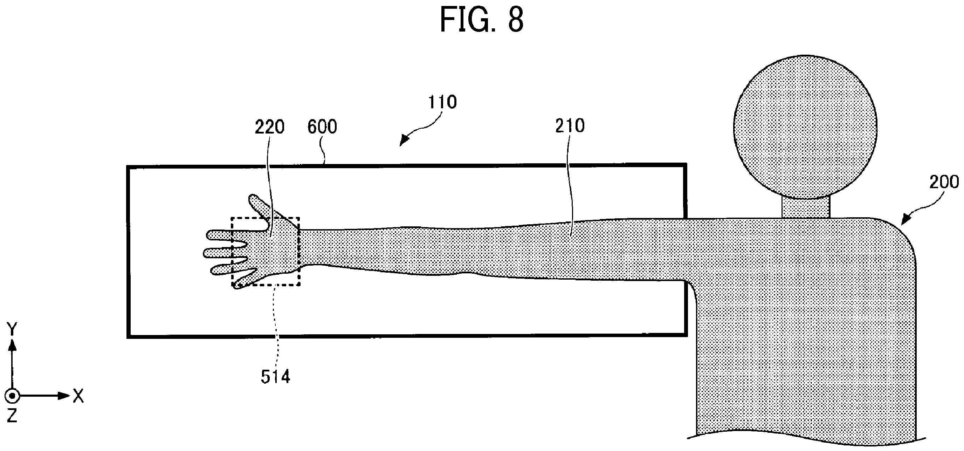

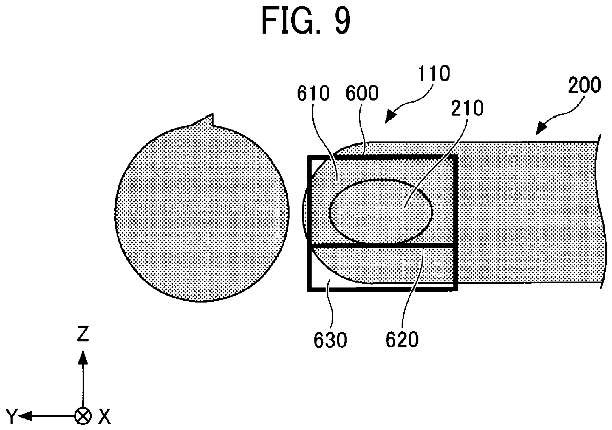

As illustrated in FIG. 7, the shielding member 600 may have an elongated shape into which an arm 210 of the subject 200 may be inserted. FIG. 7 illustrates an example in which the subject 200 inserts the arm 210 through the opening 601 of the shielding member 600 while the subject 200 is sitting; however, as illustrated in FIGS. 8 and 9, the subject 200 may insert the arm 210 through the opening 601 of the shielding member 600 while the subject 200 is lying on the back.

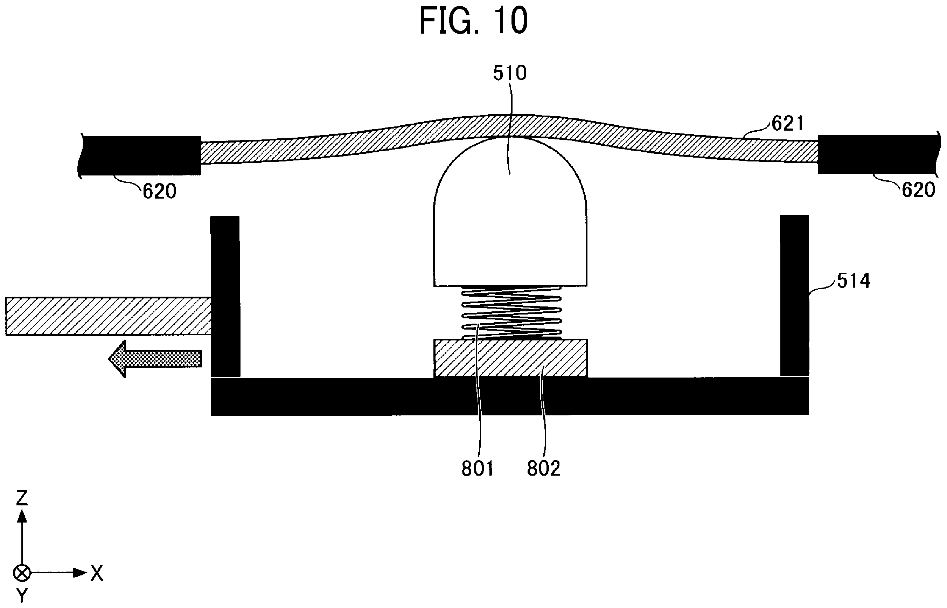

Next, a detailed configuration of the holder 514 is described. FIG. 10 is a diagram illustrating the detailed configuration of the holder 514. As illustrated in FIG. 10, in the holder 514, the sensor module 510 is supported by a support base 802 via an elastic member 801 (e.g., a spring). The support base 802 is secured to the holder 514.

In this manner, the sensor module 510 is supported via the elastic member 801 so that the end of the sensor module 510 may be pressed against and is brought into contact with the palm 220.

As described above, the sensor module 510 has the optically pumped atomic magnetometer and the position sensor built therein, and the sensor modules 510 are arranged in the Y-axis direction in the holder 514.

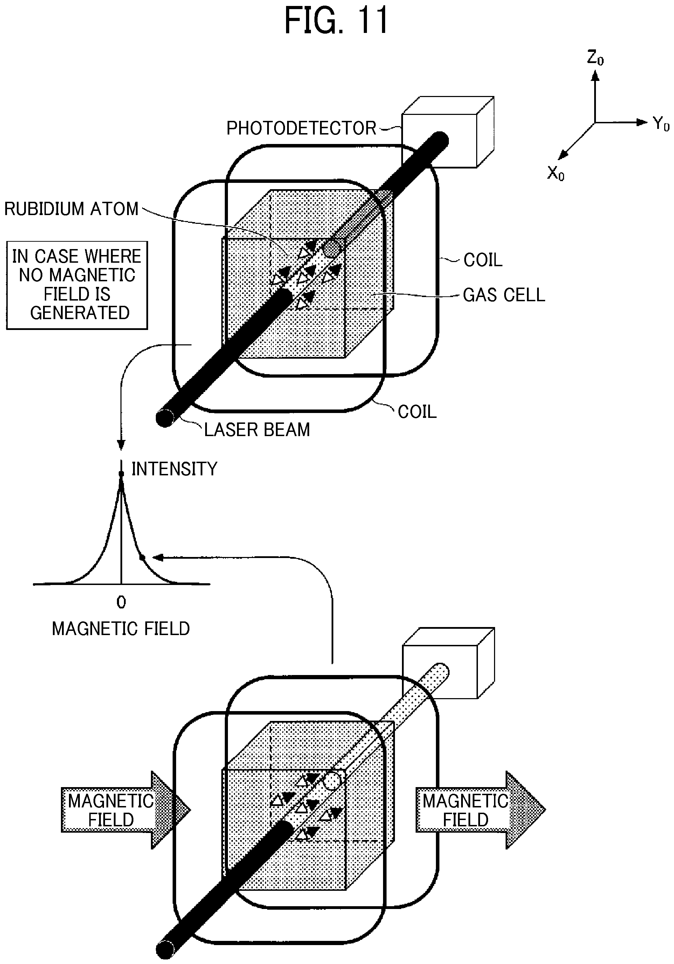

Next, a schematic configuration of the optically pumped atomic magnetometer included in the sensor module 510 is described. FIG. 11 is a diagram illustrating a schematic configuration of the optically pumped atomic magnetometer. As illustrated in FIG. 11, the optically pumped atomic magnetometer emits laser beam to a gas cell of rubidium atoms and detects the laser beam having passed through the gas cell by using a photodetector. The laser beam passing through the gas cell is absorbed depending on the magnitude of the magnetic field generated in a $Y_0$ axis direction or a $Z_0$ axis direction, and therefore the intensity of the laser beam detected by the photodetector decreases due to the magnetic field generated in the $Y_0$ axis direction or the $Z_0$ axis direction.

Thus, it is possible to calculate the magnitude of the magnetic field by comparing the intensity of the laser beam detected by the photodetector in a state where no magnetic field is generated and the intensity of the laser beam detected by the photodetector in a state where a magnetic field is generated. A coil is wound around the gas cell to apply an appropriate alternating current.

Thus, the optically pumped atomic magnetometer may detect a magnetic field in a direction substantially perpendicular to the incident direction (light propagation direction) of the laser beam. According to the present embodiment, the direction substantially parallel to the incident direction of the laser beam is an $X_0$ axis direction, and the directions substantially perpendicular to the incident direction of the laser beam are the $Y_0$ axis direction and the $Z_0$ axis direction.

For example, the gas cell is located away from the housing surface by approximately 6 mm, and the gas cell detects a magnetic field at this area. Hereinafter, the term "gas cell" refers to a detection position.

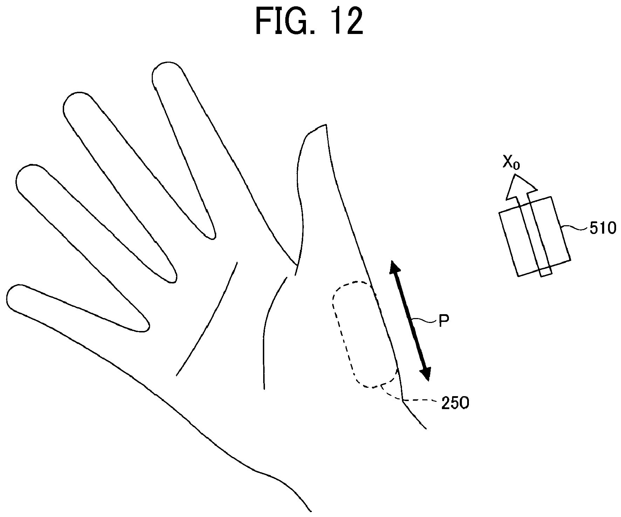

For example, the myoelectric potential propagates in the direction of the muscle fiber of the abductor pollicis brevis. The direction may be defined as a potential propagation direction. As illustrated in FIG. 12, a potential propagation direction P of a muscle fiber (the direction of a muscle fiber) of an abductor pollicis brevis 250 is substantially parallel to the light propagation direction (the $X_0$ axis direction) of the sensor module 510 so that a magnetic field may be measured with high accuracy.

As the muscle fiber direction of a muscle is substantially parallel to the light propagation direction of the sensor module 510, the depth of a firing muscle fiber may be quantified as described later. As the depth is determined, the relative distance between the gas cell of the sensor module 510 and the firing position (the depth from the skin surface) may be determined, and the amplitude (magnetic field intensity) may be corrected according to the Biot-Savart law. As the information on the amplitude is accurate due to this correction, "a high-amplitude potential (giant MUP)" in the ALS and a weak motor unit waveform in the muscular dystrophy may be determined.

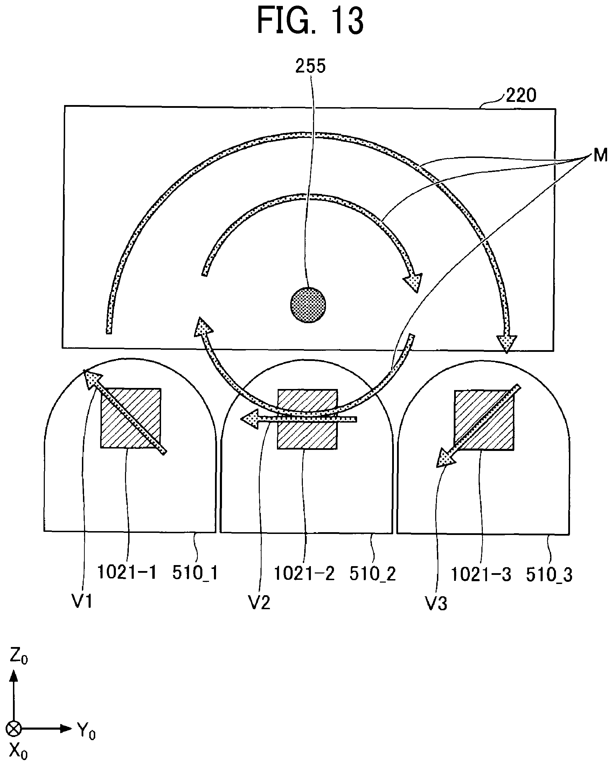

FIG. 13 is a diagram illustrating an example of the arrangement of sensor modules 510_1, 510 2, and 510_3 in the holder 514. In the example of FIG. 13, the sensor modules 510_1, 510_2, and 510_3 are arranged in parallel in the Yo axis direction. In FIG. 13, in a state where the palm 220 is placed at a predetermined position, a muscle fiber 255 of the palm 220 runs in the $X_0$ axis direction substantially perpendicular to the YZ plane.

The potential propagating in the muscle fiber direction (the $X_0$ axis direction) may be understood in the same manner as the current, and a magnetic field is generated in the direction of rotation. For example, when the current flows through the muscle fiber 255 from the front side of the drawing plane to the back side thereof, a magnetic field is generated on the YZ plane in the direction of an arrow M. As a result, magnetic fields are generated at the positions of gas cells 1021-1 to 1021-3 in different vector directions as indicated by arrows V1 to V3. Thus, the area where a myoelectric potential occurs may be identified by detecting a magnetic field in the $Y_0$ axis direction and a magnetic field in the $Z_0$ axis direction with the sensor modules provided at multiple areas instead of the sensor module provided at a single area.

As described above, the optically pumped atomic magnetometer detects a magnetic field in a direction substantially perpendicular to the incident direction of laser beam. Therefore, to incorporate an optically pumped atomic magnetometer in the sensor modules 510_1 to 510_3, the optically pumped atomic magnetometer is preferably disposed such that the YZ plane of the optically pumped atomic magnetometer is parallel to the YZ plane of the shielding member 600.

In other words, to incorporate an optically pumped atomic magnetometer in the sensor modules 510_1 to 510_3, the optically pumped atomic magnetometer is preferably disposed such that the incident direction of the laser beam is substantially parallel to the X-axis direction of the shielding member 600.

Thus, the sensor modules 510_1 to 510_3 may have a high sensitivity for detecting the magnetic field generated due to the current flowing through the muscle fiber 255 of the palm 220 in a state where the palm 220 is placed at a predetermined position.

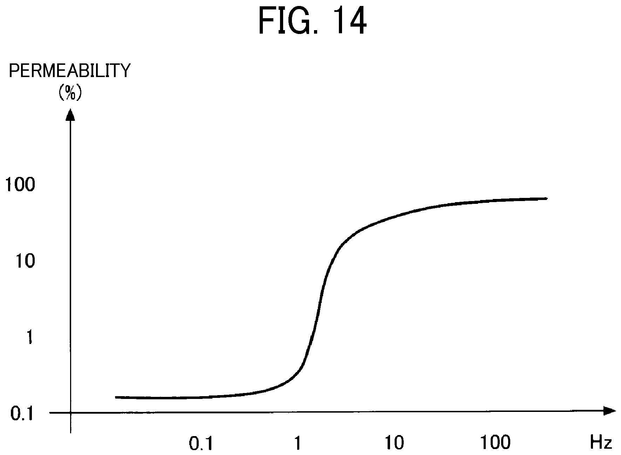

Next, the magnetic field shield characteristics of the flexible film 621 are described. FIG. 14 is a graph illustrating an example of the magnetic field shield characteristics of the flexible film 621. In FIG. 14, the horizontal axis represents a frequency, and the vertical axis represents the permeability of a magnetic field.

As illustrated in FIG. 14, the flexible film 621 secured to the boundary member 620 allows the permeability of a magnetic field having a frequency band higher than 1 [Hz] (having a filtering function to shield a magnetic field having a frequency band from 0.01 [Hz] to 1 [Hz]). Thus, the sensor modules 510_1 to 510_3 have a high sensitivity for detecting a magnetic field (100 [Hz] or more) generated due to the current flowing through the palm 220 even when the sensor modules 510_1 to 510_3 are in contact with the subject 200 via the flexible film 621.

Next, the opening/closing mechanism 602 disposed around the opening 601 in the shielding member 600 is described.

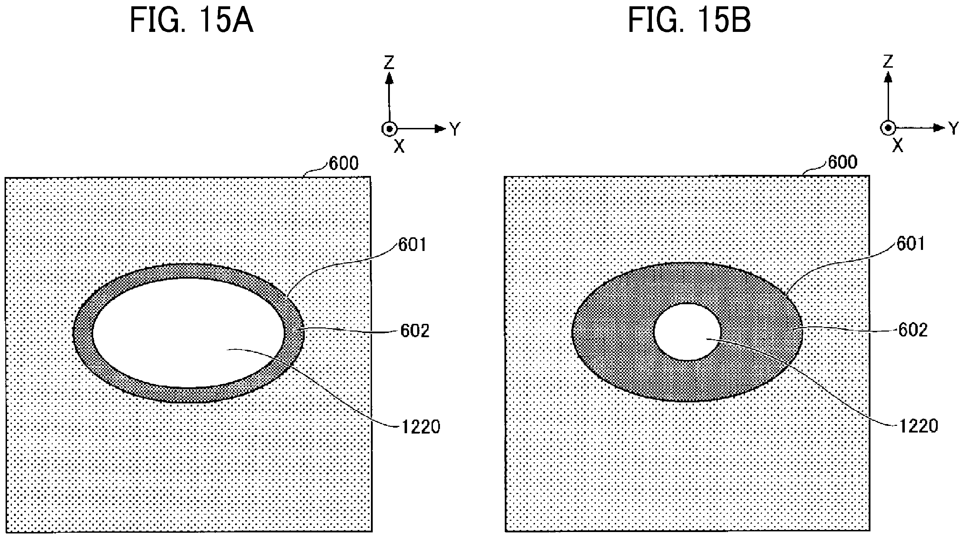

FIGS. 15A and 15B are first diagrams illustrating a function of the opening/closing mechanism 602 and illustrating a situation when the shielding member 600 is viewed in the X-axis direction. FIG. 15A illustrates a case where the opening/closing mechanism 602 is in an opened state. As illustrated in FIG. 15A, in a case where the opening/closing mechanism 602 is in an opened state, an insertion opening 1220, through which the arm of the subject 200 is inserted, has more than a certain size with respect to the opening 601 provided in the shielding member 600. This allows the subject 200 to easily insert the arm into the first space 610.

On the other hand, FIG. 15B illustrates a case where the opening/closing mechanism 602 is in a closed state. As illustrated in FIG. 15B, in a case where the opening/closing mechanism 602 is in a closed state, the insertion opening 1220 is narrower with respect to the opening 601 provided in the shielding member 600. This allows the first space 610 to be sealed in a state where the subject 200 has inserted the arm into the first space 610.

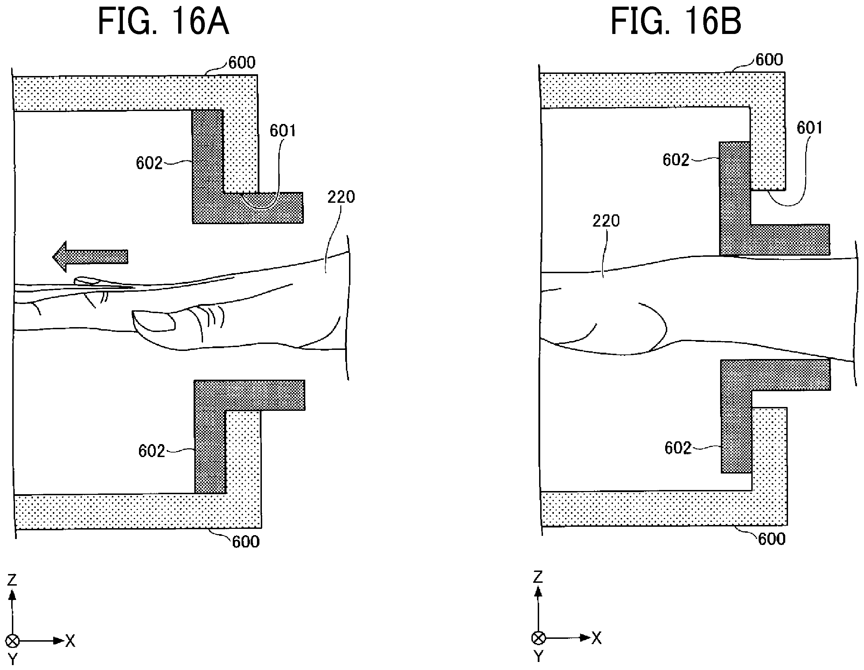

FIGS. 16A and 16B are second diagrams illustrating a function of the opening/closing mechanism 602 and illustrating a situation when the shielding member 600 is viewed in the Y-axis direction. FIG. 16A illustrates a situation where the opening/closing mechanism 602 is in an opened state and the subject 200 is inserting the palm 220.

On the other hand, FIG. 16B illustrates a situation where the opening/closing mechanism 602 is in a closed state and the subject 200 has inserted the arm. As illustrated in FIG. 16B, the opening/closing mechanism 602 holds part of the arm of the subject 200 while in a closed state. It is preferable that the part held by the opening/closing mechanism 602 is, among various sites of the subject 200, a site (e.g., wrist) that is made up of bones at a higher percentage as compared with a muscle. This is because the magnetic permeability is higher in a site made up of bones at a higher percentage as compared with a muscle.

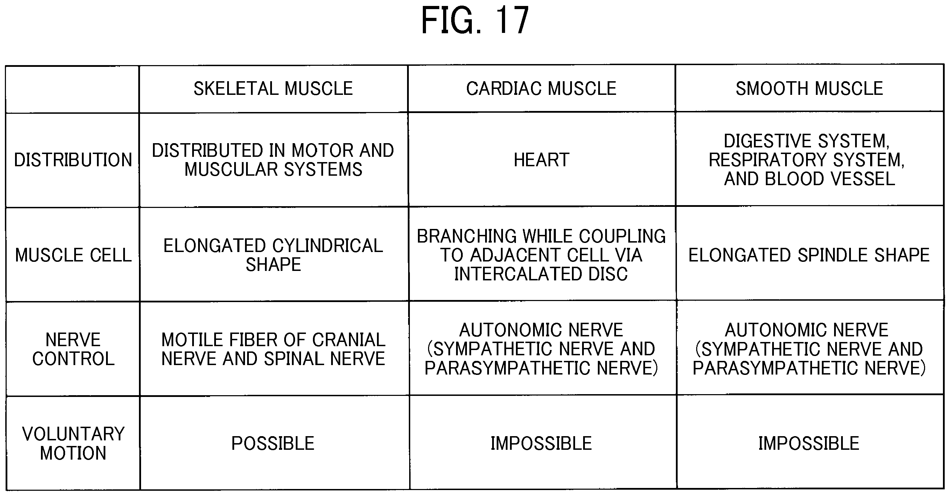

It is known that there are three types of human muscles and, as illustrated in FIG. 17, the human muscles include skeletal muscles, cardiac muscles, and smooth muscles. Although there are conventional biomagnetic-field measurement apparatuses targeted for cardiac muscles, biomagnetic-field measurement apparatuses targeted for skeletal muscles are not known.

As only skeletal muscles allow voluntary motions and are controlled by the brain and motor nerves, skeletal muscles are primarily examined for needle electromyography used to diagnose ALS and muscular dystrophy. This is because a myoelectric waveform generated during a voluntary motion is interpreted so as to determine a disease. It is known that a skeletal muscle is an elongated cylindrical muscle cell (hereinafter referred to as muscle fiber) and a potential propagates in the fiber direction over a certain distance.

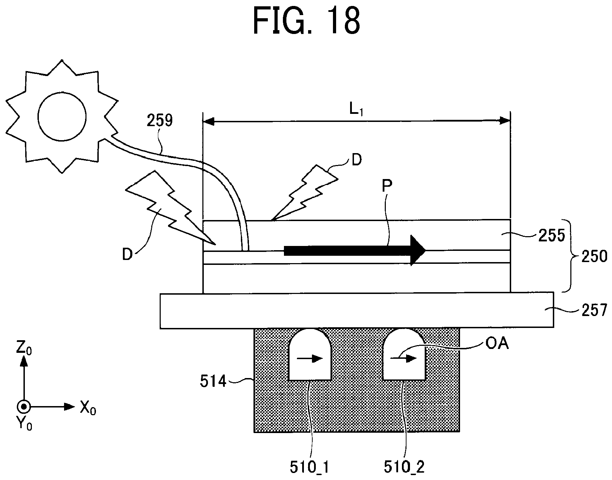

FIG. 18 is a diagram schematically illustrating the abductor pollicis brevis 250 that is an example of a skeletal muscle. As illustrated in FIG. 18, the abductor pollicis brevis 250 is a bundle of the muscle fibers 255. An overall length L1 of the abductor pollicis brevis 250 is approximately 40 mm, and the overall width thereof is approximately 20 mm. As the outer diameter of the single sensor module 510 is approximately a dozen mm, the sensor modules 510_1 and 510_2 may be arranged in parallel in the potential propagation direction P (the $X_0$ axis direction) of the muscle fibers 255, as illustrated in for example FIG. 18.

On the other hand, as cardiac muscles and smooth muscles are planar muscles, it is difficult to define a fiber direction. Furthermore, in cardiac muscles and smooth muscles, a myoelectric potential also spreads on a surface. For these reasons, it is not practical to arrange the sensor modules 510 in the muscle fiber direction or to align an optical axis OA of the sensor modules 510_1 and 510_2 with the muscle fiber direction.

FIG. 18 schematically illustrates fat 257, a motor nerve 259, and a discharge state D.

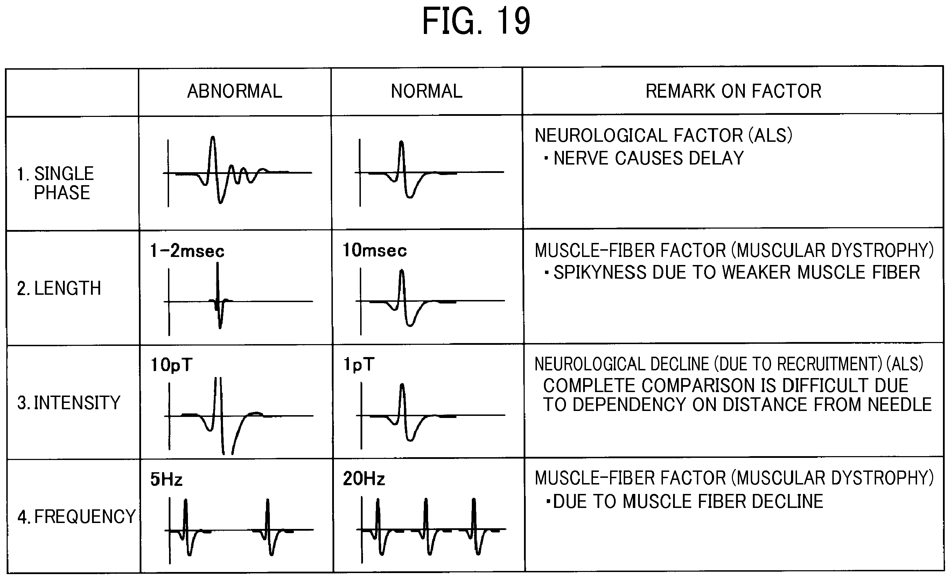

FIG. 19 is a first table illustrating waveforms (comparative example) of needle electromyography. As illustrated in FIG. 19, a waveform of the needle electromyography typically used for the examination on the ALS, etc., is a two to three phase (the negative potential repeats twice and the positive potential once) waveform of approximately 10 msec. A waveform, which is multiphase, spiky, or the like, is detected to make a diagnosis. The frequency at which a waveform is multiphase or spiky is detected to make a diagnosis. Also, the intensity (approximately 1 mV in potential and approximately 1 pT in a magnetic field as a standard) of a waveform is detected to make a diagnosis.

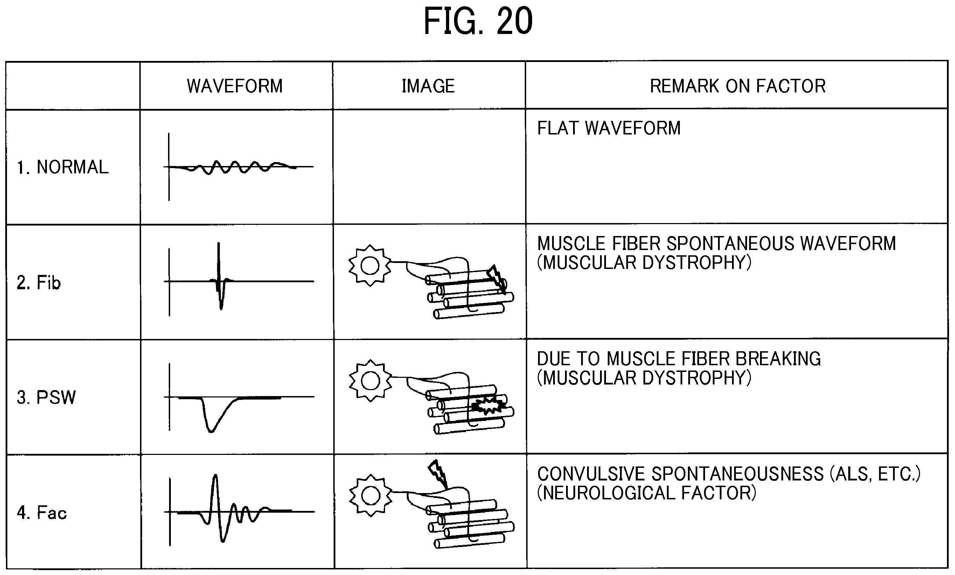

FIG. 20 is a second table illustrating a waveform (comparative example) of needle electromyography. As illustrated in FIG. 20, the waveform of a fibrillation potential, a positive sharp wave, or the like, is also observed in a resting state to examine whether there is abnormal discharge.

A typical sensor module including an optically pumped atomic magnetometer includes, for example, an OPM device manufactured by QuSpin Inc. The response speed of the OPM device is defined by the relaxation times T1 and T2 of the alkaline metal rare gas in the gas cell, and the response speed is typically approximately 200 Hz.

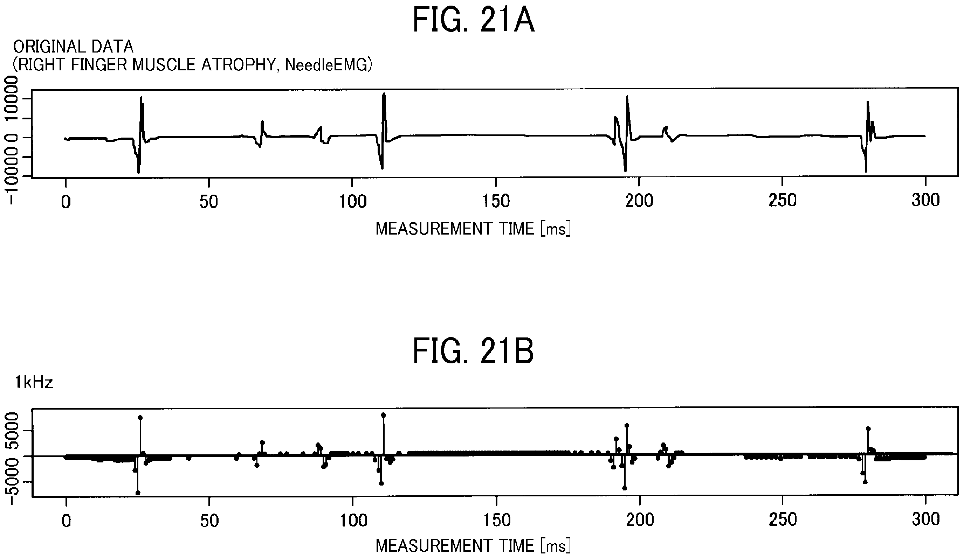

FIGS. 21A to 21D are diagrams for examining the response speed of a typical sensor module and illustrates waveforms measureable by an OPM device with one module.

The original data in FIG. 21A is a weak compression waveform of the abductor pollicis brevis of a healthy person obtained with needle electromyography, and two to three types of motor units are detected. Distinguishing between two to three types of waveforms in FIG. 21A is desirable.

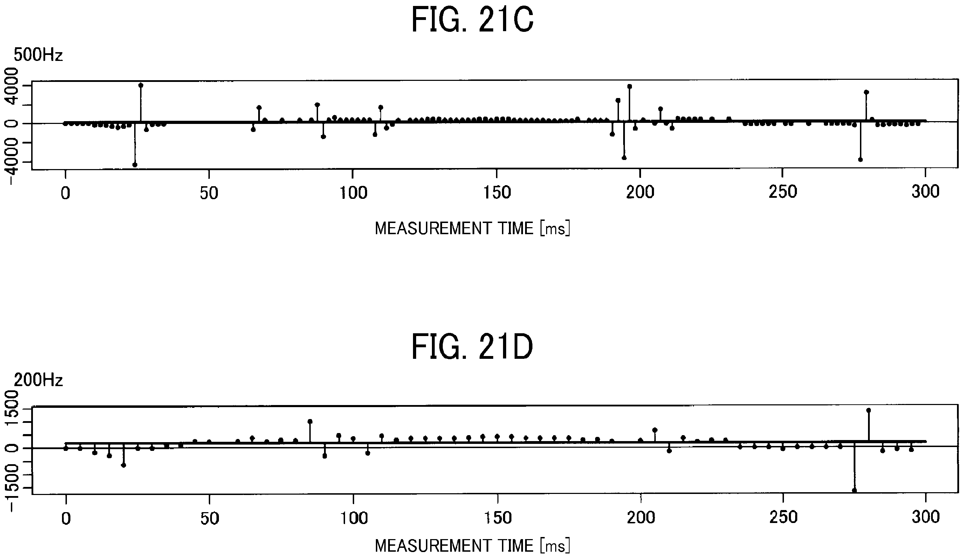

FIGS. 21B to 21D illustrate whether the two to three types of waveforms in FIG. 21A may be distinguished when a response speed is different. The waveform may be distinguished at a response speed of 1 kHz illustrated in FIG. 21B or at a response speed of 500 Hz illustrated in FIG. 21C, while it is difficult to sufficiently distinguish the waveform at a response speed of 200 Hz illustrated in FIG. 21D.

That is, in the OPM device with a response speed of 200 Hz, it is difficult to distinguish a weak compression waveform of the abductor pollicis brevis. To distinguish the weak compression waveform of the abductor pollicis brevis, a response speed higher than 200 Hz is desirable, and it is preferable to use a device having a response speed of at least approximately 500 Hz.

Next, an improvement in the accuracy of the sensor module is described. For example, a consideration is given to a case where the sensor modules 510_1 and 510_2 are arranged in parallel in the potential propagation direction P of the muscle fiber 255, as described above in FIG. 18.

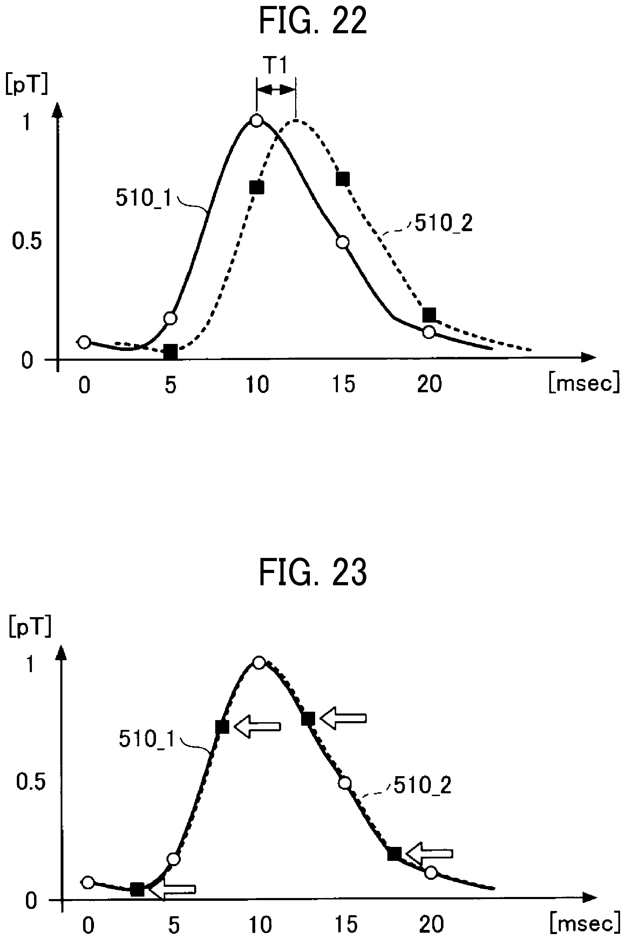

FIG. 22 is a first graph illustrating an improvement in the accuracy of the sensor modules 510_1 and 510_2. As illustrated in FIG. 22, in a case where the measurement points have an interval of 5 msec, for example, signals of the sensor modules 510_1 and 510_2 in FIG. 18 are measured at different positions, and each of the signals is meaningful data. Therefore, the signals are combined to calculate high-accurate data.

Generally, the potential propagation velocity of a skeletal muscle is several tens of m/sec, and it takes several msec to propagate several cm. Therefore, when the sensor modules 510_1 and 510_2 are arranged in the potential propagation direction P, as illustrated in FIG. 18, the detection times of the sensor modules 510_1 and 510_2 are shifted by several msec, as illustrated in FIG. 22. This time is defined as T1. T1 depends on the distance between the sensor module 510_1 and the sensor module 510_2.

FIG. 23 is a second graph illustrating an improvement in the accuracy of the sensor modules 510_1 and 510_2 and schematically illustrating a case where each data set of the sensor module 510_2 in FIG. 22 is shifted in the direction of the arrow by the time T1. As illustrated in FIG. 23, although there are five data sets in the case of the measurement at an interval of 5 msec (approximately 20 msec in total), nine data sets, almost twice as many, are presented, whereby an improvement in the accuracy of measurement data is achieved. Due to potential measurements, and the like, it is known that a propagation waveform is not largely deformed.

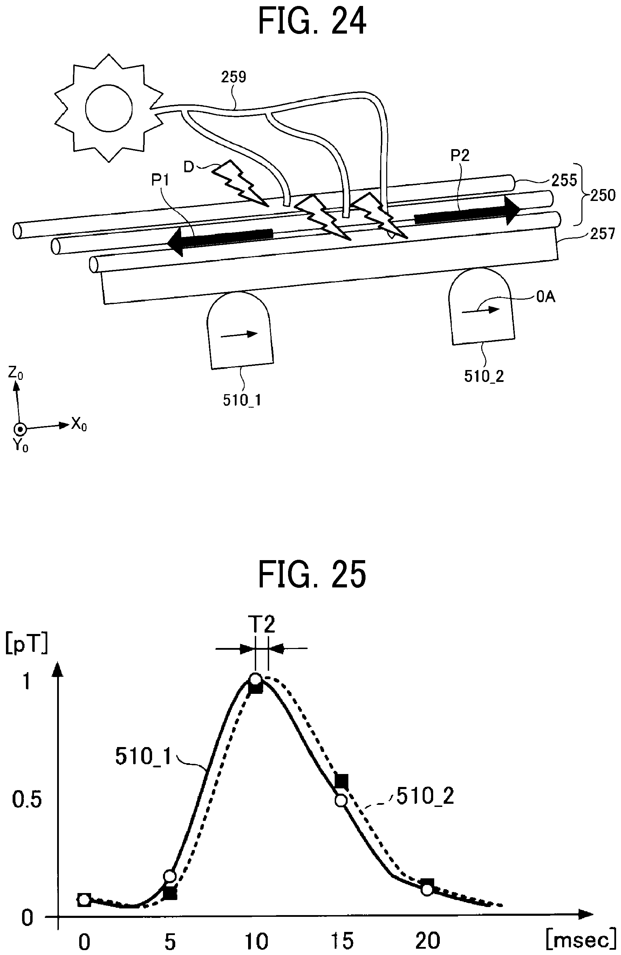

FIG. 24 is a third diagram illustrating an improvement in the accuracy of the sensor modules 510_1 and 510_2. As illustrated in FIG. 24, in some case, a motor nerve 259 is coupled to the muscle fiber 255 in the vicinity of the center thereof in the $X_0$ axis direction, and potentials propagate in two directions P1 and P2 (opposite directions) along the muscle fiber 255 due to the firing at the coupling area (central firing).

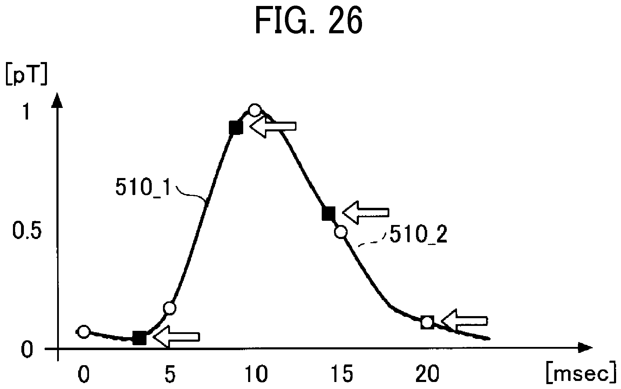

FIG. 25 is a fourth graph illustrating an improvement in the accuracy of the sensor modules 510_1 and 510_2 and illustrating signal waveforms in the case of FIG. 24. In this case, different from the potential propagation velocity of the skeletal muscle, the correction with T1 as in FIG. 23 is not proper. Central firing may be estimated to some extent by observing a signal waveform. In the case of central firing, T2 illustrated in FIG. 25 is defined, and a shift is made by T2, as illustrated in FIG. 26. As the measurement points of two waveforms are close, there is less effect on the correction due to an increase in the measurement points as compared with the case in FIG. 23; however, an improvement in the S / N, etc., is beneficial.

As described above, the parallel arrangement of the sensor modules 510_1 and 510_2 in the potential propagation direction P of the muscle fiber 255 improves the temporal resolution or the S / N.

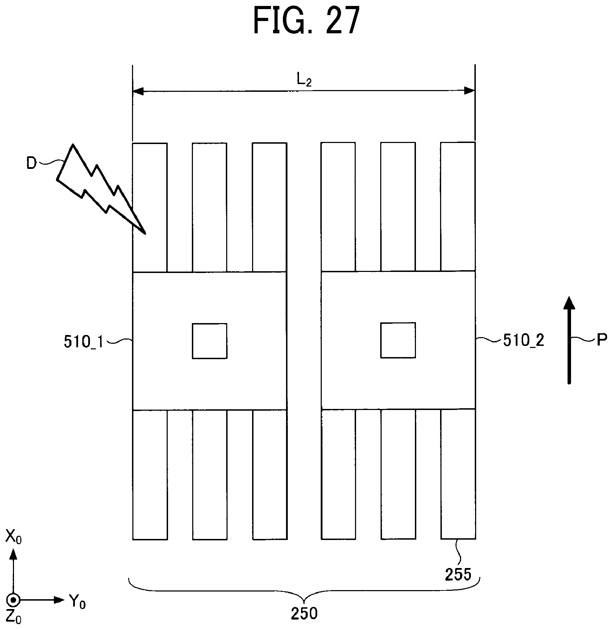

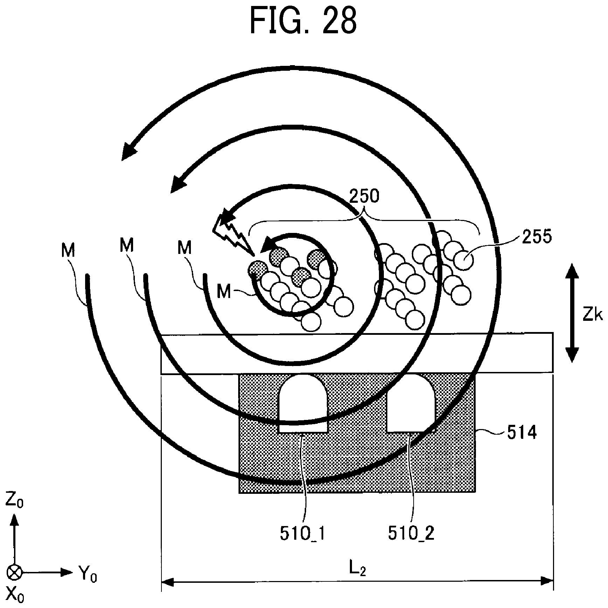

FIGS. 27 and 28 are diagrams illustrating another example of the arrangement of the two sensor modules 510_1 and 510_2. FIG. 27 illustrates a situation viewed from above when the muscle fiber direction is in the $X_0$ axis direction, and FIG. 28 illustrates a situation viewed in a direction perpendicular to the YZ plane. In FIG. 28, for example, when a current flows through the muscle fiber 255 from the back side of the drawing plane to the front side, a magnetic field is generated in the direction of an arrow M on the YZ plane.

In FIGS. 27 and 28, the sensor modules 510_1 and 510_2 are arranged in parallel in the $Y_0$ axis direction perpendicular to the $X_0$ axis direction (the same direction as the potential propagation direction P), which is the muscle fiber direction. As described above, an overall width L2 of the abductor pollicis brevis 250, which is the bundle of the muscle fibers 255, is approximately 20 mm.

As illustrated in FIGS. 27 and 28, the parallel arrangement of the sensor modules 510_1 and 510_2 in the Yo axis direction makes it possible to detect where the muscle fiber 255 has fired in the 20-mm width of the abductor pollicis brevis 250. In the examples of FIGS. 27 and 28, the firing position may be identified at the left edge of the drawing.

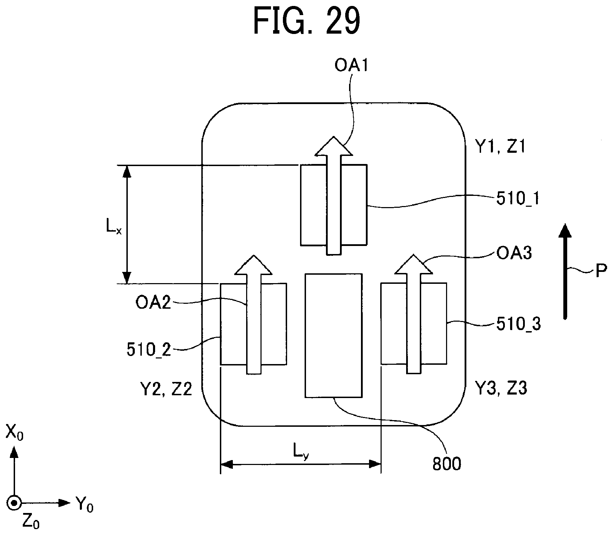

FIG. 29 is a diagram illustrating an example of the arrangement of the three sensor modules. As illustrated in FIG. 29, for example, the three sensor modules (the sensor modules 510_1 to 510_3) may be provided. In this case, it is preferable that a shielding plate 800 is sandwiched between the sensor module 510_2 and the sensor module 510_3 that are adjacent to each other in the optical axis direction. For example, the permalloy shielding plate 800 of approximately 2 mm sandwiched between the adjacent sensor modules may reduce crosstalk.

Cobalt-based amorphous metallic foil may be used as the shielding plate 800. In this case, as the cobalt-based amorphous metallic foil may be thinner than permalloy, the distance between the sensor modules 510 may be reduced to the minimum, and high-density and high-accuracy measurement is achieved. In addition, as cobalt-based amorphous metallic foil is not as heavy as permalloy, the flexibility during the movement of the sensor module 510 along the subject is not degraded.

In FIG. 29, as optical axes OA1 to OA3 (light propagation directions) of the sensor modules 510_1 to 510_3 are aligned with the potential propagation direction P (muscle fiber direction), the information on the depth direction may be obtained from the ratio of the magnetic-field data in the $Z_0$ direction to the magnetic-field data in the $Y_0$ direction.

That is, the optical axes OA1 to OA3 of the sensor modules 510_1 to 510_3 are arranged to be substantially parallel to the potential propagation direction P (muscle fiber direction). It is preferable that a distance Lx from the sensor module 510_1 to the sensor modules 510_2 and 510_3 in the $X_0$ axis direction is smaller than a distance Ly between the sensor module 510_2 and the sensor module 510_3 in the $Y_0$ axis direction.

That is, the relationship Lx<Ly is preferable. This is because the crosstalk in the light propagation direction is larger than the crosstalk in a direction perpendicular to the light propagation direction and preventing the crosstalk is desirable. Maintaining the relationship Lx<Ly makes it possible to have the closest packing position in the layout of the sensor modules and estimate the waveform of a muscle fiber with high accuracy.

A method for calculating a distance Zk in the depth direction at the firing position by comparing the magnetic-field data in the $Z_0$ direction with the magnetic-field data in the $Y_0$ direction is described. As for the information on the depth direction, as the Biot-Savart law holds for the relation between a magnetic field and a current, the intensity is proportional to the square of the distance. The distance is estimated and the detected magnetic field intensity is multiplied by the correction coefficient based on the distance so that the magnetic field independent of the depth may be detected. It is known that the magnitude of a magnetic field in the muscular dystrophy is small, and the magnitude of a magnetic field in the ALS is large (giant motor unit (MUP)), and therefore the above-described correction is desirable for the accurate diagnosis.

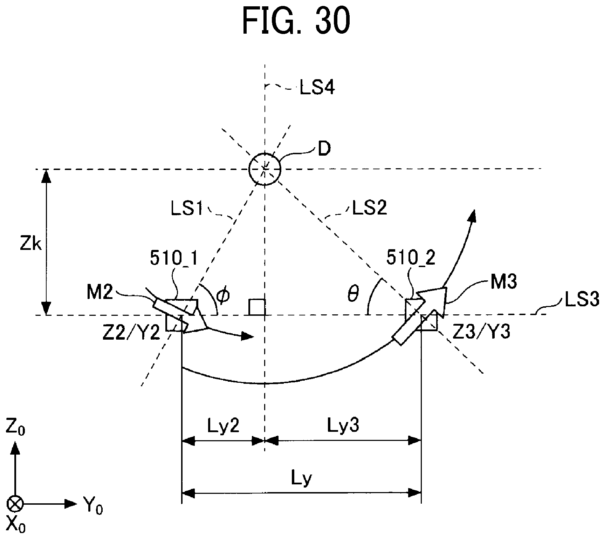

FIG. 30 is a diagram illustrating a specific method for quantifying a depth direction. In FIG. 30, a magnetic field is concentrically formed around a firing position D. A perpendicular LS1 is a perpendicular to a magnetic field M2 at the position of the sensor module 510_1. A perpendicular LS2 is a perpendicular to a magnetic field M3 at the position of the sensor module 510_2. The intersection of the perpendicular LS1 and the perpendicular LS2 is the firing position D.

A perpendicular LS4 from the firing position D to a perpendicular LS3 connecting the sensor module 510_1 and the sensor module 510_2 divides the distance Ly between the sensor module 510_1 and the sensor module 510_2 into a distance Ly2 and a distance Ly3. The distance Ly2 and the distance Ly3 may be expressed by $\theta$, $\varnothing$, and Zk.

Specifically, when the ratio "Zn/Yn" of a peak value Zn to a peak value Yn in the detection data of the sensor modules 510_1 and 510_2 is obtained, $\tan\varnothing=Z2/Y2$, $\tan\theta=Z3/Y3$. Furthermore, $Ly2 \times \tan\varnothing=Zk$, $Ly3 \times \tan\theta=Zk$, $Ly2+Ly3=Ly$. As Z2/Y2, Z3/Y3, Ly2, Ly3, and Ly are all known, the distance Zk in the depth direction at the firing position may be calculated from these values.

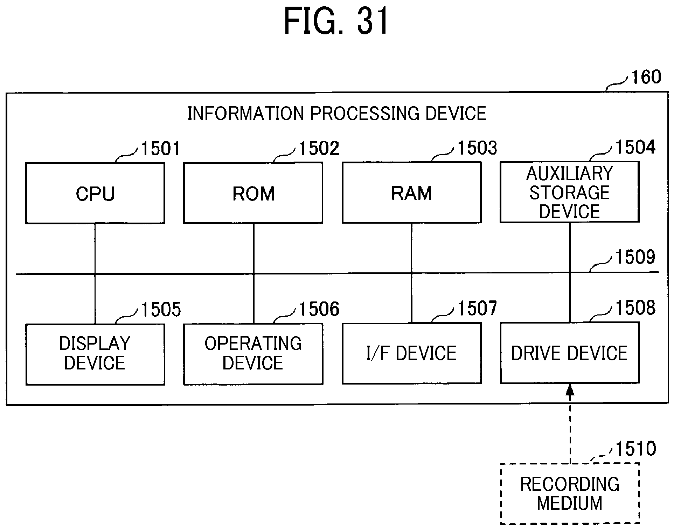

Next, the information processing device 160 is described. FIG. 31 is a diagram illustrating an example of the hardware configuration of the information processing device 160. As illustrated in FIG. 31, the information processing device 160 includes a central processing unit (CPU) 1501, a read only memory (ROM) 1502, and a random access memory (RAM) 1503. The CPU 1501, the ROM 1502, and the RAM 1503 form what is called a computer.

The information processing device 160 includes an auxiliary storage device 1504, a display device 1505, an operating device 1506, an interface (I/F) device 1507, and a drive device 1508. The hardware devices of the information processing device 160 are coupled to one another via a bus 1509.

The CPU 1501 is an arithmetic device that executes various programs (e.g., programs (referred to as information processing programs) for implementing the signal processor 560, the controller 562, and the display controller 563 described above) installed in the auxiliary storage device 1504.

The ROM 1502 is a non-volatile memory. The ROM 1502 functions as a primary memory device that stores various programs, data, and the like, for the CPU 1501 to execute various programs installed in the auxiliary storage device 1504. Specifically, the ROM 1502 functions as a primary memory device that stores boot programs such as Basic Input/Output System (BIOS) or Extensible Firmware Interface (EFI), and the like.

The RAM 1503 is a volatile memory such as a dynamic random access memory (DRAM) or a static random access memory (SRAM). The RAM 1503 functions as a primary memory device that provides a work area that is loaded when various programs installed in the auxiliary storage device 1504 are executed by the CPU 1501.

The auxiliary storage device 1504 is an auxiliary storage device that stores various programs and information acquired when various programs are executed.

For example, the data storage 561 is implemented by the auxiliary storage device 1504.

The display device 1505 is a display device that displays various types of image data (the image data 310, the waveform data 320, etc.). The operating device 1506 is an input device that allows a doctor, or the like, to input various instructions to the information processing device 160. The I/F device 1507 is a communication device that is coupled to the ultrasonic measuring device 120, the magnetic shield box 110, the electrical stimulation device 130, etc. so that the information processing device 160 communicates with each device.

The drive device 1508 is a device in which a recording medium 1510 is set. The recording medium 1510 described here includes a medium that records information optically, electrically, or magnetically, such as a CD-ROM, a flexible disk, or a magnetooptical disk. The recording medium 1510 may include a semiconductor memory that electrically records information, such as a ROM or a flash memory.

Various programs installed in the auxiliary storage device 1504 are installed when, for example, the distributed recording medium 1510 is set in the drive device 1508 and various programs recorded in the recording medium 1510 are read. Alternatively, various programs installed in the auxiliary storage device 1504 may be installed by being downloaded via the network.

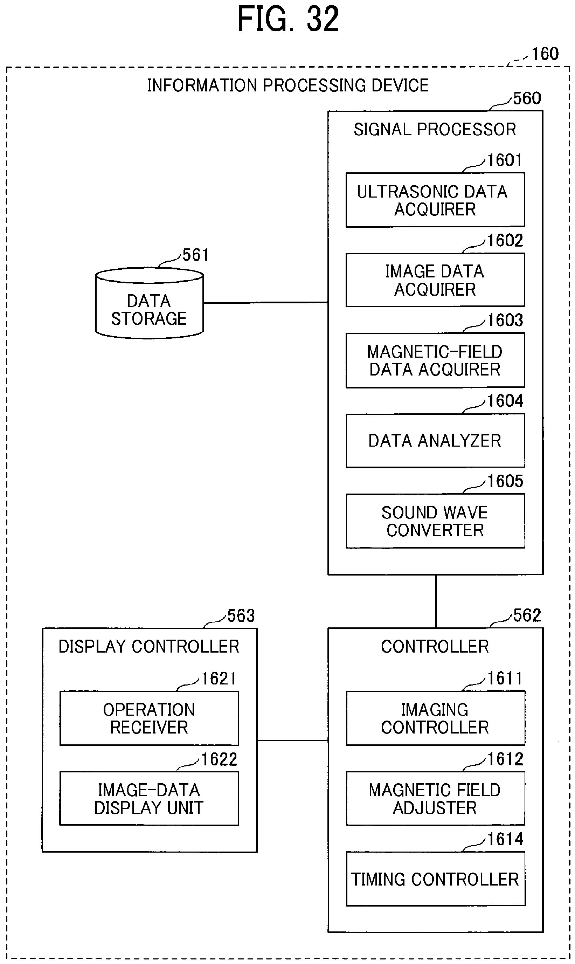

Next, a functional configuration of the information processing device 160 is described in detail. FIG. 32 is a diagram illustrating details of the functional configuration of the information processing device 160.

As illustrated in FIG. 32, the signal processor 560 includes an ultrasonic data acquirer 1601, an image data acquirer 1602, a magnetic-field data acquirer 1603, a data analyzer 1604, and a sound wave converter 1605.

The ultrasonic data acquirer 1601 acquires the ultrasonic data transmitted from the ultrasonic measuring unit 520, calculates the thickness of the fat at a predetermined position in the palm of the subject 200 based on the acquired ultrasonic data, and stores the thickness of the fat in the data storage 561.

The image data acquirer 1602 acquires the image data transmitted from the camera 511 and stores the acquired image data in the data storage 561.

The magnetic-field data acquirer1603 acquires the magnetic-field data transmitted from the sensor module 510 and stores the magnetic-field data in the data storage 561.

The data analyzer 1604 reads the magnetic-field data stored in the data storage 561, interpolates the magnetic-field data to generate waveform data, calculates the numerical data based on the waveform data, and stores the waveform data and the numerical data in the data storage 561.

The sound wave converter 1605 reads the waveform data stored in the data storage 561 and converts the waveform data into a sound wave. That is, the intensity of the magnetic field measured by the sensor module 510 is converted into a sound wave.

As illustrated in FIG. 32, the controller 562 includes an imaging controller 1611, a magnetic field adjuster 1612, and a timing controller 1614.

The imaging controller 1611 transmits an imaging instruction to the camera 511 in response to a received imaging instruction from an operation receiver 1621 of the display controller 563.

After receiving an instruction for starting the magnetic field measurement from the operation receiver 1621 of the display controller 563, the magnetic field adjuster 1612 acquires the internal magnetic-field data measured by the MI sensor 512, calculates the current value of the current flowing through the coil 513, and transmits the current value to the current generator 540.

After receiving an instruction for starting the magnetic field measurement from the operation receiver 1621 of the display controller 563, the timing controller 1614 transmits the instruction to the electrical stimulation controller 530 at predetermined timing.

As illustrated in FIG. 32, the display controller 563 includes the operation receiver 1621 and an image-data display unit 1622.

In response to an input imaging instruction, the operation receiver 1621 notifies the imaging controller 1611 of the imaging instruction. Furthermore, in response to an instruction for starting the magnetic field measurement input from a doctor, or the like, the operation receiver 1621 notifies the magnetic field adjuster 1612 and the timing controller 1614 of the instruction.

The image-data display unit 1622 reads at least one of the waveform data and the numerical data on the muscle magnetic field stored in the data storage 561 and displays at least one of the waveform data and the numerical data.

Next, the positional relationship between the position where the electrical stimulation is applied to the subject 200 with the attached input terminal 130b of the electrical stimulation device 130 and the position where the magnetic field is detected by the sensor module 510 is described.

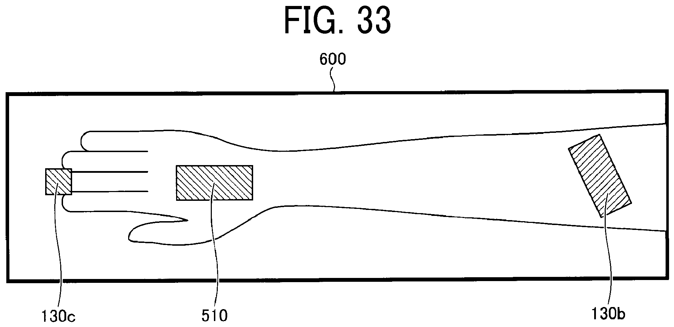

FIG. 33 is a diagram illustrating the positional relationship between the position where the electrical stimulation is applied and the position where the magnetic field is detected. As illustrated in FIG. 33, the input terminal 130b of the electrical stimulation device 130 is attached to a part (e.g., the elbow, which is a site different from the palm) of the arm of the subject 200.

That is, the electrical stimulation device 130 may apply electrical stimulation to the subject 200 via the input terminal 130b that is an electrode provided outside the shielding member 600. As the input terminal 130b is provided outside the shielding member 600, noise due to the electrical stimulation inside the shielding member 600 may be reduced, and a magnetic field waveform may be detected with high accuracy.

The palm 220 is placed at a predetermined position inside the shielding member 600, and the sensor module 510 detects the magnetic field at the position. Therefore, the position where the electrical stimulation is applied is separated from the position where the magnetic field is detected by a predetermined distance. As a result, there is a time difference corresponding to the predetermined distance from when the electrical stimulus is applied until the sensor module 510 detects the magnetic field.

A surface electrode pad 130c is attached to the position of a finger of the subject 200 so as to detects a signal when an electrical stimulation is applied, whereby the amount of evoked current, which serves as a reference to determine the amount of current for an eliciting stimulus, may be monitored.

It is possible to determine whether the position of the sensor module 510 is the optimal position depending on the occurrence of the muscle contraction in the abductor pollicis brevis due to an eliciting stimulus. For example, the amount of evoked current may be displayed live on a monitor so that the subject moves the thumb, which is the object under examination, to the position where the amount of evoked current displayed on the monitor reaches a maximum.

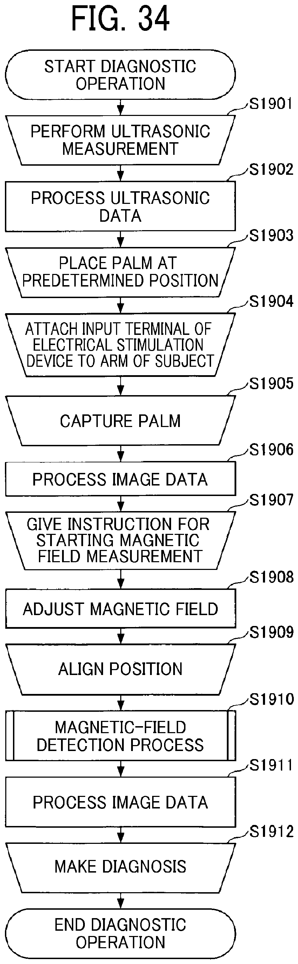

Next, a diagnostic operation when the doctor or the like performs a diagnosis based on the muscle magnetic field of the skeletal muscle of the subject 200 using the biomagnetic-field measurement apparatus 100 is described. FIG. 34 is a diagram illustrating the flow of an operation to make a diagnosis based on the muscle magnetic field of the skeletal muscle. Here, an example of detecting the muscle magnetic field of the abductor pollicis brevis of the hand is described.

At Step S1901, the doctor, or the like, uses the ultrasonic measuring device 120 to perform the ultrasonic measurement of the palm of the subject 200. Thus, the ultrasonic data is transferred from the ultrasonic measuring device 120 to the information processing device 160.

At Step S1902, the ultrasonic data acquirer 1601 of the information processing device 160 processes the ultrasonic data on the subject 200 and calculates the thickness of the fat at the position corresponding to the abductor pollicis brevis of the hand of the subject 200.

At Step S1903, the doctor or the like prompts the subject 200 to insert the arm into the shielding member 600 and place the hand of the subject 200 at a predetermined position. It is preferable to form a guide inside the shielding member 600 so that the hand of the subject 200 is in a generally appropriate position. This allows the abductor pollicis brevis of the subject 200 to be guided roughly to the position of the sensor module. It is preferable to lightly fix the hand of the subject 200 with an auxiliary guide in the above state.

At Step S1904, the doctor, or the like, attaches the input terminal 130b of the electrical stimulation device 130 to the arm of the subject 200.

At Step S1905, the doctor, or the like, operates the information processing device 160 to input an imaging instruction and drive the camera 511. Thus, the camera 511 captures the palm of the subject 200 placed at the predetermined position and transmits the image data to the information processing device 160.

At Step S1906, the image data acquirer 1602 of the information processing device 160 acquires the image data on the palm of the subject 200.

At Step S1907, the doctor or the like operates the information processing device 160 to input an instruction for starting the magnetic field measurement.

At Step S1908, the magnetic field adjuster 1612 acquires the internal magnetic-field data measured by the MI sensor 512 and calculates the current value. The current generator 540 applies the current with the calculated current value to the coil 513 to reduce the magnetic field inside the shielding member 600. The magnetic field at the position of the sensor module 510 inside the shielding member 600 may be reduced to, for example, 50 nT or less.

At Step S1909, the doctor, or the like, aligns the position of the sensor module 510. The positional alignment is described in detail later.

At Step S1910, each unit of the biomagnetic-field measurement apparatus 100 performs a magnetic-field detection process. Details of the magnetic-field detection process are given later.

At Step S1911, the image-data display unit 1622 displays the muscle magnetic-field data on the skeletal muscle of the subject 200. For example, the information processing device 160 displays the waveform data 320 on the muscle magnetic field of the abductor pollicis brevis illustrated in FIG. 3B.

At Step S1912, the doctor or the like diagnoses the muscle magnetic field of the skeletal muscle of the subject 200 based on the data obtained at Step S1909.

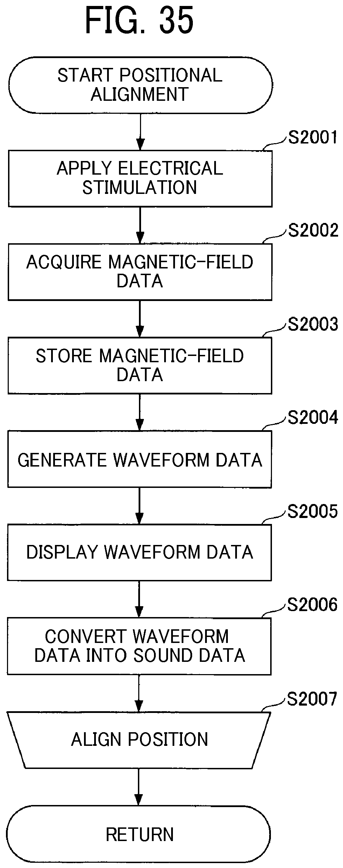

There is a method for detecting a spontaneous signal for the positional alignment between the sensor module 510 and the abductor pollicis brevis of the subject 200; however, in the example described according to the present embodiment, electrical stimulation is applied from outside and an evoked signal generated at that point is detected so that the positional alignment is conducted. FIG. 35 is a flowchart illustrating the flow of the positional alignment.

At Step S2001, the electrical stimulation device 130 applies electrical stimulation to the subject 200 via the surface electrode pad 130c.

At Step S2002, the sensor module 510 starts to detect a magnetic field, and the magnetic-field data acquirer 1603 acquires the data on the muscle magnetic field of the abductor pollicis brevis.

At Step S2003, the magnetic-field data acquirer 1603 acquires the magnetic-field data transmitted from the sensor module 510 and stores the magnetic-field data in the data storage 561.

At Step S2004, the data analyzer 1604 reads the magnetic-field data from the data storage 561, generates waveform data from the magnetic-field data, and stores the waveform data in the data storage 561.

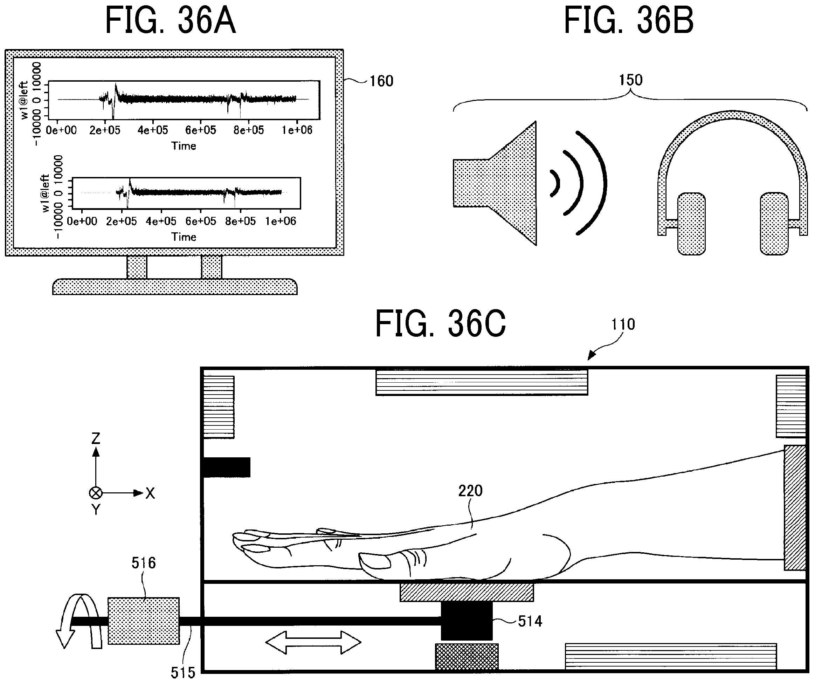

At Step S2005, the image-data display unit 1622 causes the information processing device 160 to display the waveform data stored in the data storage 561. For example, the information processing device 160 displays the waveform illustrated in FIG. 36A.

At Step S2006, the sound wave converter 1605 converts the waveform data displayed in FIG. 36A into a sound wave and causes the sound generating device 150 to generate a sound as illustrated in FIG. 36B. The sound generating device 150 includes, for example, a speaker or a headphone, and the doctor, or the like, may listen to the sound via the sound generating device 150.

For example, each data set of the sensor modules 510_1 to 510_3 having a response speed of approximately 200 Hz is converted to generate a waveform of approximately 1 kHz, and is generated by the sound generating device 150 as a sound for the doctor, etc. The conversion into a wavelength band that is most audible to the human ear allows easy auditory understanding.

The audible range of the human is said to be from 20 Hz to 20000 Hz. The response speed of the sensor module 510 is controlled by the relaxation times T1 and T2 of the alkaline metal in the rare gas and is said to be from DC to 200 Hz. As a signal in reaction to a myoelectric potential of the human is about 1 kHz, the conversion of the signal into a sound enables auditory detection in combination with visual determination.

Here, a waveform of approximately 200 Hz detected by one of the sensor modules 510 and a pseudo-waveform of approximately 400 Hz detected and corrected by the sensor modules 510 are used and are expanded to an audible range. Thus, it is possible to generate a sound wave that is audible to the ear and facilitates judgments. Furthermore, as a signal flows in real time during the measurement, it is desirable to instantly recognize abnormal discharge, etc. The use of ears and eyes enables more accurate recognition. Also, it is possible to promote learning for doctors who are not used to using the biomagnetic-field measurement apparatus 100.

At Step S2007, the doctor or the like moves the lever 515 to align the position of the sensor module 510 so as to maximize the sound from the sound generating device 150.

As illustrated in FIG. 36C, the holder 514 including the sensor modules 510_1 to 510_3 are coupled to the moving mechanism 516 with the lever 515. The moving mechanism 516 is configured to move the holder 514 in the tilt direction (θ) due to the rotation of the lever 515 and in a front-back direction (the X-axis direction) due to the insertion and the removal of the lever 515.

By moving the lever 515 in at least one of the rotation direction and the front-back direction, the sensor modules 510_1 to 510_3 and the abductor pollicis brevis of the subject 200 may be easily aligned at appropriate positions. After the positional alignment, the lever 515 is locked.

This method is desirable in that it is possible to easily align the sensor module 510 and the abductor pollicis brevis of the subject 200 at appropriate positions by listening to sound without the doctor, or the like, paying attention to the subject 200 or the waveform displayed on the information processing device 160.

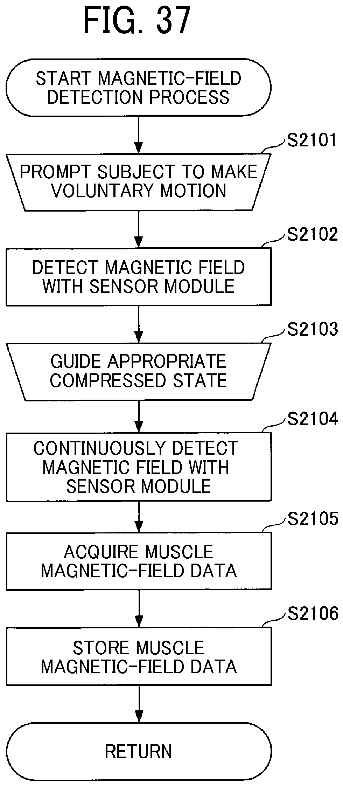

Next, the details of the magnetic-field detection process (Step S1910) are described. FIG. 37 is a flowchart illustrating the flow of the magnetic-field detection process.

At Step S2101, the doctor, or the like, prompts the subject 200 to make a voluntary motion (weak compression action). Here, the electrical stimulation device 130 stops electrical stimulation to the subject 200.

At Step S2102, the sensor module 510 starts to detect the magnetic field during the voluntary motion.

At Step S2103, while monitoring the detection result of the sensor module 510, the doctor, or the like, guides the subject 200 in voice so as to have an appropriate compressed state so that the appropriate waveform is output.

At Step S2104, the sensor module 510 continuously detects the muscle magnetic field of the abductor pollicis brevis during the voluntary motion for approximately one minute.

At Step S2105, the sensor module 510 stops detecting the magnetic field, and the magnetic-field data acquirer 1603 acquires the data on the muscle magnetic field of the abductor pollicis brevis.

At Step S2106, the magnetic-field data acquirer1603 stores the acquired data on the muscle magnetic field of the abductor pollicis brevis together with the position data in the data storage 561.

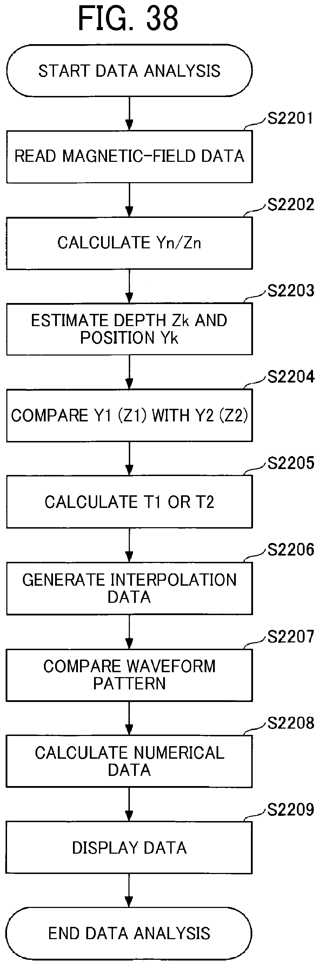

Next, data analysis is described. FIG. 38 is a flowchart illustrating an example of the flow of data analysis. It is assumed that the sensor modules are arranged as illustrated in FIG. 29.

At Step S2201, the data analyzer 1604 reads the magnetic-field data from the data storage 561. As each of the sensor modules 510_1 to 510_3 has data on the two axes, the data analyzer 1604 read six data sets in total. Here, each data is described as Yn, Zn. Data on the sensor modules adjacent in the Y direction are denoted by Y2 and Y3, and data on the sensor modules adjacent in the X-axis direction are denoted by Y1 and Y2.

At Step S2202, the data analyzer 1604 calculates Yn/Zn for all the data read at Step S2201. Information on a depth direction may be obtained from the values of Yn/Zn, as described above.

At Step S2203, the data analyzer 1604 refers to the ultrasonic-measurement form data (i.e., data on the thickness of fat at the position corresponding to the abductor pollicis brevis of the hand of the subject 200) obtained at Step S1902 to estimate the depth Zk and a position Yk. After Zk is determined, the depth may be corrected according to the Biot-Savart law.

At Step S2204, the data analyzer 1604 compares Y1 (Z1) with Y2 (Z2). The principle of the processes from Step S2204 to Step S2206 is as described above referring to FIGS. 23 and 26.

At Step S2205, the data analyzer 1604 calculates the amount of time gap (T1 or T2) illustrated in FIG. 22 or 25 based on the comparison result at Step S2204.

At Step S2206, the data analyzer 1604 performs data shift based on the values of T1 and T2 calculated at Step S2205, generates interpolation data, and stores, in the data storage 561, the waveform data for which the interpolation data has been generated. Due to the interpolation, the measurement data of 200 Hz (a sampling rate of 5 msec) may be changed into, apparently, the waveform data of 400 Hz (a sampling rate of 2.5 msec).

At Step S2207, the data analyzer 1604 compares the waveform data, for which the interpolation data has been generated at Step S2206, with the typical waveform pattern previously stored in the data storage 561.

At Step S2208, the data analyzer 1604 calculates numerical data for four items, e.g., multiphase/single phase, waveform length, waveform amplitude (intensity), and frequency, based on the comparison result at Step S2207 and stores the numerical data in the data storage 561.

At Step S2209, the image-data display unit 1622 causes the information processing device 160 to display at least one of the waveform data and the numerical data stored in the data storage 561.

A waveform due to spontaneity may be displayed with high accuracy as described below. Furthermore, the measurement of a magnetic field waveform due to spontaneity makes it possible to determine whether it is the ALS or the muscular dystrophy.

First, at a first step, the sensor modules 510 arranged parallel to the muscle fiber direction are used to measure a magnetic field waveform due to an eliciting stimulus multiple times.

Then, at a second step, with regard to each of the sensor modules 510, the magnetic field waveforms due to the eliciting stimulus measured multiple times are integrated.

Then, at a third step, a time gap T of the integrated magnetic field waveforms is calculated with regard to the adjacent sensor modules 510. The calculation of the time gap T is as described above referring to FIGS. 26 and 29.

Then, at a fourth step, by using the sensor modules 510, a spontaneous magnetic field waveform is measured at the position identical to the position where the magnetic field waveform due to eliciting stimulus is measured.

Then, at a fifth step, the spontaneous magnetic field waveform measured by one of the adjacent sensor modules 510 is shifted on the time axis by the time gap T relative to the spontaneous magnetic field waveform measured by the other one of the adjacent sensor modules 510, and the spontaneous magnetic field waveforms are combined. Specifically, the spontaneous magnetic field waveform measured by the sensor module 510 located on the back side in the muscle fiber direction is shifted by the time gap T to generate waveform data.

Then, at a sixth step, the combined spontaneous magnetic field waveform is displayed on the information processing device 160.

In the above-described biomagnetic-field measurement method, the magnetic field waveforms due to the eliciting stimulus may be easily integrated at the eliciting timing as a trigger.

In the case of a signal in one motor unit of the skeletal muscle, as the junction point of a motor nerve and a muscle fiber (muscle cell) is almost at the same position, and therefore the time response of the potential propagating from the position in the muscle fiber indicates the time gap that is determined depending on the position of each of the sensor modules 510.

That is, once the time gap T is defined, it is likely that a waveform becomes similar to those of the other sensor modules due to the correction on the time gap T. It may be expected that, even if the positions are different, the waveforms are similar due to the correction on the time gap T.

Particularly, as it is considered that, in a case where the sensor modules are close to each other by several tens of mm, the potential propagation velocity of the skeletal muscle is approximately 4 m/sec (4 mm/msec), it is estimated that the time gap T is approximately 7.5 msec at the position parallel to and away from the skeletal muscle fiber by 30 mm.

This value is actually measured in the actual positional relationship between the muscle fiber and the sensor module to determine the correction value. It is beneficial that the time gap T is determined with high accuracy by using data as accurate as possible due to the integration based on eliciting. The waveform due to spontaneity may be detected with using the time gap T.

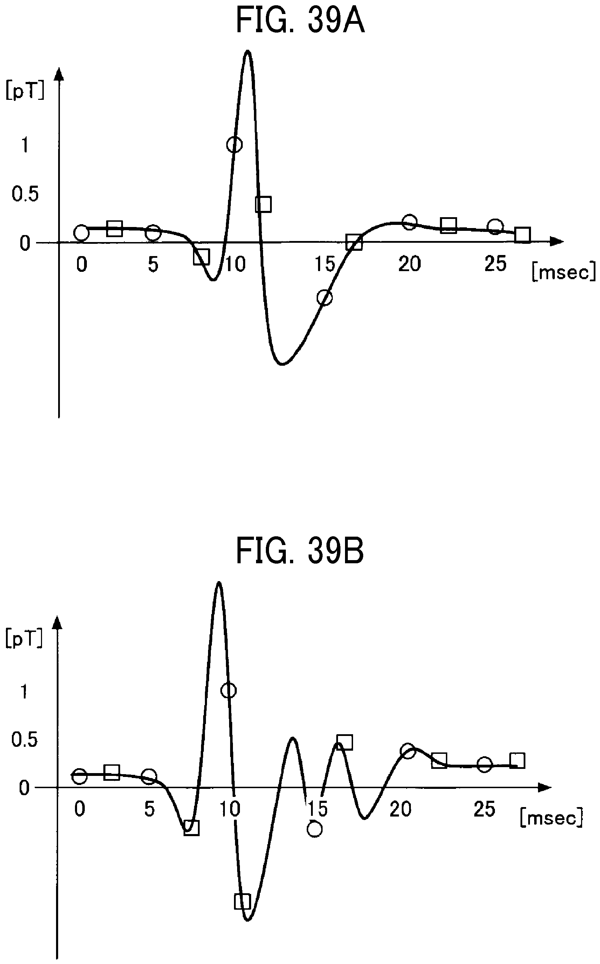

Next, the pattern recognition of a waveform is described by using specific examples of waveforms. FIGS. 39A and 39B are first graphs illustrating the pattern recognition of a waveform. FIG. 39A is a graph illustrating a motor unit waveform of a typical healthy person and illustrating an example of a single-phase waveform. FIG. 39B is a graph illustrating a motor unit waveform of an ALS patient and illustrating an example of a multiphase waveform.

FIGS. 39A and 39B illustrate the combination of data (circle) of 200 Hz (5 msec) measured by the sensor module 510_1 and data (square) of 200 Hz (5 msec) measured by the sensor module 510_2. In FIGS. 39A and 39B, the solid line indicates the waveform that is supposed to be acquired.

It is understood that, in FIGS. 39A and 39B, the data in the circle and the square acquired by the sensor modules 510 are on the waveform that is supposed to be acquired. When the waveform in FIG. 39A is compared with the waveform in FIG. 39B, the waveforms seem to be almost identical; however, the data is different in the vicinity of 10 msec and 20 msec. Although it is a slight difference, the pattern of the waveform that is difficult to be discriminated in the 200-Hz data may be discriminated in the data of approximately 400 Hz.

Like the discrimination on different waveforms due to the pattern recognition, feature data is extracted from a plurality of data waveforms, and it is determined whether the waveform is a normal waveform depending on whether the waveform exhibits the feature.

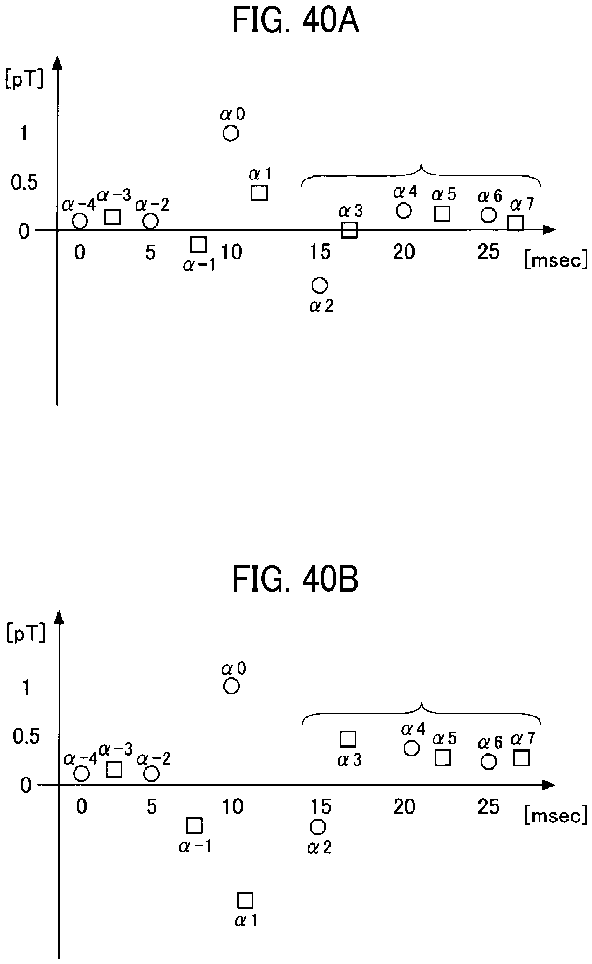

For example, in FIGS. 39A and 39B, the pattern recognition on a multiphase or a single phase depends on the magnitudes at 10 msec and 20 msec. Therefore, when the point indicating the peak is $\alpha 0$ and the subsequent points are $\alpha 1$ to $\alpha 7$ in the waveforms in FIGS. 39A and 39B, the values of $\alpha 2$ to $\alpha$ are compared to determine whether it is a multiphase or a single phase, as illustrated in FIGS. 40A and 40B.

For example, when the standard deviation of $\alpha 2$ to $\alpha 7$ is used as an index for determination, a single phase generally exhibits a value close to zero, and a multiphase exhibits a larger value. Instead of the standard deviation of $\alpha 2$ to $\alpha 7$, the average of the absolute values of $\alpha 2$ to $\alpha 7$, or the like, may be used for comparison. The method for waveform comparison is not limited to these and, for example, an AI (machine learning) technique may be used.

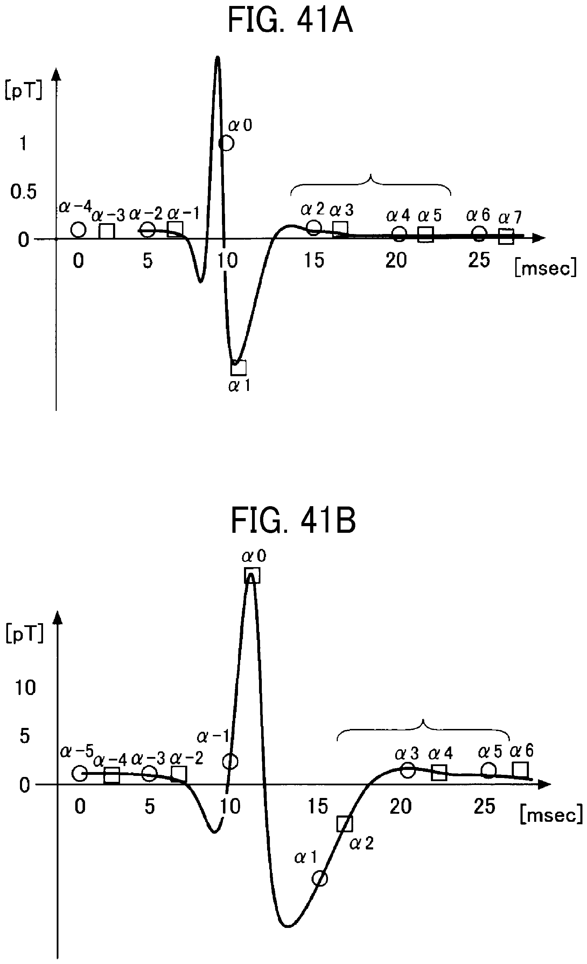

FIG. 41A illustrates an example of a spiky waveform presented by a muscular dystrophy patient, and FIG. 41B illustrates an example of a giant amplitude waveform presented by an ALS patient. As it is seen from the waveforms in FIGS. 41A and 41B, it is understood that combining the data of the sensor modules to change the response speed of 200 Hz to 400 Hz in a pseudo manner is effective.

In FIGS. 41A and 41B, in order to quantify the shortness (spikyness) of the waveform, a determination may be made with a focus on $\alpha 2$ to $\alpha 5$. For example, in a case where the length of the waveform is approximately 5 msec, as illustrated in FIG. 41A, $\alpha 2$ is almost zero, whereas in a case where the length of the waveform is 10 msec, as illustrated in FIG. 41B, $\alpha 2$ has a finite value.

That is, it is possible to quantify the shortness of a waveform depending on which value $\alpha 2$ has. This quantification is on a scale of no more than one to five, and the expression is not much detailed. However, it is appropriate as long as there is information to determine whether a waveform is short or long. As several hundred waveforms are read during the measurement, there is much point in a statistical value, and therefore a scale of no more than one to five is also beneficial.

Furthermore, $\alpha 0$ is recoded and is corrected in the depth direction according to the Biot-Savart law so that the intensity may be compared. For example, the corrected intensity is quantified. This results in effective information due to the quantification on a scale of about one to ten.

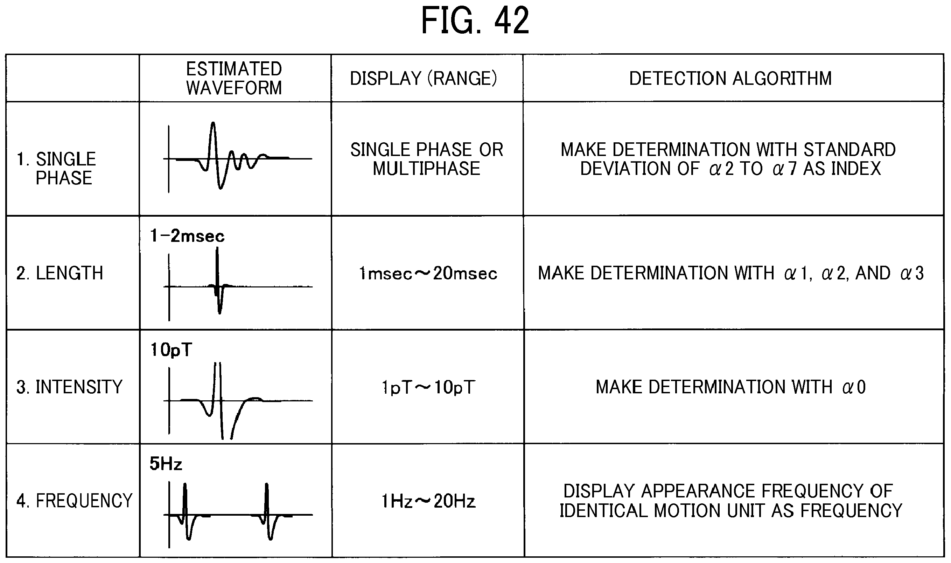

After a pseudo-waveform of 400 Hz is obtained, for example, the four items illustrated in FIG. 42 are calculated for one type of waveform and displayed on the monitor. The detection algorithm is the method described above. The detection frequency represents how often the identical motor unit appears. An individual motor unit is obtained as a pattern and is labelled, and the frequency is quantified.

As the firing position differs depending on each motor unit, it is also effective to label the firing position based on the calculated position information using the ratio of Y2 to Y3 as an index. In the case of firing at substantially the same position, Y2/Y3 or Z2/Z3 has the identical value.

As described above, in the biomagnetic-field measurement apparatus 100 according to the present embodiment, the sensor modules 510 are provided at the measurement position on the site of the live subject so that the magnetic field of the site of the live subject may be measured by the sensor modules 510.

For example, in a case where the sensor modules 510 are disposed parallel to the muscle fiber direction, the following effects are produced.

Although it is known that a potential propagates in the muscle fiber direction, the potential propagation velocity is several tens of m/sec, and it takes several msec to propagate several cm. For this reason, the arrangement of the sensor modules 510 in parallel to the muscle fiber direction (potential propagation direction) causes a difference in the detection time of the two sensor modules 510 by several msec. With the use of the two pieces of information, the waveforms are combined to produce an accurate waveform.

Generally, at 200 Hz (an interval of 5 msec), which is the response speed of the sensor module 510, it is difficult to distinguish between waveforms. However, with the data of 400 Hz, which is twice as high as 200 Hz, four to five measurement points may be obtained for uneven waveforms of approximately 10 msec, and the waveforms may be distinguished. Although it is difficult to distinguish between waveforms with three points, five points, which may represent various forms, are sufficient for items (1. single phase/multiphase, 2. long/short, 3. small/large) to distinguish between waveforms.

That is, the arrangement of the sensor modules 510 in parallel to the muscle fiber direction may improve the apparent response speed and, in the biomagnetic-field measurement apparatus using the optically pumped atomic magnetometer, the detection accuracy of the muscle magnetic field may be improved.

In a case where the sensor modules 510 are arranged perpendicular to the muscle fiber direction, the following effects are produced.

That is, it is possible to determine which area in the bundle of muscle fibers has fired. This makes it easy to determine which motor unit has the waveform. A difficulty in distinguishing between motor units causes a difficulty in quantifying the firing frequency, or the like, of the motor unit. However, as the biomagnetic-field measurement apparatus 100 may distinguish between motor units, it is easy to quantify the firing frequency, or the like, of the motor unit, and it is possible to improve the detection accuracy of the muscle magnetic field.

SECOND EMBODIMENT OF FIRST GROUP OF EMBODIMENTS

In a second embodiment of the first group of embodiments, an example of mounting a plurality of sensor modules in a fixed holder is described. In the second embodiment of the first group of embodiments, the description for the same components as those in the above-described embodiment may be omitted.

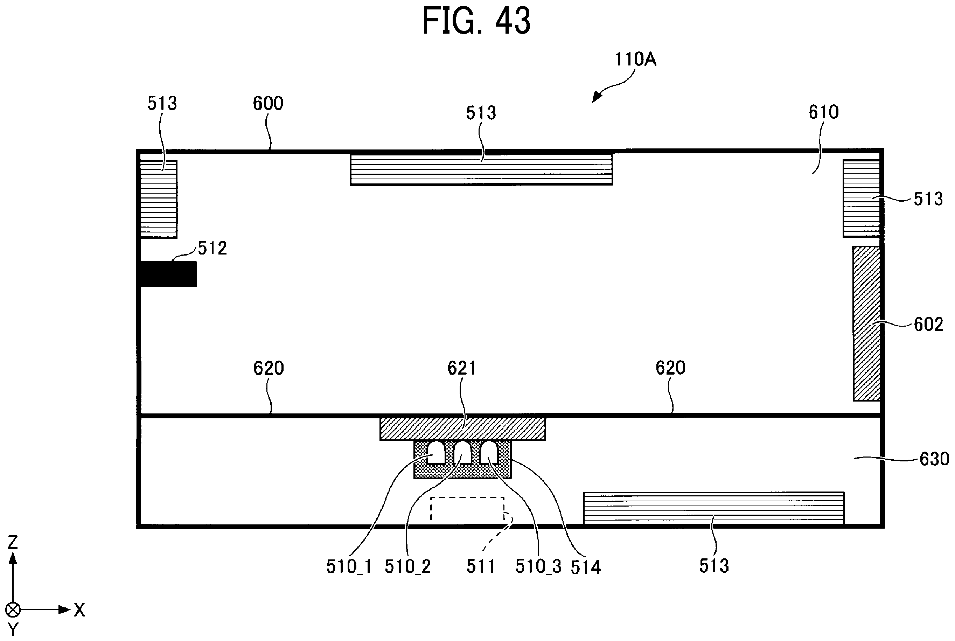

FIG. 43 is a diagram illustrating an internal configuration of a magnetic shield box according to the second embodiment of the first group of embodiments. As illustrated in FIG. 43, a magnetic shield box 110A is different from the magnetic shield box 110 (see FIG. 5) in that the holder 514 is fixed and is not movable and the sensor modules 510 are arranged in a matrix in the X-axis direction and in the Y axis direction. The magnetic shield box 110A does not include the lever 515 or the moving mechanism 516.

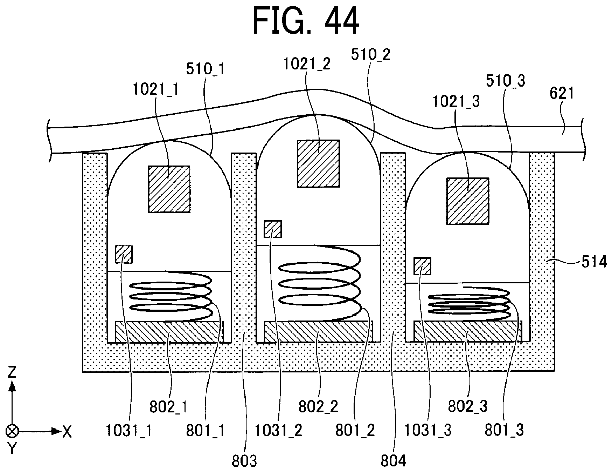

FIG. 44 is a diagram illustrating a detailed configuration of the holder. As illustrated in FIG. 44, the sensor module 510_1 is supported by a support base 802_1 via an elastic member 801_1 (e.g., a spring). The sensor module 510_2 is supported by a support base 802_2 via an elastic member 801_2 (e.g., a spring). The sensor module 510_3 is supported by a support base 802_3 via an elastic member 801_3 (e.g., a spring). The support bases 802_1, 802_2, and 802_3 are fixed to the holder 514.

As the sensor modules 510_1 to 510_3 are supported via the elastic members 801_1 to 801_3, the ends of the sensor modules 510_1 to 510_3 may be pressed against and brought into contact with the palm 220.

The sensor module 510_1 and the sensor module 510 2 are separated by a permalloy partition wall 803. The sensor module 510_2 and the sensor module 510_3 are separated by a permalloy partition wall 804. With the provision of the permalloy partition wall between the sensor modules, the occurrence of crosstalk between the sensor modules may be prevented.

As illustrated in FIG. 44, the sensor module 510_1 includes a gas cell 1021-1 and a position sensor 1031_1. The sensor module 510_2 includes a gas cell 1021-2 and a position sensor 1031_2. The sensor module 510_3 includes a gas cell 1021-3 and a position sensor 1031_3.

The gas cell 1021-1, the gas cell 1021-2, and the gas cell 1021-3 detect the magnetic field generated in, for example, the abductor pollicis brevis. The position sensor 1031_1, the position sensor 1031_2, and the position sensor 1031_3 detect the position when the magnetic field is detected.

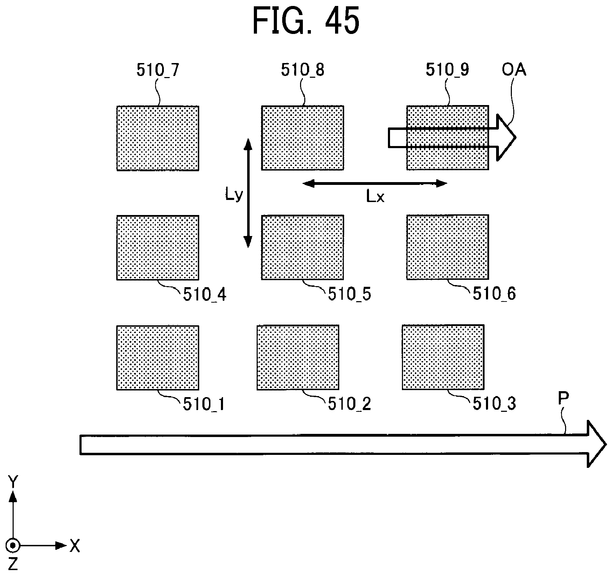

FIG. 45 is a diagram illustrating an example of the arrangement of sensor modules in the holder and schematically illustrating a state when the holder 514 is viewed from above.

As illustrated in FIG. 45, for example, the holder 514 may include the nine (three rows and three columns) sensor modules 510. This is an example, and the number of the sensor modules 510 included in the holder 514 is not limited to nine.

It is preferable that the nine sensor modules 510 illustrated in FIG. 45 are all arranged such that the incident direction OA (light propagation direction) of the laser beam of the built-in optically pumped atomic magnetometer is substantially parallel to the X-axis direction. Thus, the incident direction of the laser beam may match the potential propagation direction P (the direction of the muscle fiber), and the magnetic field generated due to the current flowing through the palm 220 may be detected with high sensitivity.

It is preferable that the sensor modules 510 are arranged such that the interval between the adjacent sensor modules in the X-axis direction is larger than the interval in the Y-axis direction. For example, it is preferable that the distance Lx between a sensor module 510_8 and a sensor module 510_9 in the X-axis direction is larger than the distance Ly between the module 510_8 and a sensor module 510_5 in the Y-axis direction.

That is, it is preferable to have the relationship of Lx<Ly. This is because the crosstalk in the light propagation direction is larger than the crosstalk in a direction perpendicular to the light propagation direction and preventing the crosstalk is desirable.

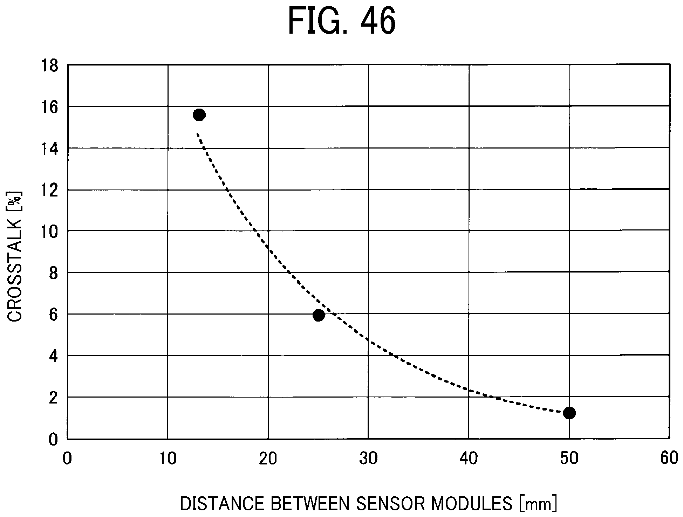

FIG. 46 is a graph illustrating an example of the crosstalk. The crosstalk illustrated in FIG. 46 is obtained by quantifying the distance between the two sensor modules arranged in the Y-axis direction and the change in the baseline of the 140-Hz signal.

It is understood, from FIG. 46, that when the distance between the sensor modules is close to 13 mm, for example, the baseline of 140 Hz increases by approximately 16%. In this case, a problem such as an increase in the noise of the sensor module may occur. When the distance between the sensor modules is approximately 23 mm, an increase (i.e., crosstalk) in the baseline of 140 Hz is approximately 6%, and the effect of crosstalk is reduced.

Maintaining the relationship Lx<Ly makes it possible to have the closest packing position in the layout of the sensor modules and estimate the waveform of a muscle fiber with high accuracy.

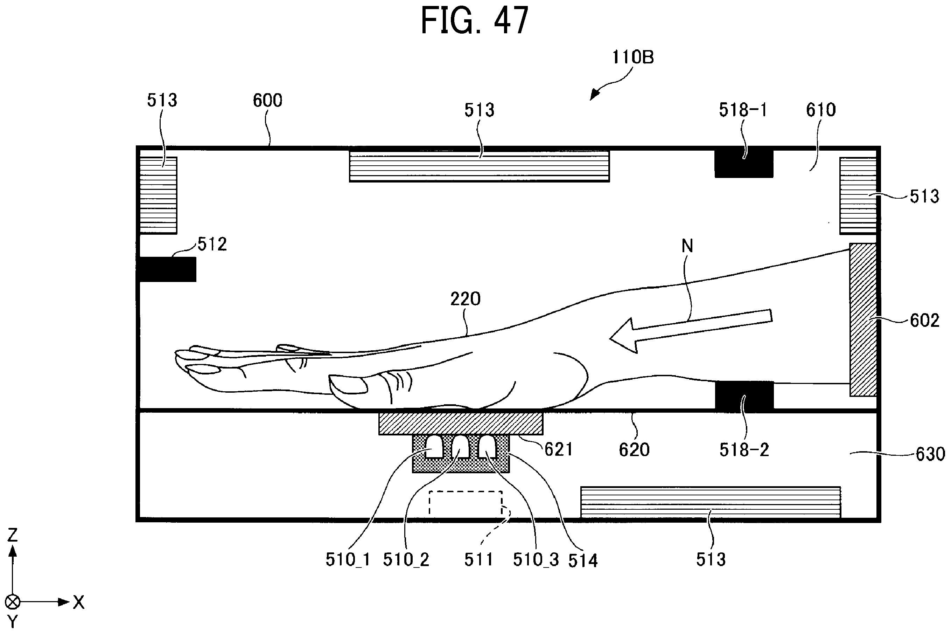

FIG. 47 is a diagram illustrating an internal configuration of the magnetic shield box according to a modification of the second embodiment of the first group of embodiments. As illustrated in FIG. 47, a magnetic shield box 110B is different from the magnetic shield box 110A (see FIG. 43) in that two MR sensors that measure a noise magnetic field are additionally included. One MR sensor may be additionally included, or three or more MR sensors may be additionally included.

As illustrated in FIG. 47, magneto resistive (MR) sensors 518_1 and 518_2 that detect a signal (i.e., a noise magnetic field) of a muscle other than the abductor pollicis brevis (the site to be examined) may be provided inside the shielding member 600. This allows the MR sensors 518_1 and 518_2 to detect a signal of a muscle other than the abductor pollicis brevis (the site to be examined) and to eliminate artifacts. In FIG. 47, the arrow N schematically illustrates a signal of a different muscle that causes noise.

The MR sensor has low detection sensitivity, which is several pT at most. On the other hand, the sensor module 510 may detect 100 fT. The major factor of noise is the propagation of a large magnetic field, generated from a nearby muscle, to the sensor module 510.

A magnetic field is proportional to the square of a distance according to the Lambert law. In the case of the measurement of the abductor pollicis brevis, etc., the noise due to a large muscle of the forearm or the upper arm in the neighborhood has an adverse effect. Therefore, the MR sensor is provided near the upper arm or the forearm to detect a signal generated by the corresponding part so that, for example, signal processing may be performed to remove the signal from a detection signal of the sensor module 510.

THIRD EMBODIMENT OF FIRST GROUP OF EMBODIMENTS

In a third embodiment of the first group of embodiments, an example of attaching, to the subject 200, an auxiliary member 680 for preventing the magnetic field from entering through the opening 601 of the shielding member 600 is described. In the third embodiment of the first group of embodiments, the description of the same components as those in the above-described embodiment may be omitted.

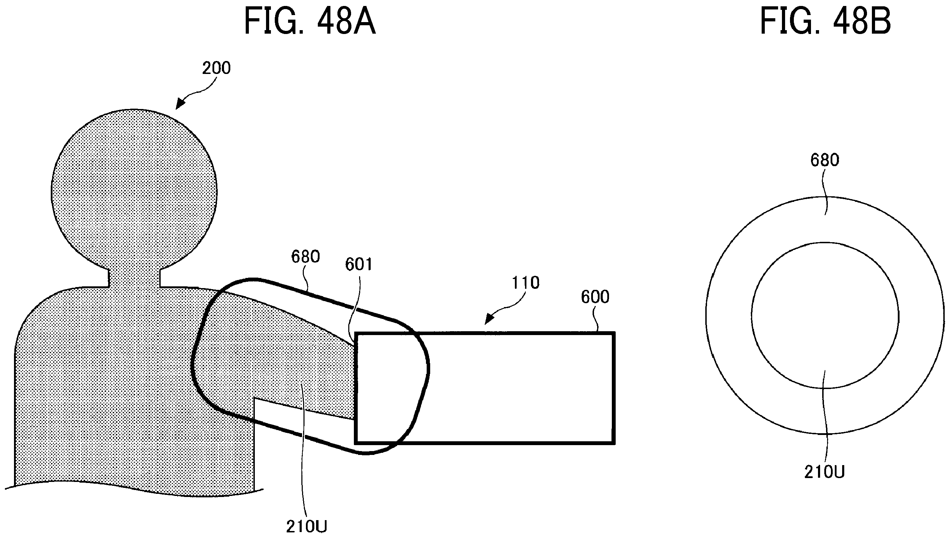

FIGS. 48A and 48B are diagrams illustrating the auxiliary member 680 according to the third embodiment of the first group of embodiments. FIG. 48A illustrates a state where the auxiliary member 680 is attached to the subject 200, and FIG. 48B illustrates the auxiliary member 680.

As illustrated in FIG. 48A, the auxiliary member 680 may be attached to an upper arm portion 210U of the subject 200. For example, the subject 200 first wears the auxiliary member 680 around the upper arm portion 210U and then wears the magnetic shield box 110.

The auxiliary member 680 includes, for example, a ring-shaped sponge member produced by knitting chemical fibers containing an amorphous metal, which is shield material. The upper arm portion 210U of the subject 200 is insertable into the inner side of the ring.

As the auxiliary member 680 is attached to the upper arm portion 210U of the subject 200, the magnetic field may be prevented from entering through the opening 601 of the shielding member 600 so that the residual magnetic field inside the shielding member 600 may be reduced.

The auxiliary member 680 is deformable so that the auxiliary member 680 is attachable to the upper arm portion 210U having a different shape depending on the sex or the individual difference. As the auxiliary member 680 is deformed when the subject 200 having the auxiliary member 680 attached to the upper arm portion 210U moves, the subject 200 feels less tired during the measurement, or the like.

FOURTH EMBODIMENT OF FIRST GROUP OF EMBODIMENTS

In a fourth embodiment of the first group of embodiments, an example of a magnetic shield box 110c that detects a magnetic field for a leg 230 of the subject 200 is described. In the fourth embodiment of the first group of embodiments, the description of the same components as those in the above-described embodiment may be omitted.

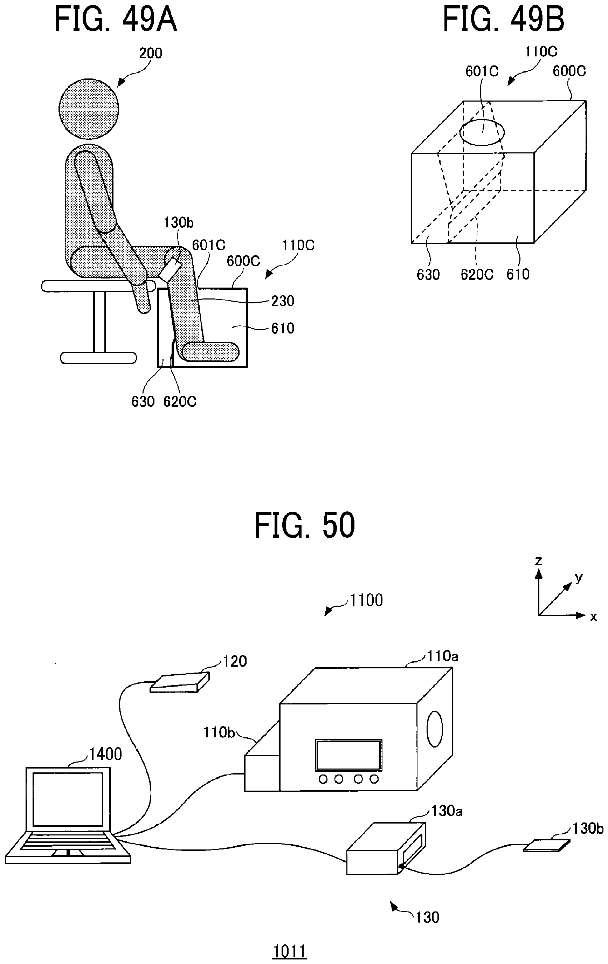

FIGS. 49A and 49B are diagrams illustrating the magnetic shield box 110c according to the fourth embodiment of the first group of embodiments. FIG. 49A illustrates a state where the magnetic shield box 110c is attached to the subject 200, and FIG. 49B illustrates the magnetic shield box 110c. In FIGS. 49A and 49B, although the sensor module 510, the MI sensor 512, and the like, are not illustrated, they may be appropriately placed at a position suitable for the magnetic field measurement of the leg 230 of the subject 200.

As illustrated in FIGS. 49A and 49B, a shielding member 600C of the magnetic shield box 110C is a cuboid and has an opening 601C, through which the leg 230 is insertable, on the top surface of the shielding member 600C. A boundary member 620C provided between the first space 610 and the second space 630 is bent in such a shape that the leg 230 may be easily inserted.

The configuration of the biomagnetic-field measurement apparatus except for the magnetic shield box 110C may be the same as the biomagnetic-field measurement apparatus 100 illustrated in, for example, FIG. 1.

In the biomagnetic-field measurement apparatus including the magnetic shield box 110C, for example, for the measurement of the magnetic field around the sural nerve of the leg 230, the subject 200 first sits on a chair and inserts the leg 230 into the shielding member 600C through the opening 601C. Then, the input terminal 130b is attached to the thigh (which may be a site located outside the shielding member 600C and different from the leg 230), or the like, of the subject 200. This makes it possible to apply electrical stimulation to the sural nerve and to measure a magnetic field around the sural nerve.

As described above, the site of the live subject targeted for the detection of a magnetic field is not limited to the hand of the subject and may be a leg that is a part of a limb of the subject 200.

In the case of muscular dystrophy, etc., a predictor is unlikely to appear in a muscle of the hand, and a predictor may appear in the leg. In that case, it is desirable to diagnose the muscle of the leg and, as described in the present embodiment, it is effective to use the technique for mearing a magnetic field around the sural nerve.

Although the shielding member 600C is a cuboid in the example of FIGS. 49A and 49B, the shielding member 600C may have any shape such as a cylindrical shape or a truncated cone shape.

Although the embodiments have been described above in detail, there is no limitation on the above-described embodiments, and various modifications and substitutions may be made to the above-described embodiments without departing from the range described in the scope of claims.

For example, although the MI sensor is used as a magnetic sensor for the feedback in the example described above according to the first embodiment to the fourth embodiment of the first group of embodiments, a solid magnetic sensor (e.g., a magneto resistive (MR) sensor, a tunnel magneto resistance (TMR) sensor) other than the MI sensor may be used.

SECOND GROUP OF EMBODIMENTS

Next, a biomagnetic-field detection apparatus according to a second group of embodiments of the present invention is described.

There are conventionally known biomagnetic-field detection apparatuses that detect a biomagnetic field using a room-temperature magnetic sensor to calculate a weak current flowing through a nerve. For example, WO 2015/129756 discloses a biomagnetic-field detection apparatus that detects the magnetic field of the head or the chest of the subject by using an optically pumped atomic magnetometer. The use of the biomagnetic-field detection apparatus may visualize the nerve activity of the head or the chest of the subject.

If other sites (e.g., limbs) of the subject are applicable as the detection target of the biomagnetic-field detection apparatus, it is considered that the neurological disorder (e.g., carpal tunnel syndrome, cubital tubular syndrome, or diabetic neuropathy in legs) specific to the corresponding site may be properly diagnosed.

With regard to the above points, each embodiment of the second group of embodiments is described below referring to the accompanying drawings. In the description and the drawings, the components having substantially the same functional configuration are attached with the same reference numeral, and duplicate description is omitted.

FIRST EMBODIMENT OF SECOND GROUP OF EMBODIMENTS

First, an external configuration of a biomagnetic-field detection apparatus according to a first embodiment of the second group of embodiments is described. FIG. 50 is a diagram illustrating the external configuration of the biomagnetic-field detection apparatus. As illustrated in FIG. 50, the biomagnetic-field detection apparatus includes a detecting device 1100 including a measuring unit 110a and a drive 110b, the ultrasonic measuring device 120, the electrical stimulation device 130 including the generator 130a and the input terminal 130b, and an information processing device 1400.

The detecting device 1100 is a device that detects a magnetic field generated in the object under examination (the palm that is a part of a limb of the subject according to the first embodiment of the second group of embodiments). In a state where the arm of the subject is inserted and the palm is placed at a predetermined position, the measuring unit 110a of the detecting device 1100 detects the magnetic field generated in the palm and transmits the magnetic-field data (data group on the magnetic flux density at each time) to the information processing device 1400. The drive 110b of the detecting device 1100 drives each unit in the measuring unit 110a based on an instruction from the information processing device 1400.

According to the first embodiment of the second group of embodiments, the width direction of the measuring unit 110a of the detecting device 1100 illustrated in FIG. 50 is the x-axis direction, the depth direction thereof is the y-axis direction, and the height direction thereof is the z-axis direction.

The ultrasonic measuring device 120 is a device that transmits and receives ultrasonic waves to measure the cross-sectional shape of the palm. The ultrasonic measuring device 120 transmits the measured ultrasonic data to the information processing device 1400.

The electrical stimulation device 130 is a device that applies electrical stimulation to a predetermined site of the subject in a state where the subject's arm is inserted into the measuring unit 110a of the detecting device 1100 and the palm is placed at a predetermined position.

The generator 130a of the electrical stimulation device 130 generates a voltage to be applied to an electrode of the input terminal 130b in response to an instruction from the information processing device 1400. The input terminal 130b of the electrical stimulation device 130 is attached to a predetermined site of the subject to apply the voltage to the site so as to give electrical stimulation.

The information processing device 1400 processes ultrasonic data transmitted from the ultrasonic measuring device 120 and calculates the position of each tissue (e.g., nerve) in the palm. The information processing device 1400 transmits an instruction to the electrical stimulation device 130 at predetermined timing to drive the electrical stimulation device 130.

The information processing device 1400 transmits an instruction for driving each unit of the measuring unit 110a to the drive 110b of the detecting device 1100. The information processing device 1400 receives magnetic-field data transmitted from the measuring unit 110a of the detecting device 1100. The information processing device 1400 calculates the current distribution of the palm when electrical stimulation is applied to the subject by using the position of each tissue in the palm and the received magnetic-field data and then displays the current distribution.

As described above, as the biomagnetic-field detection apparatus 1011 may calculate and display the current distribution of the palm when electrical stimulation is applied to the subject, the doctor, or the like, may properly diagnose carpal tunnel syndrome of the palm.

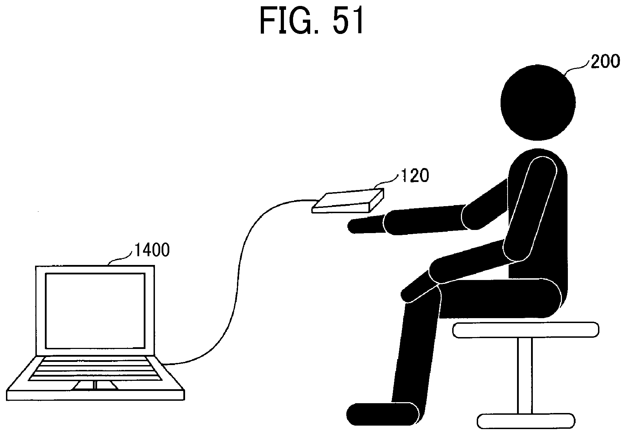

Next, the flow of an operation to diagnose carpal tunnel syndrome of the palm by using the biomagnetic-field detection apparatus 1011 is described. FIGS. 51 to 53 are first to third diagrams illustrating the flow of an operation to diagnose carpal tunnel syndrome.

FIG. 51 illustrates a situation where the doctor, or the like, measures the cross-sectional shape of the palm of the subject 200 using the ultrasonic measuring device 120. As illustrated in FIG. 51, the ultrasonic measuring device 120 measures the palm of the subject 200 so that the information processing device 1400 calculates the position of each tissue in the palm.

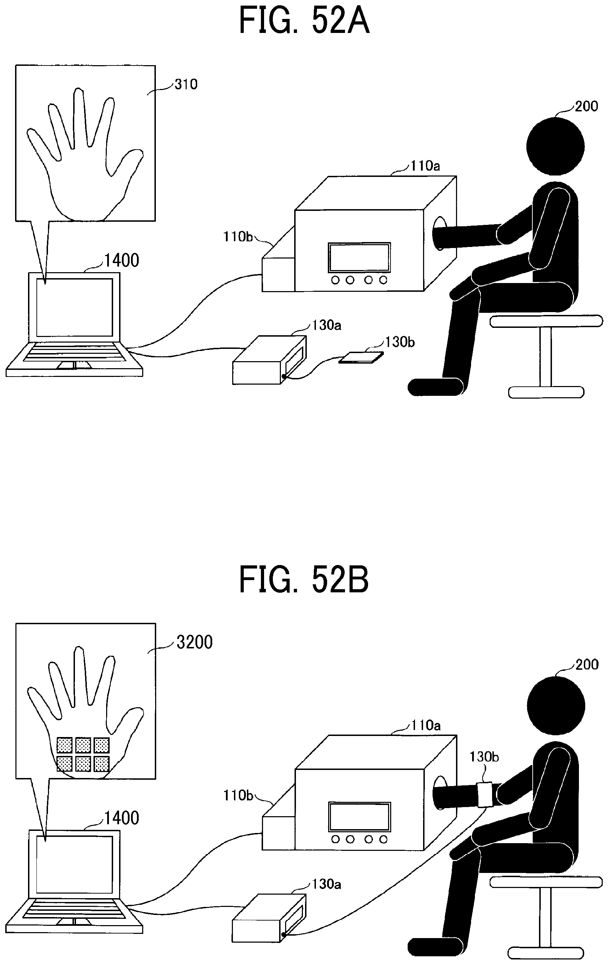

FIG. 52A illustrates a situation where the doctor, or the like, guides the subject 200 so as to insert the right arm into the measuring unit 110a of the detecting device 1100 and place the palm at a predetermined position within the measuring unit 110a.

Furthermore, FIG. 52A illustrates a situation where the doctor, or the like, operates the information processing device 1400 to drive the drive 110b of the detecting device 1100 so that a camera provided in the measuring unit 110a captures the palm. As illustrated in FIG. 52A, as the palm of the subject 200 is captured, the information processing device 1400 displays the image data 310.

FIG. 52B illustrates a situation where the doctor, or the like, attaches the input terminal 130b to the elbow (a site different from the palm that is the object under examination) of the subject 200. When the doctor, or the like, operates the information processing device 1400 in a state where the input terminal 130b is attached, the generator 130a is driven to apply electrical stimulation to the subject 200 (e.g., elbow median nerve). The measuring unit 110a detects the magnetic field generated in the palm and transmits the magnetic-field data to the information processing device 1400.

The information processing device 1400 calculates the current distribution of the palm using the position of each tissue in the palm and the magnetic-field data, superimposes the current distribution on the image data 310 to generate superimposition image data 3200, and displays the superimposition image data 3200.

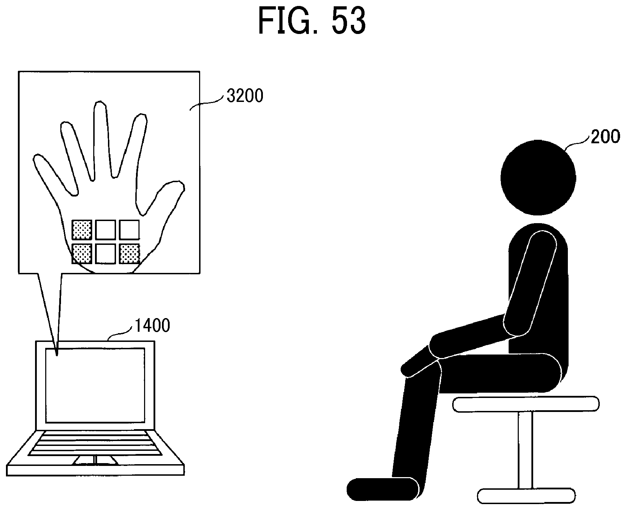

FIG. 53 illustrates a situation where the doctor, or the like, diagnoses carpal tunnel syndrome of the palm of the subject 200 based on the superimposition image data 3200. As illustrated in FIG. 53, the doctor, or the like, views the superimposition image data 3200 displayed on the information processing device 1400 so as to properly diagnose carpal tunnel syndrome of the subject 200.

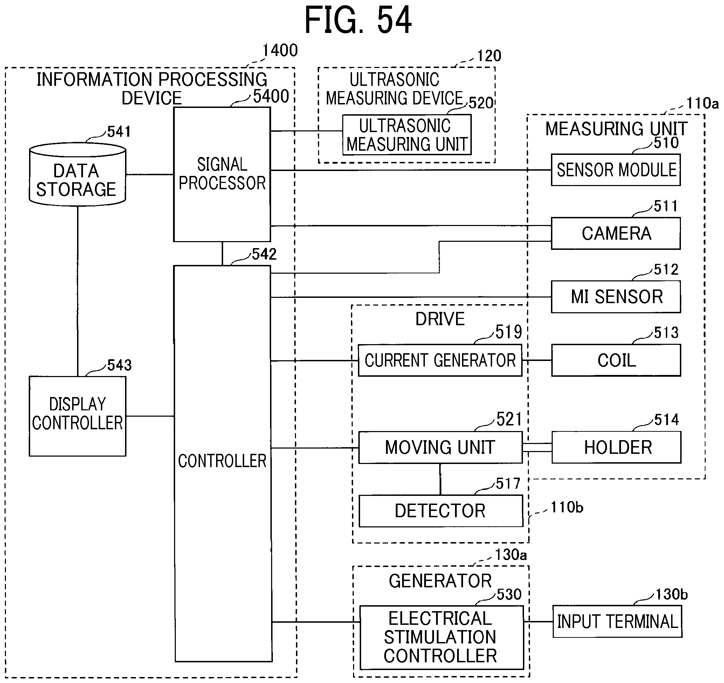

Next, a system configuration of the biomagnetic-field detection apparatus 1011 is described. FIG. 54 is a diagram illustrating an example of the system configuration of the biomagnetic-field detection apparatus 1011.

As illustrated in FIG. 54, in the biomagnetic-field detection apparatus 1011 according to the first embodiment of the second group of embodiments, the measuring unit 110a includes the sensor module 510, the camera 511, the MI sensor 512, the coil 513, and the holder 514.

The sensor module 510 has an optically pumped atomic magnetometer and a position sensor built therein. The sensor module 510 detects the magnetic field generated in the palm in a state where the end of the sensor module 510 is pressed against and is brought into contact with the palm placed at a predetermined position. In the sensor module 510, the built-in position sensor detects the position of the sensor module 510 when the magnetic field is detected. The sensor module 510 transmits the detected magnetic-field data and the detected position data to a signal processor 5400 of the information processing device 1400.

Although FIG. 54 illustrates the single sensor module 510, it is assumed that three sensor modules are arranged in the y-axis direction in the measuring unit 110a according to the first embodiment of the second group of embodiments.

The camera 511 captures the palm placed at a predetermined position and transmits the image data to the signal processor 5400 of the information processing device 1400.

The MI sensor 512 is a magnetic-field measurement sensor using a magneto-impedance element to measure a magnetic field in the measuring unit 110a. The MI sensor 512 transmits the measured internal magnetic-field data to a controller 542 of the information processing device 1400.

The coil 513 has the flowing current controlled by a current generator 519. Thus, the magnetic field inside the measuring unit 110a is set to 50 [nT] or less.

The holder 514 is a member that holds the sensor module 510. The holder 514 holds the sensor module 510 such that the end of the sensor module 510 is pressed against and is brought into contact with the palm placed at a predetermined position.

The holder 514 is attached so as to be movable in the x-axis direction in the measuring unit 110a. Thus, the holder 514 is movable in the x-axis direction while the end of the sensor module 510 is pressed against and is brought into contact with the palm placed at the predetermined position.

As illustrated in FIG. 54, in the biomagnetic-field detection apparatus 1011 according to the first embodiment of the second group of embodiments, the drive 110b includes the current generator 519, the moving unit 521, and a detector 517.

The current generator 519 acquires the current value calculated by the controller 542 of the information processing device 1400 based on the internal magnetic-field data measured by the MI sensor 512 and controls the current flowing through the coil 513 based on the acquired current value.

The moving unit 521 is a mechanism that moves the holder 514 in the x-axis direction. The moving unit 521 moves the holder 514 by a predetermined distance in the x-axis direction based on an instruction from the controller 542 of the information processing device 1400. The detector 517 detects the position of the holder 514 that has been moved in the x-axis direction by the moving unit 521.

As illustrated in FIG. 54, the ultrasonic measuring device 120 includes the ultrasonic measuring unit 520. The ultrasonic measuring unit 520 starts the measurement in response to an input instruction for starting ultrasonic measurement from the doctor, or the like, and transmits ultrasonic data to the signal processor 5400 of the information processing device 1400.

As illustrated in FIG. 54, the generator 130a includes the electrical stimulation controller 530. The electrical stimulation controller 530 generates the voltage to be applied to an electrode provided in the input terminal 130b in response to an instruction from the controller 542 of the information processing device 1400.

As illustrated in FIG. 54, the information processing device 1400 includes the signal processor 5400, the controller 542, and a display controller 543.

The signal processor 5400 calculates the position of each tissue in the palm of the subject 200 based on the ultrasonic data transmitted from the ultrasonic measuring unit 520 and stores the position in a data storage 541. The signal processor 5400 stores the image data transmitted from the camera 511 in the data storage 541. The signal processor 5400 uses the position of each tissue in the palm stored in the data storage 541 and the magnetic-field data and the position data transmitted from the sensor module 510 to calculate the current distribution of the palm and stores the current distribution data in the data storage 541.

The controller 542 receives a capturing instruction input by the doctor, or the like, via the display controller 543 and transmits an instruction to the camera 511. The controller 542 calculates the current value based on the internal magnetic field data measured by the MI sensor 512 and transmits the current value to the current generator 519. The controller 542 receives an instruction for starting the current distribution generation input by the doctor, or the like, via the display controller 543 and transmits an instruction to the current generator 519, the moving unit 521, and the electrical stimulation controller 530.

After the sensor module 510 detects the magnetic field generated in the palm at a predetermined position, the controller 542 transmits an instruction for moving the sensor module 510 to the next position to the moving unit 521.

The display controller 543 notifies the controller 542 of an instruction for starting the current distribution generation input by the doctor, or the like. The display controller 543 reads the current distribution data stored in the data storage 541 in response to the notification of the instruction for starting the current distribution generation, visualizes the data, superimposes the data in the image data stored in the data storage 541, and displays the superimposition image data.

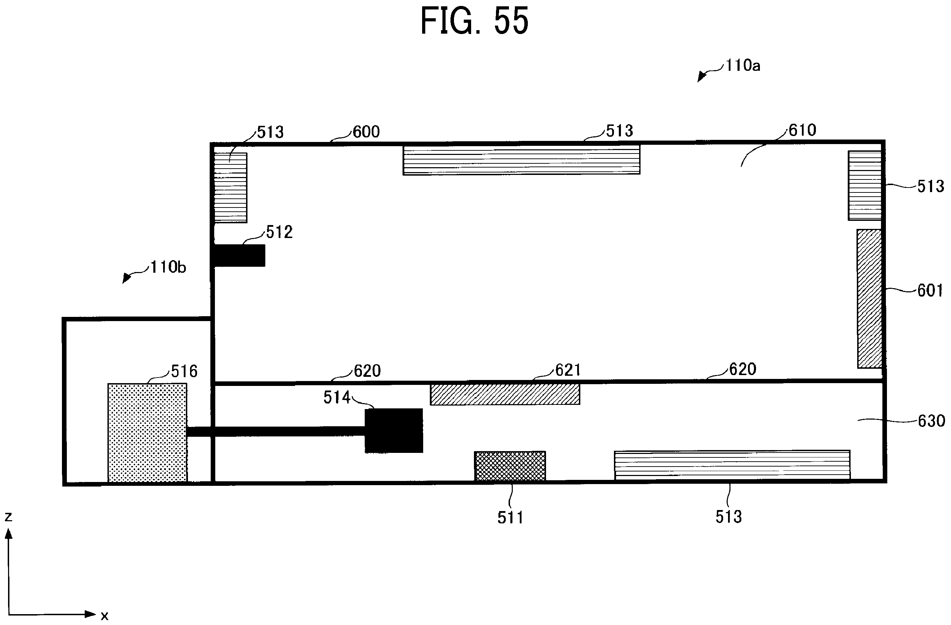

Next, an internal configuration of the measuring unit 110a and the drive 110b is described. FIG. 55 is a first diagram illustrating the internal configuration of the measuring unit 110a and the drive 110b. As illustrated in FIG. 55, the measuring unit 110a has a box shape covered with the shielding member 600 that shields electromagnetic waves. The box shape of the shielding member 600 is formed by bending, for example, a permalloy flat plate (a thickness of approximately 1 mm). In order to set the residual magnetic field inside the measuring unit 110a to 50 [nT] or less, the permalloy used for the shielding member 600 is formed in a three-layer structure.

The internal space of the measuring unit 110a is divided into the first space 610 in which the palm is placed and the second space 630 in which the holder 514 for holding the sensor module 510 is disposed. The boundary member 620 is provided between the first space 610 and the second space 630.

As illustrated in FIG. 55, the coils 513 are provided in the first space 610. The MI sensor 512 is disposed in the first space 610. The opening 601, through which the subject 200 inserts the arm into the first space 610, is provided in the first space 610, and the opening/closing mechanism 602 is disposed around the opening 601.

The camera 511, the coil 513, and the holder 514 are provided in the second space 630. The holder 514 is coupled to one end of a shaft and, when the moving unit 521 provided in the drive 110b operates the other end of the shaft in the x-axis direction, the holder 514 moves in the x-axis direction.

The boundary member 620 includes, for example, permalloy. An opening is provided near the center position of the boundary member 620, and a flexible film 621 is secured to the opening. The opening to which the flexible film 621 is secured is in the position where the palm is placed in the first space 610, and the end of the sensor module 510 held by the holder 514 is in contact with the palm placed in the first space 610 via the flexible film 621.

The flexible film 621 is pressed by the sensor module 510 in the holder 514 and is therefore deformed along the surface shape of the object under examination. The flexible film 621 includes a Teflon (registered trademark) film, or the like, having high smoothness so that the end of the sensor module 510 may smoothly move in contact with the object under examination via the flexible film 621 in accordance with the movement of the holder 514 in the X-axis direction. Alternatively, the flexible film 621 may include such as a magnetic shield film in which the front and back surfaces of an amorphous metallic foil are processed with a PET film (a magnetic shield film having an amorphous metallic foil sandwiched therein).

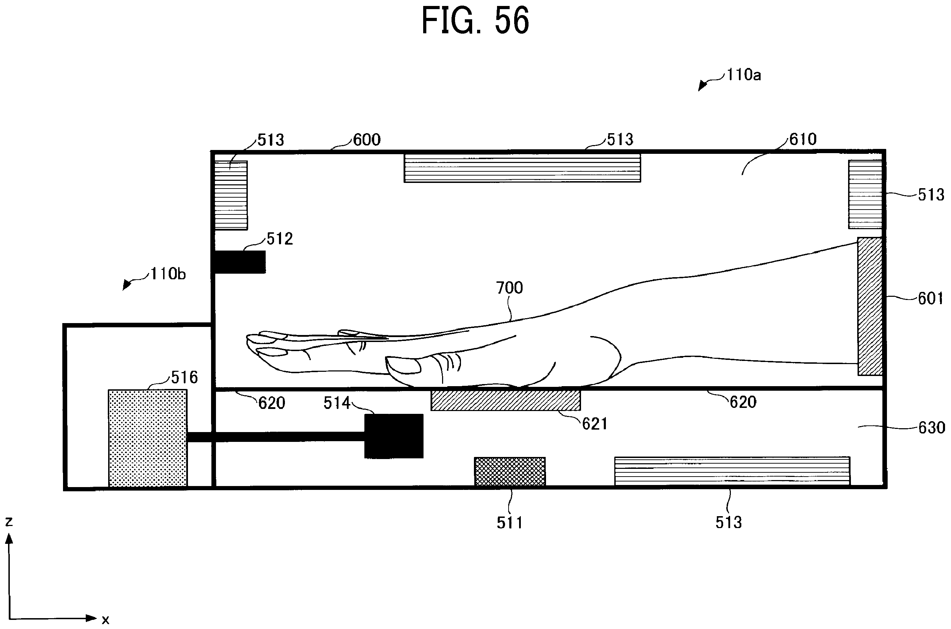

Next, the positional relationship with each unit in a case where the palm is placed in the measuring unit 110a is described. FIG. 56 illustrates a situation where the palm is placed in the measuring unit 110a. As illustrated in FIG. 56, the subject 200 inserts the arm through the opening 601 provided in the first space 610 to place a palm 220 at the position where the flexible film 621 is fixedly mounted.

As described later, as the opening/closing mechanism 602 provided around the opening 601 is closed in a state where the palm 220 is placed, the first space 610 is sealed. Thus, the shielding member 600 of the measuring unit 110a is configured such that the palm 220 of the subject 200 may be placed (may be held) inside the shielding member 600. The camera 511 may capture the palm 220 in a state where the palm 220 is placed. As the holder 514 is driven in the x-axis direction in a state where the palm 220 is placed, the sensor module 510 inside the holder 514 may detect the magnetic field generated in the palm 220 at each position of the palm 220 in the x-axis direction.

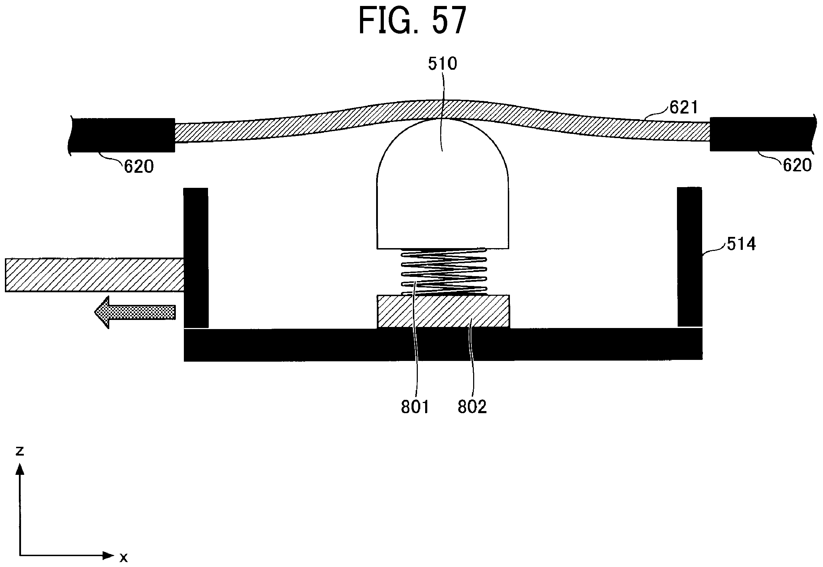

Next, a detailed configuration of the holder 514 in the measuring unit 110a is described. FIG. 57 is a first diagram illustrating the detailed configuration of the holder 514 in the measuring unit 110a. As illustrated in FIG. 57, in the holder 514, the sensor module 510 is supported by the support base 802 via the elastic member 801 (e.g., a spring). The support base 802 is secured to the holder 514.

In this manner, the sensor module 510 is supported via the elastic member 801 so that the end of the sensor module 510 may be pressed against and is brought into contact with the palm 220.

As described above, the sensor module 510 has the optically pumped atomic magnetometer and the position sensor built therein, and the sensor modules 510 are arranged in the Y-axis direction in the holder 514.

Next, a schematic configuration of the optically pumped atomic magnetometer included in the sensor module 510 is described. FIG. 11 is a diagram illustrating a schematic configuration of the optically pumped atomic magnetometer. As illustrated in FIG. 11, the optically pumped atomic magnetometer emits laser beam to a gas cell of rubidium atoms and detects the laser beam having passed through the gas cell by using a photodetector. The laser beam passing through the gas cell is absorbed depending on the magnitude of the magnetic field generated in a $Y_0$ axis direction or a $Z_0$ axis direction, and therefore the intensity of the laser beam detected by the photodetector decreases due to the magnetic field gen-

erated in the $Y_0$ axis direction or the $Z_0$ axis direction.

Thus, it is possible to calculate the magnitude of the magnetic field by comparing the intensity of the laser beam detected by the photodetector in a state where no magnetic field is generated and the intensity of the laser beam detected by the photodetector in a state where a magnetic field is generated. A coil is wound around the gas cell to apply an appropriate alternating current.

Thus, the optically pumped atomic magnetometer may detect a magnetic field in a direction substantially perpendicular to the incident direction (light propagation direction) of the laser beam. According to the present embodiment, the direction substantially parallel to the incident direction of the laser beam is an $X_0$ axis direction, and the directions substantially perpendicular to the incident direction of the laser beam are the $Y_0$ axis direction and the $Z_0$ axis direction.

Next, an example of the arrangement of the sensor modules in the holder 514 is described. FIG. 13 is a diagram illustrating an example of the arrangement of the sensor modules in the holder 514. As described above, the three sensor modules (the sensor modules 510_1 to 510_3 in the example of in FIG. 13) are arranged in the y-axis direction in the holder 514.

As illustrated in FIG. 13, the sensor modules 510_1 to 510_3 include gas cells 1021-1 to 1021-3, respectively, near the end portions thereof. In a state where the palm 220 is placed at a predetermined position, a muscle fiber 255 in the carpal tunnel of the palm 220 runs in a direction substantially perpendicular to the yz plane.

When a current flows through the muscle fiber 255 from the front side of the drawing plane to the back side thereof, a magnetic field is generated in the direction of an arrow 1000 on the yz plane. As a result, at the positions of the gas cells 1021-1 to 1021-3, magnetic fields are generated in the directions indicated by arrows V1 to V3.

As described above, the optically pumped atomic magnetometer detects a magnetic field in a direction substantially perpendicular to the incident direction of laser beam. Therefore, to incorporate an optically pumped atomic magnetometer in the sensor modules 510_1 to 510_3, the optically pumped atomic magnetometer is preferably disposed such that the $Y_0 Z_0$ plane of the optically pumped atomic magnetometer is parallel to the yz plane of the measuring unit 110a.

In other words, to incorporate an optically pumped atomic magnetometer in the sensor modules 510_1 to 510_3, the optically pumped atomic magnetometer is preferably disposed such that the incident direction of the laser beam is substantially parallel to the x-axis direction of the measuring unit 110a.

Thus, the sensor modules 510_1 to 510_3 may have a high sensitivity for detecting the magnetic field generated due to the current flowing through the muscle fiber 255 of the palm 220 in a state where the palm 220 is placed at a predetermined position.

Next, the magnetic field shield characteristics of the flexible film 621 are described. FIG. 14 is a diagram illustrating an example of the magnetic field shield characteristics of the flexible film 621. In FIG. 14, the horizontal axis represents a frequency, and the vertical axis represents the permeability of a magnetic field.

As illustrated in FIG. 14, the flexible film 621 secured to the boundary member 620 is configured to allow the permeability of a magnetic field having a frequency band higher than 1 [Hz] (having a filtering function to shield a magnetic field having a frequency band from 0.01 [Hz] to 1 [Hz]). Thus, the sensor modules 510_1 to 510_3 have a high sensitivity for detecting a magnetic field (100 [Hz] or more) generated due to the current flowing through the muscle fiber 255 of the palm 220 even when the sensor modules 510_1 to 510_3 are in contact with the subject 200 via the flexible film 621.

Next, the opening/closing mechanism 602 disposed around the opening 601 (the opening 601 through which the subject 200 inserts the arm into the first space 610) in the shielding member 600 is described.

FIGS. 15A and 15B are first diagrams illustrating a function of the opening/closing mechanism 602 and illustrating a situation when the measuring unit 110a is viewed in the x-axis direction. FIG. 15A illustrates a case where the opening/closing mechanism 602 is in an opened state. As illustrated in FIG. 15A, in a case where the opening/closing mechanism 602 is in an opened state, the insertion opening 1220, through which the arm of the subject 200 is inserted, has more than a certain size with respect to the opening 601 provided in the shielding member 600. This allows the subject 200 to easily insert the arm into the first space 610.

On the other hand, FIG. 15B illustrates a case where the opening/closing mechanism 602 is in a closed state. As illustrated in FIG. 15B, in a case where the opening/closing mechanism 602 is in a closed state, the insertion opening 1220 is narrower with respect to the opening 601 provided in the shielding member 600. This allows the first space 610 to be sealed in a state where the subject 200 has inserted the arm into the first space 610.

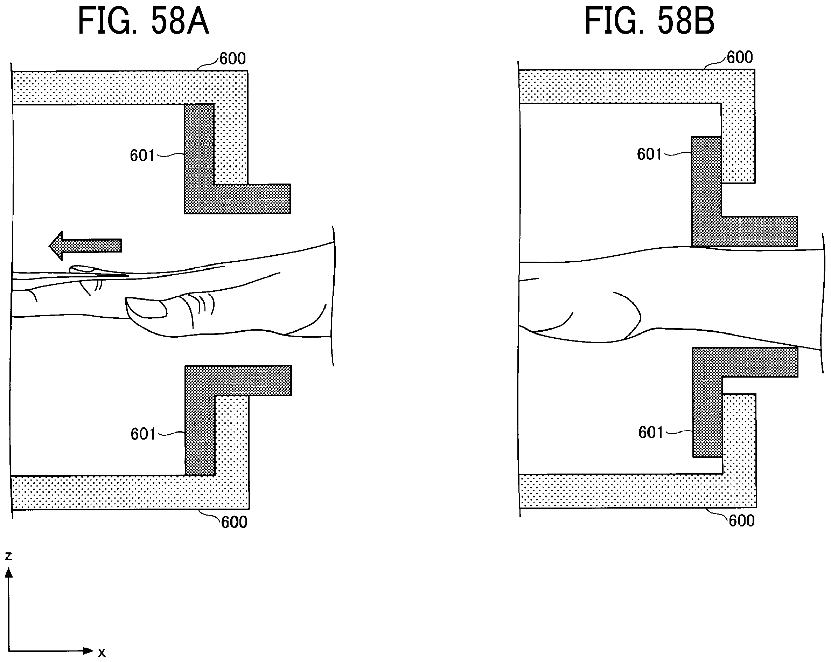

FIGS. 58A and 58B are second diagrams illustrating a function of the opening/closing mechanism 602 and illustrating a situation when the measuring unit 110a is viewed in the y-axis direction. FIG. 58A illustrates a situation where the opening/closing mechanism 602 is in an opened state and the subject 200 is inserting the arm.

On the other hand, FIG. 58B illustrates a situation where the opening/closing mechanism 602 is in a closed state and the subject 200 has inserted the arm. As illustrated in FIG. 58B, the opening/closing mechanism 602 holds part of the arm of the subject 200 while in the closed state. It is preferable that the part held by the opening/closing mechanism 602 is, among various sites of the subject 200, a site (e.g., wrist) that is made up of bones at a higher percentage as compared with a muscle. This is because the magnetic permeability is higher in a site made up of bones at a higher percentage as compared with a muscle.

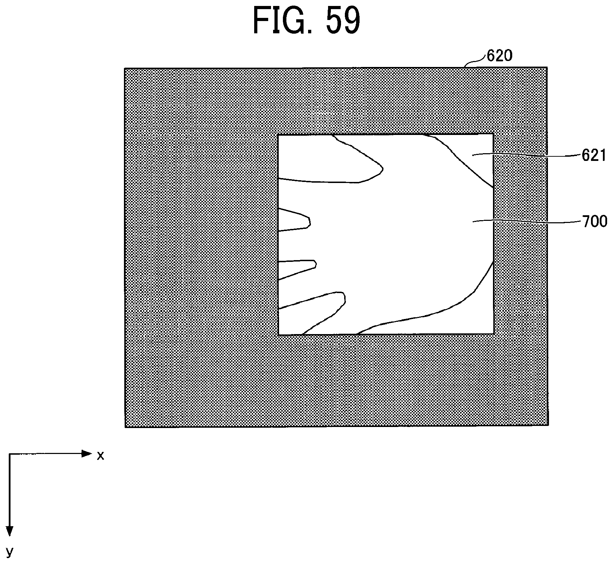

Next, an example of capturing a palm 700 with the camera 511 provided in the measuring unit 110a is described. FIG. 59 is a diagram illustrating an example of capturing with the camera 511. As illustrated in FIG. 55, the camera 511 is provided under the opening of the boundary member 620 in the second space 630. The camera 511 captures the palm 700 in a state where the palm 700 is placed at the position where the flexible film 621 is fixedly mounted.

Thus, the camera 511 may capture part of the palm 700 located at the opening of the boundary member 620 through the flexible film 621 (see FIG. 59).

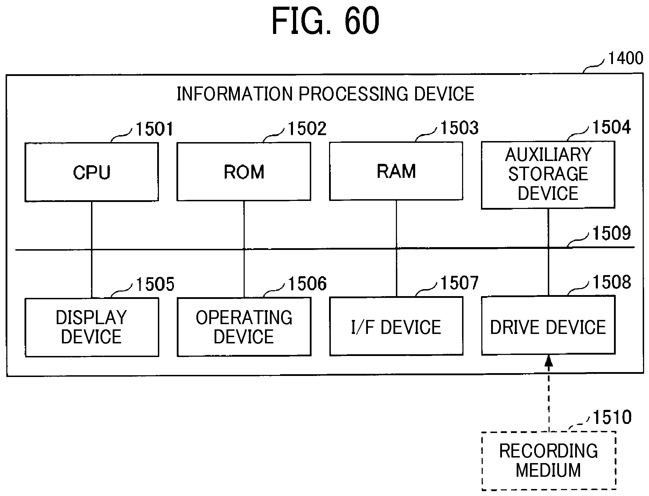

Next, the information processing device 1400 is described. FIG. 60 is a diagram illustrating an example of the hardware configuration of the information processing device 1400. As illustrated in FIG. 60, the information processing device 1400 includes a central processing unit (CPU) 1501, a read only memory (ROM) 1502, and a random access memory (RAM) 1503. The CPU 1501, the ROM 1502, and the RAM 1503 form what is called a computer.

The information processing device 1400 includes an auxiliary storage device 1504, a display device 1505, an operating device 1506, an interface (I/F) device 1507, and a drive device 1508. The hardware devices of the information processing device 1400 are coupled to one another via a bus 1509.

The CPU 1501 is an arithmetic device that executes various programs (e.g., programs (referred to as information processing programs) for implementing the signal processor 5400, the controller 542, and the display controller 543 described above) installed in the auxiliary storage device 1504.

The ROM 1502 is a non-volatile memory. The ROM 1502 functions as a primary memory device that stores various programs, data, and the like, for the CPU 1501 to execute various programs installed in the auxiliary storage device 1504. Specifically, the ROM 1502 functions as a primary memory device that stores boot programs such as Basic Input/Output System (BIOS) or Extensible Firmware Interface (EFI), and the like.

The RAM 1503 is a volatile memory such as a dynamic random access memory (DRAM) or a static random access memory (SRAM). The RAM 1503 functions as a primary memory device that provides a work area that is loaded when various programs installed in the auxiliary storage device 1504 are executed by the CPU 1501.

The auxiliary storage device 1504 is an auxiliary storage device that stores various programs and information acquired when various programs are executed. For example, the data storage 541 is implemented by the auxiliary storage device 1504.

The display device 1505 is a display device that displays various types of image data (the image data 310, the superimposition image data 3200, etc.). The operating device 1506 is an input device that allows the doctor, or the like, to input various instructions to the information processing device 1400. The I/F device 1507 is a communication device that is coupled to the ultrasonic measuring device 120, the detecting device 1100, the electrical stimulation device 130, etc. so that the information processing device 1400 communicates with each device.

The drive device 1508 is a device in which the recording medium 1510 is set. The recording medium 1510 described here includes a medium that records information optically, electrically, or magnetically, such as a CD-ROM, a flexible disk, or a magnetooptical disk. The recording medium 1510 may include a semiconductor memory that electrically records information, such as a ROM or a flash memory.

Various programs installed in the auxiliary storage device 1504 are installed when, for example, the distributed recording medium 1510 is set in the drive device 1508 and various programs recorded in the recording medium 1510 are read. Alternatively, various programs installed in the auxiliary storage device 1504 may be installed by being downloaded via the network.

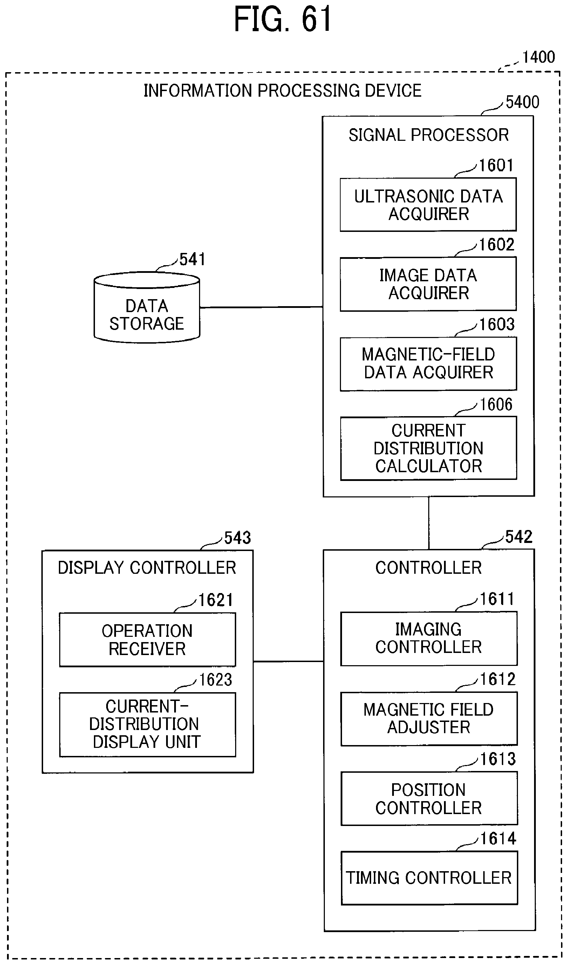

Next, a functional configuration of the information processing device 1400 is described in detail. FIG. 61 is a diagram illustrating details of the functional configuration of the information processing device 1400.

As illustrated in FIG. 61, the signal processor 5400 includes the ultrasonic data acquirer 1601, the image data acquirer 1602, the magnetic-field data acquirer 1603, and a current distribution calculator 1606.

The ultrasonic data acquirer 1601 acquires the ultrasonic data transmitted from the ultrasonic measuring unit 520, calculates the position of each tissue in the palm of the subject 200 based on the acquired ultrasonic data, and stores the position in the data storage 541.

The image data acquirer 1602 acquires the image data transmitted from the camera 511 and stores the acquired image data in the data storage 541.

The magnetic-field data acquirer 1603 acquires the magnetic-field data transmitted from the sensor modules 510_1 to 510_3 and stores the magnetic-field data in the data storage 541.

The current distribution calculator 1606 calculates the current distribution of the palm using the position of each tissue in the palm, stored in the data storage 541, and the magnetic-field data. The current distribution calculator 1606 stores the calculated current distribution data in the data storage 541.

As illustrated in FIG. 61, the controller 542 includes the imaging controller 1611, the magnetic field adjuster 1612, a position controller 1613, and the timing controller 1614.

The imaging controller 1611 transmits an imaging instruction to the camera 511 in response to a received imaging instruction from the operation receiver 1621 of the display controller 543.

After receiving an instruction for starting the current distribution generation from the operation receiver 1621 of the display controller 543, the magnetic field adjuster 1612 acquires the internal magnetic-field data measured by the MI sensor 512, calculates the current value of the current flowing through the coil 513, and transmits the current value to the current generator 519.

After receiving an instruction for starting the current distribution generation from the operation receiver 1621 of the display controller 543, the position controller 1613 transmits, to the moving unit 521, an instruction for moving the holder 514 to the default position. The position controller 1613 transmits, to the moving unit 521, an instruction for controlling the position of the holder 514 in the x-axis direction. Specifically, the position controller 1613 transmits, to the moving unit 521, an instruction for moving the sensor module 510 to the next position in accordance with the detection of a magnetic field by the sensor modules 510_1 to 510_3 at a predetermined position in the x-axis direction.

The position controller 1613 controls the moving unit 521 so that the holder 514 moves within a predetermined moving range.

After receiving an instruction for starting the current distribution generation from the operation receiver 1621 of the display controller 543, the timing controller 1614 transmits the instruction to the electrical stimulation controller 530 at predetermined timing.

As illustrated in FIG. 61, the display controller 543 includes the operation receiver 1621 and a current-distribution display unit 1623.

In response to an input imaging instruction, the operation receiver 1621 notifies the imaging controller 1611 of the imaging instruction. Furthermore, in response to an instruction for starting the current distribution generation input from the doctor, or the like, the operation receiver 1621 notifies the magnetic field adjuster 1612, the position controller 1613, and the timing controller 1614 of the instruction.

The current-distribution display unit 1623 visualizes the current distribution data stored in the data storage 541, reads the image data stored in the data storage 541, superimposes the visualized current distribution data, and displays the superimposition image data.

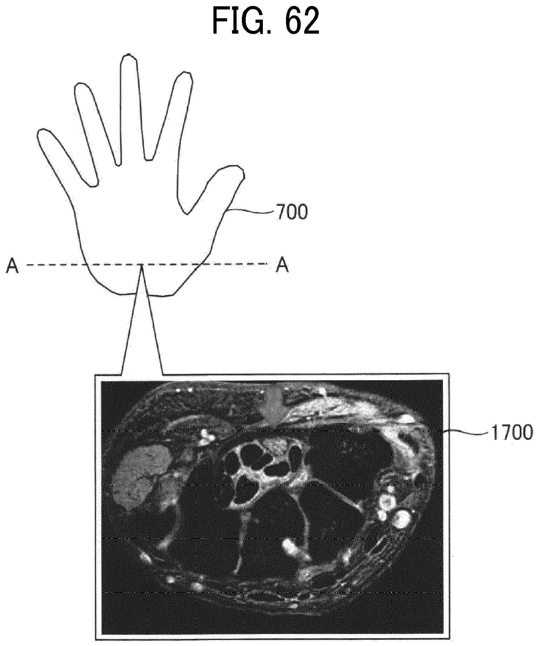

Next, a specific example of the ultrasonic data acquired by the ultrasonic data acquirer 1601 is described. FIG. 62 is a diagram illustrating a specific example of the ultrasonic data. In FIG. 62, ultrasonic data 1700 represents ultrasonic data (the cross-sectional image taken through A-A) measured by the ultrasonic measuring device 120 at the position indicated by the dashed line in the palm 700.

The ultrasonic data acquirer 1601 identifies each tissue in the ultrasonic data 1700 illustrated in FIG. 62 to calculate the position coordinates (three-dimensional position coordinates) of each tissue in the palm 700.

Next, the positional relationship between the position where the electrical stimulation is applied to the subject 200 with the attached input terminal 130b of the electrical stimulation device 130 and the position where the magnetic field is detected by the sensor modules 510_1 to 510_3 is described.

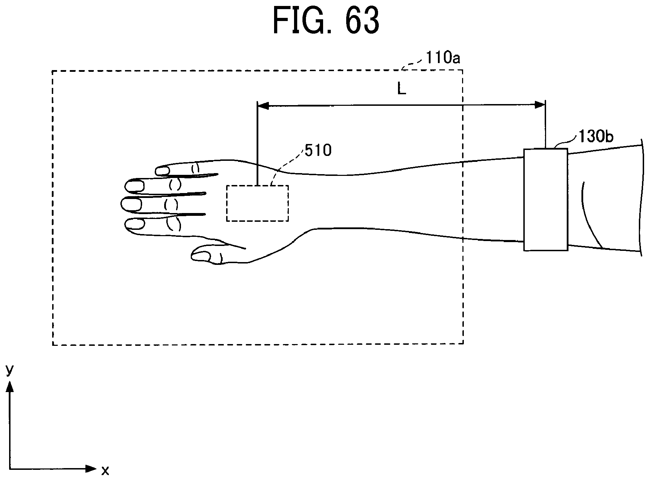

FIG. 63 is a diagram illustrating the positional relationship between the position where the electrical stimulation is applied and the position where the magnetic field is detected. As illustrated in FIG. 63, the input terminal 130b of the electrical stimulation device 130 is attached to a site (a site different from the palm) that is a part of the arm of the subject 200 and is located outside the measuring unit 110a. The reason why electrical stimulation is applied to a site located outside the measuring unit 110a is that the electromagnetic wave generated due to the application of the electrical stimulation is prevented from affecting the sensor modules 510_1 to 510_3.

The palm 700 is placed at a predetermined position in the measuring unit 110a and, at the position, the sensor modules 510_1 to 510_3 detect a magnetic field. For this reason, the position where the electric stimulus is applied is separated from the position where the magnetic field is detected by a distance "L."

In other words, as the input terminal 130b is attached to a site located outside the measuring unit 110a so as to eliminate the effect of the electrical stimulation, there is a time difference corresponding to the distance L from when the electrical stimulus is applied until the sensor modules 510_1 to 510_3 detect a magnetic field.

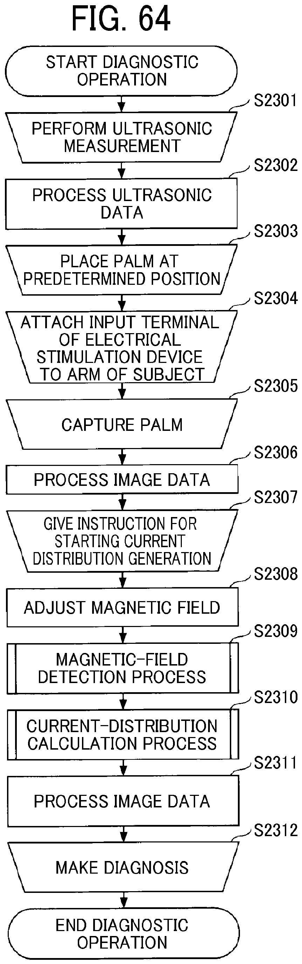

Next, an operation to diagnose carpal tunnel syndrome of the subject 200 with the biomagnetic-field detection apparatus 1011 used by the doctor, or the like, is described. FIG. 64 is a diagram illustrating the flow of an operation to diagnose carpal tunnel syndrome.

At Step S2301, the doctor, or the like, uses the ultrasonic measuring device 120 to perform the ultrasonic measurement on the palm of the subject 200. As a result, the ultrasonic measuring device 120 transmits the ultrasonic data to the information processing device 1400.

At Step S2302, the ultrasonic data acquirer 1601 of the information processing device 1400 processes the ultrasonic data on the subject 200 and calculates the position of each tissue in the palm of the subject 200.

At Step S2303, the doctor, or the like, prompts the subject 200 to insert the arm into the measuring unit 110a and place the palm of the subject 200 at a predetermined position.

At Step S2304, the doctor, or the like, attaches the input terminal 130b of the electrical stimulation device 130 to the arm of the subject 200.

At Step S2305, the doctor, or the like, operates the information processing device 1400 to input an imaging instruction and drive the camera 511. Thus, the camera 511 captures the palm of the subject 200 placed at the predetermined position and transmits the image data to the information processing device 1400.

At Step S2306, the image data acquirer 1602 of the information processing device 1400 acquires the image data on the palm of the subject 200.

At Step S2307, the doctor, or the like, operates the information processing device 1400 to input an instruction for starting the current distribution generation.

At Step S2308, the magnetic field adjuster 1612 acquires the internal magnetic-field data measured by the MI sensor 512 and calculates the current value. The current generator 519 applies the current with the calculated current value to the coil 513 to set the magnetic field inside the measuring unit 110a to, for example, 50 nT or less.

At Step S2309, each unit of the biomagnetic-field detection apparatus 1011 performs a magnetic-field detection process. The details of the magnetic-field detection process are given later.

At Step S2310, the information processing device 1400 performs a current-distribution calculation process. The details of the current-distribution calculation process are given later.

At Step S2311, the current-distribution display unit 1623 visualizes the current distribution data on the palm of the subject 200, superimposes the current distribution data on the image data, and displays the superimposition image data.

At Step S2312, the doctor, or the like, diagnoses carpal tunnel syndrome of the subject 200 based on the superimposition image data on which the visualized current distribution data is superimposed.

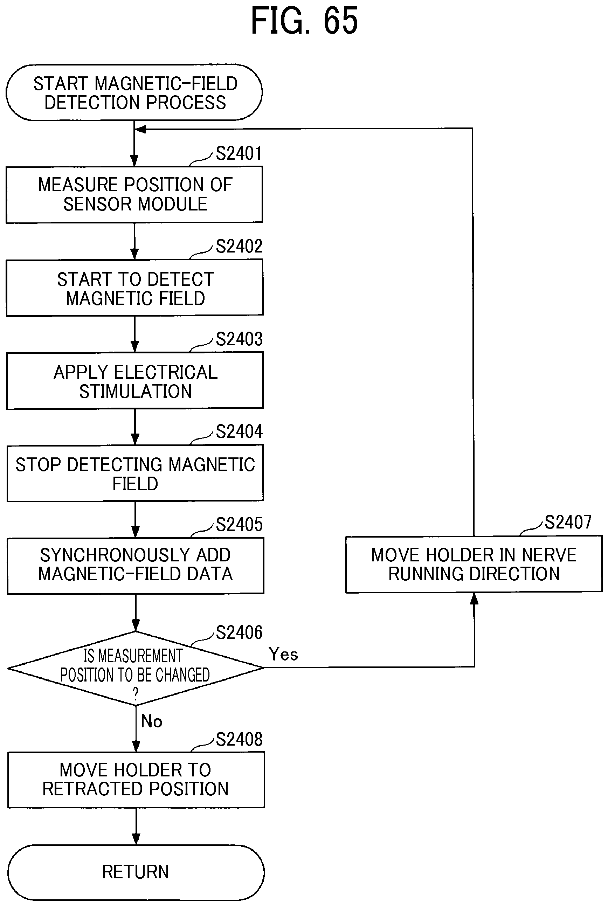

Next, the magnetic-field detection process (Step S2309) is described in detail. FIG. 65 is a flowchart illustrating the flow of the magnetic-field detection process.

At Step S2401, the position sensors built in the sensor modules 510_1 to 510_3 measure the current positions of the sensor modules 510_1 to 510_3 and acquire the position data.

At Step S2402, each of the sensor modules 510_1 to 510_3 starts to detect a magnetic field.

At Step S2403, the electrical stimulation device 130 applies electrical stimulation to the subject 200.

At Step S2404, the sensor modules 510_1 to 510_3 stop detecting a magnetic field, and the magnetic-field data acquirer 1603 acquires the magnetic-field data.

At Step S2405, the magnetic-field data acquirer 1603 stores the acquired magnetic-field data together with the position data in the data storage 541. At this point, the magnetic-field data acquirer 1603 synchronously adds the currently acquired magnetic-field data to the already acquired magnetic-field data.

At Step S2406, the position controller 1613 determines whether the holder 514 is to be moved. It is determined that the holder 514 is to be moved (Yes at Step S2406) in a case where the holder 514 has not moved in a predetermined movement range, and the process proceeds to Step S2407.

At Step S2407, the position controller 1613 moves the holder 514 by a predetermined distance (e.g., 5 [mm]) in the x-axis direction (the direction (the longitudinal direction) in which a nerve of the palm of the subject 200 runs), and the process returns to Step S2401.

Conversely, at Step S2406, when it is determined that the holder 514 is not to be moved (No at Step S2406) in a case where the holder 514 has moved in a predetermined movement range, the process proceeds to Step S2408.

At Step S2408, the position controller 1613 moves the holder 514 to a retracted position, and then the process returns to Step S2310.

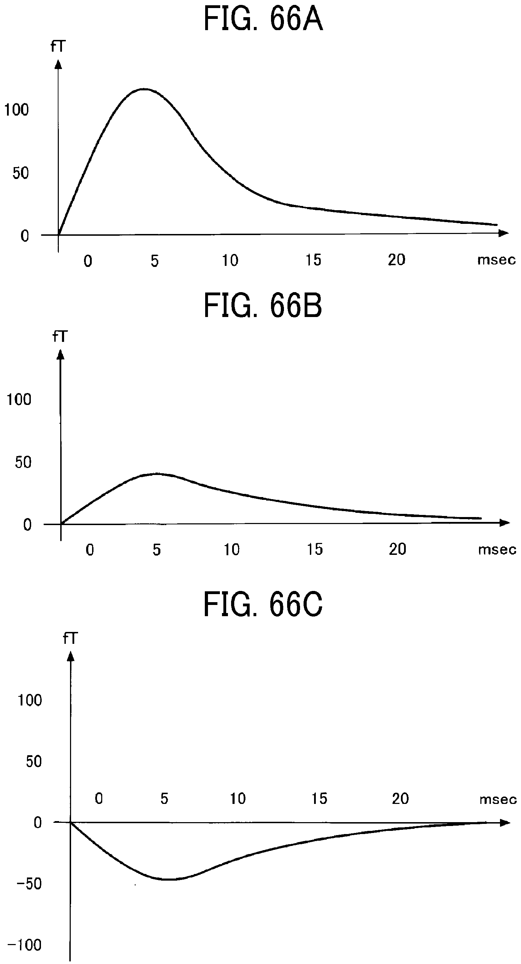

Next, a specific example of the magnetic-field data acquired by the magnetic-field data acquirer1603 after the magnetic-field detection process (Step S2309) is performed is described. FIGS. 66A, 66B, and 66C are graphs illustrating specific examples of the magnetic-field data. In FIGS. 66A, 66B, and 66C, the horizontal axis indicates a time, and the vertical axis indicates a magnetic flux density. In the horizontal axis, 0 [msec] represents the time when the electrical stimulation device 130 applies electrical stimulation to the subject 200.

According to the present embodiment, the sampling period of the optically pumped atomic magnetometer is 5 [msec]. Furthermore, according to the present embodiment, the electrical stimulation applied by the electrical stimulation device 130 is 10 [Hz].

As illustrated in FIGS. 66A, 66B, and 66C, the magnetic flux density reaches its peak at the time in the vicinity of 5 to 10 [msec] because of the time difference corresponding to the distance L after the electrical stimulation is applied until when the sensor modules 510_1 to 510_3 detect a magnetic field. In other words, each time in the horizontal axis of the magnetic-field data corresponds to each position of the palm in the x-axis direction (the position data measured by the position sensor).

The magnetic-field data acquirer 1603 synchronously adds the magnetic-field data detected at each position within the movement range of the holder 514 in such a manner that the timings of application of the electrical stimulation are matched. Thus, it is possible to generate the magnetic-field data in which the magnetic flux density at each time is more than a predetermined value, and it is possible to improve the spatial resolution to calculate a current distribution.

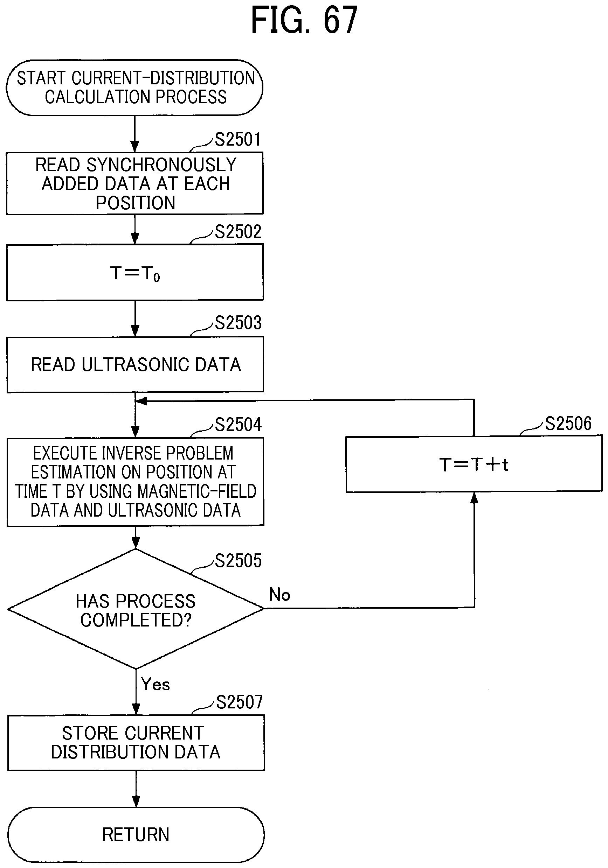

Next, the current-distribution calculation process (Step S2310) is described in detail. FIG. 67 is a flowchart illustrating the flow of the current-distribution calculation process.

At Step S2501, the current distribution calculator 1606 reads the synchronously added magnetic-field data from the data storage 541. The current distribution calculator 1606 also reads the position data corresponding to each time in the magnetic-field data.

At Step S2502, the current distribution calculator 1606 inputs "T0" to time T. "T0" corresponds to the position (distribution-calculation start position) closest to the position where the electrical stimulation is applied in the range in the x-axis direction for calculating a current distribution. Specifically, the time "T0" corresponds to the time it takes for the current to flow in the distance from the position where the electrical stimulation is applied to the distribution-calculation start position.

At Step S2503, the current distribution calculator 1606 reads, from the data storage 541, the position of each tissue in the palm of the subject 200 calculated based on the ultrasonic data.

At Step S2504, the current distribution calculator 1606 executes inverse problem estimation based on the magnetic-field data and the position of each tissue.

Here, the position (three-dimensional position coordinates) of a nerve out of the positions of tissues is rs, the position data (three-dimensional position coordinates) of the optically pumped atomic magnetometer built in the sensor module 510 is rd, and the magnetic flux density at the time T is ø(rd). In this case, the magnetic flux density ø(rd) may be expressed as below by using the current value ø(rs) of the current flowing through the nerve.

### First Equation

$$\emptyset(rd) = A(r) \times \emptyset(rs)$$

Here, A(r) is the sensitivity distribution at a three-dimensional position coordinate r. The current distribution calculator 1606 assumes the permeability distribution from the position of each tissue in the palm and the permeability of each tissue to perform simulation so as to obtain the magnetic field distribution when the current flows through the nerve in the palm. Thus, the current distribution calculator 1606 may obtain the sensitivity distribution A(r).

The current distribution calculator 1606 calculates the current value ø(rs) by using the obtained sensitivity distribution A(r) and the magnetic flux density ø(rd) to estimate the current distribution. To estimate the current distribution, the current distribution calculator 1606 uses an inverse-problem estimation method that is called L2 norm regularization.

At Step S2505, the current distribution calculator 1606 determines whether the current distribution has been calculated for the entire range (each position from the distribution-calculation start position to the distribution-calculation end position) for calculating the current distribution in the x-axis direction. When it is determined at Step S2505 that there is a position for which the current distribution has not been calculated (No at Step S2505), the process proceeds to Step S2506.

At Step S2506, the current distribution calculator 1606 adds a calculation interval t to the time T, and then the process returns to Step S2504. The calculation interval t is, for example, 5 [msec].

Conversely, when it is determined at Step S2505 that the current distribution has been calculated for the entire range for calculating the current distribution in the x-axis direction (Yes at Step S2505), the process proceeds to Step S2507.

At Step S2507, the current distribution calculator 1606 stores the calculated current distribution data in the data storage 541.

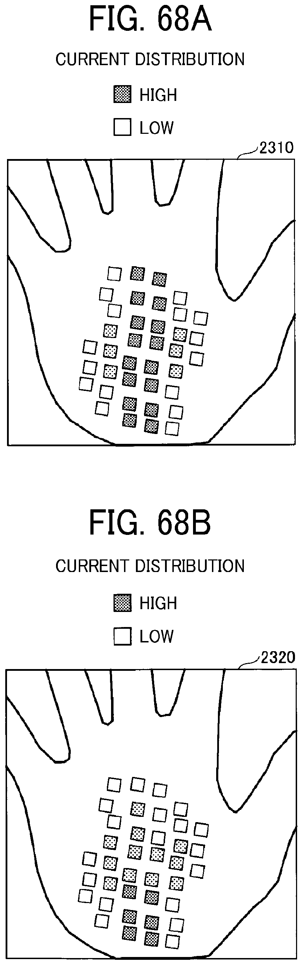

Next, an example of the display of the current distribution by the current-distribution display unit 1623 is described. FIGS. 68A and 68B are first diagrams illustrating examples of the display of the current distribution. As described above, the current-distribution display unit 1623 visualizes the current distribution data stored in the data storage 541, reads the image data stored in the data storage 541, superimposes the image data on the visualized current distribution data, and displays the superimposition image data.

FIG. 68A illustrates the current distribution in a case where the nerve of the carpal tunnel in the palm of the subject 200 is normal. As illustrated in FIG. 68A, the current-distribution display unit 1623 represents the position where the current distribution data is calculated as the position of the rectangle having the size corresponding to the spatial resolution and represents the inside of the rectangle in the color corresponding to the size of the current distribution data to visualize the current distribution data.

The current-distribution display unit 1623 superimposes the visualized current distribution data on image data 2310 and displays the superimposition image data. This allows the doctor, or the like, to visually recognize the image data 2310 and therefore determine that a strong current flows along the carpal tunnel in the center portion of the palm. As a result, the doctor, or the like, may make a diagnosis that the nerve of the carpal tunnel in the palm of the subject 200 is normal.

FIG. 68B illustrates the current distribution in a case where the nerve of the carpal canal in the palm of the subject 200 is abnormal. The doctor, or the like, may visually recognize the image data 2320 and therefore determine that the current has weakened in the middle of the carpal tunnel at the center portion of the palm. As a result, the doctor, or the like, may make a diagnosis that the nerve of the carpal tunnel in the palm of the subject 200 is abnormal.

It is understood from the above description that the biomagnetic-field detection apparatus 1011 according to the first embodiment of the second group of embodiments includes the shielding member 600 that covers the space around the palm of the subject to shield electromagnetic waves. The biomagnetic-field detection apparatus 1011 includes the sensor modules 510_1 to 510_3 including the optically pumped atomic magnetometers that detect a magnetic field generated in the palm of the subject in a state where the surrounding space is covered with the shielding member 600. In the sensor modules 510_1 to 510_3, an optically pumped atomic magnetometer is disposed in the direction (the direction in which the nerve running direction matches the light propagation direction) corresponding to the direction toward the end of the palm (the direction in which the nerve runs).

Thus, with the biomagnetic-field detection apparatus 1011 according to the first embodiment of the second group of embodiments, a magnetic field generated in the palm of the subject may be detected, and superimposition image data may be displayed to make a diagnosis as to whether the carpal tunnel nerve is normal.

SECOND EMBODIMENT OF SECOND GROUP OF EMBODIMENTS

According to the above first embodiment of the second group of embodiments, the three sensor modules are provided in the y-axis direction in the holder 514 of the measuring unit 110a. However, this is not a limitation on the number of sensor modules provided in the holder 514. For example, a plurality of sensor modules may be provided in the x-axis direction as well as in the y-axis direction. Providing the sensor modules in the x-axis direction makes it possible to reduce the number of times the holder 514 is moved in the x-axis direction. A second embodiment of the second group of embodiments is described below, focusing on a difference from the above-described first embodiment of the second group of embodiments.

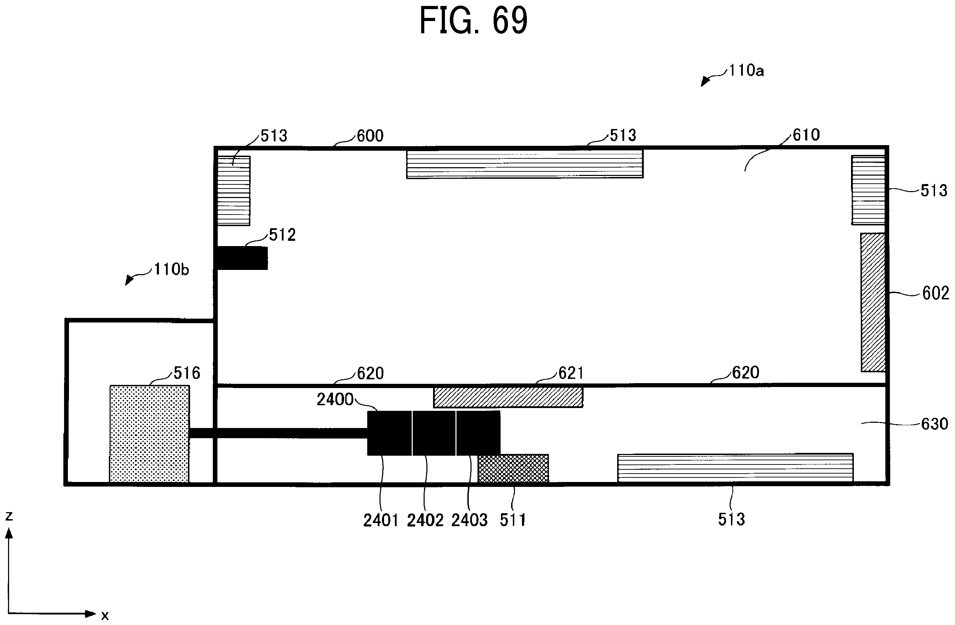

First, internal configurations of the measuring unit 110a and the drive 110b are described. FIG. 69 is a second diagram illustrating the internal configurations of the measuring unit 110a and the drive 110b. FIG. 69 is different from FIG. 55 in a holder 2400. In the case of FIG. 69, in the holder 2400, three sensor modules are arranged in the y-axis direction, and the sensor modules are arranged in three rows (see the reference numerals 2401 to 2403) in the x-axis direction.

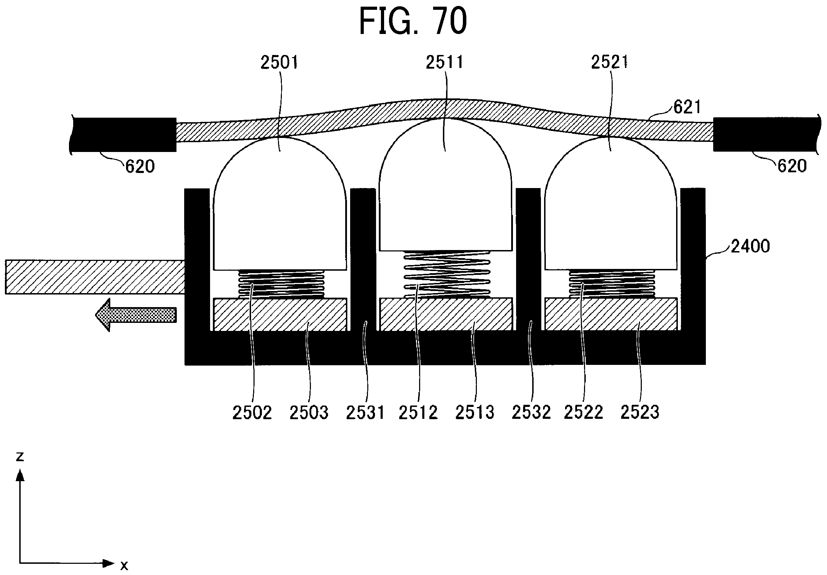

FIG. 70 is a second diagram illustrating a detailed configuration of the holder 2400. As illustrated in FIG. 70, in the holder 2400, sensor modules 2501, 2511 and 2521, arranged in the x-axis direction, are supported by support bases 2503, 2513, and 2523 via elastic members 2502, 2512, and 2522, respectively. The support bases 2503, 2513, and 2523 are fixedly mounted in the holder 2400.

The sensor modules 2501, 2511, and 2521 are separated by permalloy partition walls 2531 and 2532 to prevent crosstalk between the sensor modules 2501, 2511, and 2521.

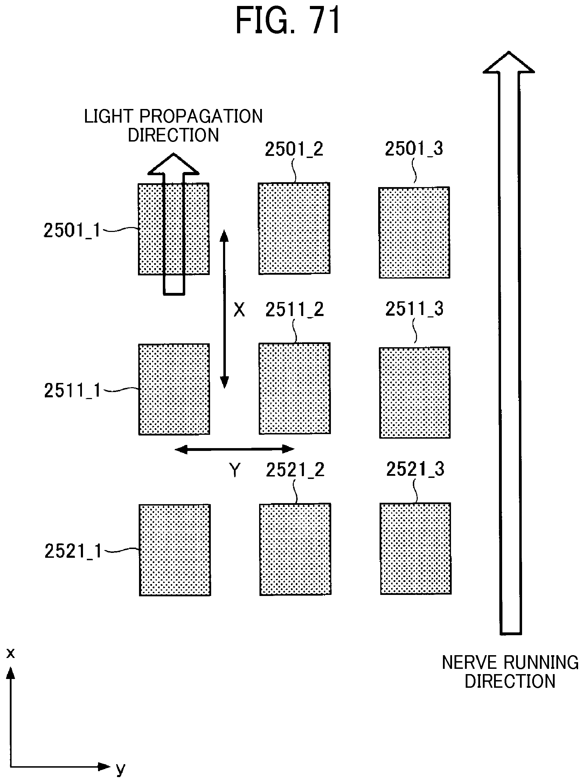

FIG. 71 is a diagram illustrating an example of the arrangement of sensor modules 2501_1 to 2521_3 in the holder 2400 and illustrating a schematic view of the holder 2400 from above.

It is assumed that the nine sensor modules 2501 1 to 2521 3 illustrated in FIG. 71 are all provided such that the incident direction (light propagation direction) of the laser beam of the built-in optically pumped atomic magnetometer is substantially parallel to the x-axis direction of the measuring unit 110a. Thus, the incident direction of the laser beam may match the running direction of the nerve of the subject 200, and the magnetic field generated due to the current flowing through the nerve of the palm 700 may be detected with high sensitivity.

The sensor modules 2501_1 to 2521_3 are provided such that the distance between adjacent sensor modules in the x-axis direction is larger than the distance between adjacent sensor modules in the y-axis direction. For example, a distance X between the sensor module 2501_1 and the sensor module 2511_1 in the x-axis direction is larger than a distance Y between the sensor module 2501_1 and the sensor module 2501_2 in the y-axis direction. That is, each sensor module is provided such that X>Y with respect to adjacent sensor modules.

This is because, generally, the crosstalk in a direction substantially parallel to the incident direction of the laser beam is larger than the crosstalk in a direction substantially perpendicular to the incident direction of the laser beam.

It is understood from the above description that the biomagnetic-field detection apparatus 1011 according to the second embodiment of the second group of embodiments includes the shielding member 600 that covers the space around the palm of the subject to shield electromagnetic waves. The biomagnetic-field detection apparatus 1011 includes the sensor modules 2501_1 to 2521_3 including the optically pumped atomic magnetometers that detect a magnetic field generated in the palm of the subject in a state where the surrounding space is covered with the shielding member 600. In the sensor modules 2501_1 to 2521_3, an optically pumped atomic magnetometer is disposed in the direction (the direction in which the nerve running direction matches the light propagation direction) corresponding to the direction toward the end of the palm (the direction in which the nerve runs).

Thus, with the biomagnetic-field detection apparatus 1011 according to the second embodiment of the second group of embodiments, a magnetic field generated in the palm of the subject may be detected, and superimposition image data may be displayed to make a diagnosis as to whether the carpal tunnel nerve is normal.

THIRD EMBODIMENT OF SECOND GROUP OF EMBODIMENTS

According to the first embodiment and the second embodiment of the second group of embodiments described above, the measuring unit 110a is covered with the shielding member 600 to reduce the residual magnetic field in the measuring unit 110a. In a biomagnetic-field detection apparatus 2700 according to a third embodiment of the second group of embodiments, in order to prevent electromagnetic waves from entering through the insertion opening 1220, an auxiliary member covering the opening 601 is attached to the subject 200 to further reduce the residual magnetic field in the measuring unit 110a. The third embodiment of the second group of embodiments is explained below, focusing on a difference from the first embodiment and the second embodiment of the second group of embodiments described above.

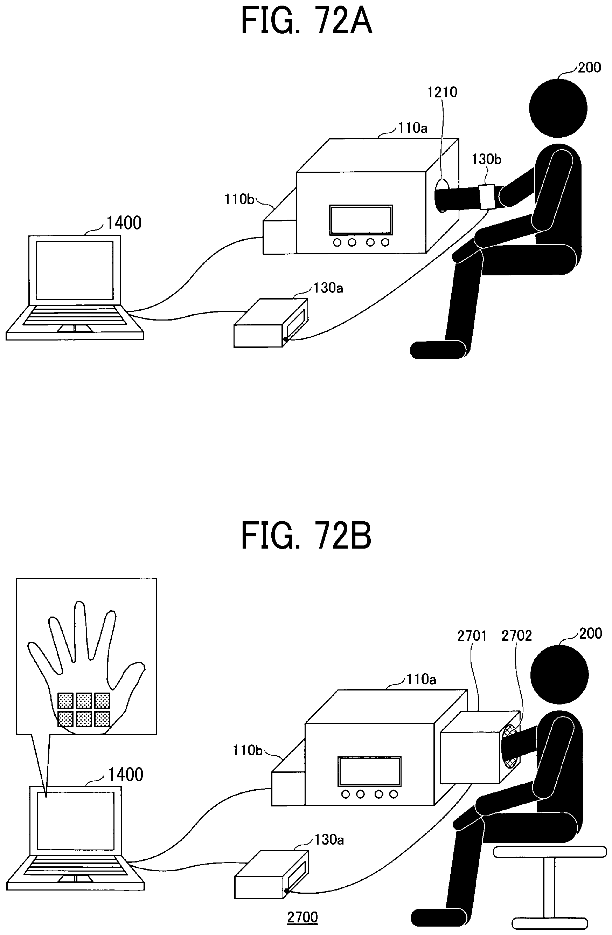

FIGS. 72A and 72B are diagrams illustrating an auxiliary member 2701. FIG. 72A illustrates a situation before the auxiliary member 2701 is attached (illustrates the same situation as that illustrated in FIG. 52B). As illustrated in FIG. 72A, the doctor, or the like, attaches the input terminal 130b to a part near the elbow of the subject 200 and then attaches the auxiliary member 2701 to the subject 200.

FIG. 72B illustrates a situation after the auxiliary member 2701 is attached to the subject 200. As illustrated in FIG. 72B, the doctor, or the like, attaches the auxiliary member 2701 to the upper arm portion of the subject 200. For example, the auxiliary member 2701 is configured to be separated into upper and lower portions so that the upper arm portion is sandwiched between the upper and lower portions while the auxiliary member 2701 is attached to the subject 200. The auxiliary member 2701 may be formed by bending a permalloy flat plate in the same manner as the shielding member 600.

Thus, as the opening 601 of the measuring unit 110a is covered with the auxiliary member 2701, electromagnetic waves may be prevented from entering through the insertion opening 1220, whereby the residual magnetic field in the measuring unit 110a may be further reduced.

A sponge fixing member 2702 is provided inside the auxiliary member 2701. When the auxiliary member 2701 is attached to the upper arm portion of the subject 200, the sponge fixing member 2702 is pressed against the upper arm portion. This prevents the body movement of the subject 200.

The fixing member 2702 is formed in a sponge by knitting chemical fibers containing an amorphous metal. With the use of chemical fibers containing an amorphous metal for the fixing member 2702, the residual magnetic field in the measuring unit 110a may be reduced.

It is understood from the above description that the biomagnetic-field detection apparatus 2700 according to the third embodiment of the second group of embodiments includes the auxiliary member 2701 in addition to the biomagnetic-field detection apparatus 1011 according to the first embodiment and the second embodiment of the second group of embodiments described above. Thus, with the biomagnetic-field detection apparatus 2700 according to the third embodiment of the second group of embodiments, the residual magnetic field in the measuring unit 110a may be further reduced in addition to the effect of the biomagnetic-field detection apparatus 1011 according to the first embodiment and the second embodiment of the second group of embodiments described above.

Although the auxiliary member 2701 includes a permalloy flat plate according to the above-described third embodiment of the second group of embodiments, the material of the auxiliary member 2701 is not limited thereto. For example, the auxiliary member may be a sheet containing PET (polyethylene terephthalate) as its base material and wound around the upper arm portion of the subject 200.

FOURTH EMBODIMENT OF SECOND GROUP OF EMBODIMENTS

In the case described according to the first embodiment to the third embodiment of the second group of embodiments described above, the object under examination is the palm, which is a part of a limb of the subject 200, when the biomagnetic-field detection apparatus detects a magnetic field. However, the object under examination for which a magnetic field is detected is not limited to the palm of the subject 200. For example, a leg that is a part of a limb of the subject 200 may be the object under examination to detect a magnetic field. A fourth embodiment of the second group of embodiments is described below, focusing on a difference from the first embodiment to the third embodiment of the second group of embodiments described above.

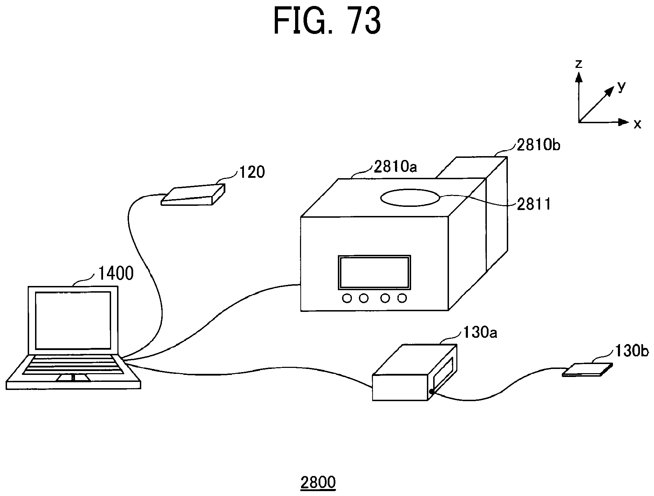

First, an external configuration of a biomagnetic-field detection apparatus 2800 according to the fourth embodiment of the second group of embodiments is described. FIG. 73 is a second diagram illustrating the external configuration of the biomagnetic-field detection apparatus 2800. The biomagnetic-field detection apparatus 2800 is different from the biomagnetic-field detection apparatus 1011 according to the first embodiment of the second group of embodiments described referring to FIG. 50 in configurations of a measuring unit 2810a and a drive 2810b.

As illustrated in FIG. 73, in the case of the measuring unit 2810a of the biomagnetic-field detection apparatus 2800, an opening 2811 for inserting the leg, which is the object under examination, is provided on the upper surface of the measuring unit 2810a. Although not illustrated in FIG. 73, as the opening 2811 is provided on the upper surface of the measuring unit 2810a, each unit in the measuring unit 2810a is also provided at the position corresponding to the direction of the leg that is the object under examination.

Also, the drive 2810b of the biomagnetic-field detection apparatus 2800 is provided along the z-axis direction (not the y-axis direction) to move the holder in the measuring unit 2810a in the z-axis direction.

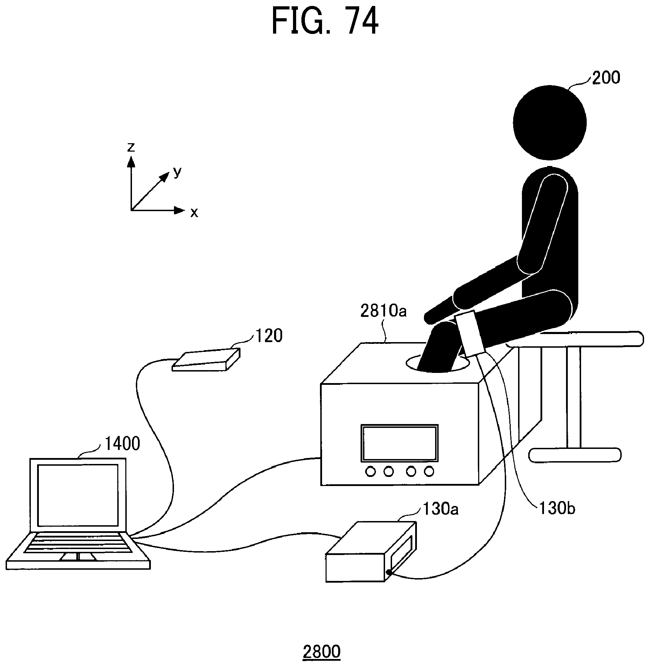

Next, part of the flow of an operation to diagnose a neurological disorder of the leg of the subject 200 using the biomagnetic-field detection apparatus 2800 according to the fourth embodiment of the second group of embodiments is described. FIG. 74 is a diagram illustrating part of the flow of an operation to diagnose the neurological disorder of the leg.

As illustrated in FIG. 74, to diagnose the neurological disorder of the leg of the subject 200, the leg of the subject 200 is inserted into the measuring unit 2810a through the opening 2811 provided on the upper surface of the measuring unit 2810a. The input terminal 130b is attached to the thigh (a site located outside the measuring unit 2810a and different from the leg that is the object under examination) of the subject 200. Thus, the electrical stimulation is applied to at least one of the sural nerve and the tibial nerve.

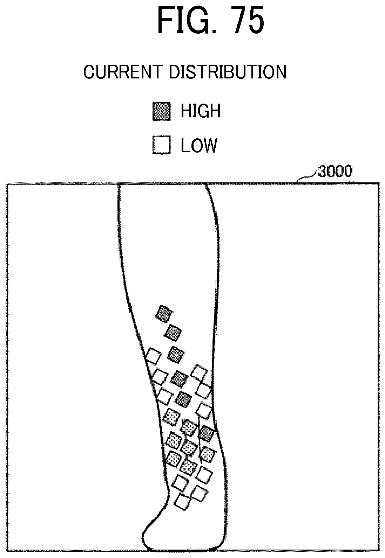

Next, a display example of the current distribution displayed on the biomagnetic-field detection apparatus 2800 according to the fourth embodiment of the second group of embodiments is described. FIG. 75 is a second diagram illustrating a display example of the current distribution. As is the case with the biomagnetic-field detection apparatus 1011 according to the first embodiment of the second group of embodiments, the current distribution data stored in the data storage 541 is visualized and displayed on the image data (here, the image data on the leg) stored in the data storage 541 in a superimposed manner.

The example of FIG. 75 illustrates the current distribution in a case where the nerve in the leg of the subject 200 is abnormal. The doctor, or the like, visually recognizes superimposition image data 3000 to determine whether the current is weak in the middle of the leg of the subject 200. As a result, the doctor, or the like, may make a diagnosis that the nerve in the leg of the subject 200 is abnormal.

It is understood from the above description that the biomagnetic-field detection apparatus 2800 according to the fourth embodiment of the second group of embodiments includes the shielding member that covers the space around the leg of the subject to shield electromagnetic waves. The biomagnetic-field detection apparatus 2800 includes the sensor module including the optically pumped atomic magnetometer that detects a magnetic field generated in the leg of the subject in a state where the surrounding space is covered with the shielding member. In the sensor module, the optically pumped atomic magnetometer is disposed in the direction (the direction in which the nerve running direction matches the light propagation direction) corresponding to the direction toward the end of the leg (the direction in which the nerve runs).

Thus, with the biomagnetic-field detection apparatus 2800 according to the fourth embodiment of the second group of embodiments, a magnetic field generated in the leg of the subject may be detected, and superimposition image data may be displayed to make a diagnosis as to whether the nerve of the leg is normal.

FIFTH EMBODIMENT OF SECOND GROUP OF EMBODIMENTS

In the description according to the first embodiment to the fourth embodiment of the second group of embodiments described above, the sensor module is in contact with part of the limb of the subject 200 in a specific direction around the circumference of the part. Conversely, in the description according to a fifth embodiment of the second group of embodiments, the sensor module is in contact with the entire circumference of part of the limb of the subject 200. The fifth embodiment of the second group of embodiments is described, focusing on a difference from the first embodiment to the fourth embodiment of the second group of embodiments. In the case described according to the fifth embodiment of the second group of embodiments, the object under examination is the elbow of the subject 200.

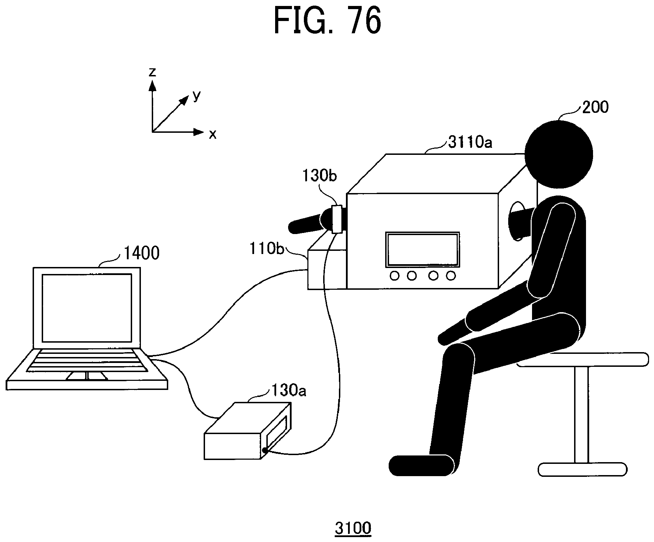

First, a part of the flow of the operation to diagnose the neurological disorder (e.g., cubital tubular syndrome) of the subject 200 by using the biomagnetic-field detection apparatus according to the fifth embodiment of the second group of embodiments is described. FIG. 76 is a diagram illustrating a part of the flow of the operation to diagnose the neurological disorder of the elbow.

As illustrated in FIG. 76, to diagnose a neurological disorder of the subject 200, the arm of the subject 200 is inserted into a measuring unit 3110a through an opening of the measuring unit 3110a. The measuring unit 3110a is also provided with an opening on the surface opposite to the surface where the opening for inserting the arm is provided. A part from the wrist to the finger tip of the arm inserted through one of the openings of the measuring unit 3110a is exposed to outside through the other one of the openings. Thus, the elbow, which is the object under examination, is placed at a predetermined position of the measuring unit 3110a.

As illustrated in FIG. 76, the input terminal 130b is attached to the wrist of the subject 200 (a site located outside the measuring unit 3110a and different from the elbow that is the object under examination).

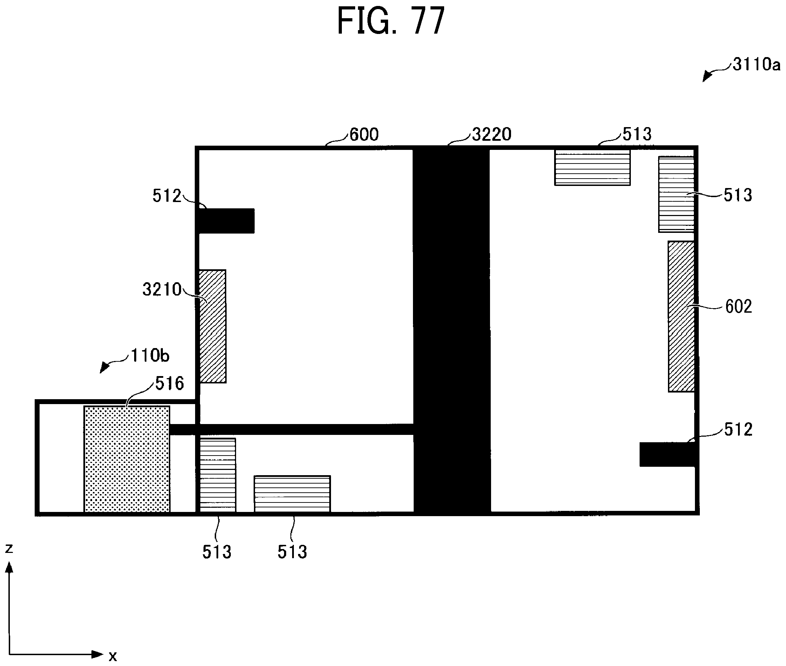

Next, internal configurations of the measuring unit 3110a and the drive 110b are described. FIG. 77 is a third diagram illustrating the internal configurations of the measuring unit 3110a and the drive 110b. The measuring unit 3110a and the drive 110b are different from the measuring unit 110a and the drive 110b of the biomagnetic-field detection apparatus 1011 according to the first embodiment of the second group of embodiments described referring to FIG. 55 in an opening/closing mechanism 3210 and a holder 3220.

As illustrated in FIG. 77, in the case of the measuring unit 3110a, the opening/closing mechanism 3210 is provided at the position opposed to the opening/closing mechanism 602. Accordingly, the opening/closing mechanism 3210 may hold the wrist in a state where the part from the wrist to the finger tip is exposed to outside through the opening on which the opening/closing mechanism 3210 is provided.

In the case of the measuring unit 3110a, the holder 3220 holds sensor modules in the entire circumferential direction so as to detect a magnetic field all around the elbow of the subject 200.

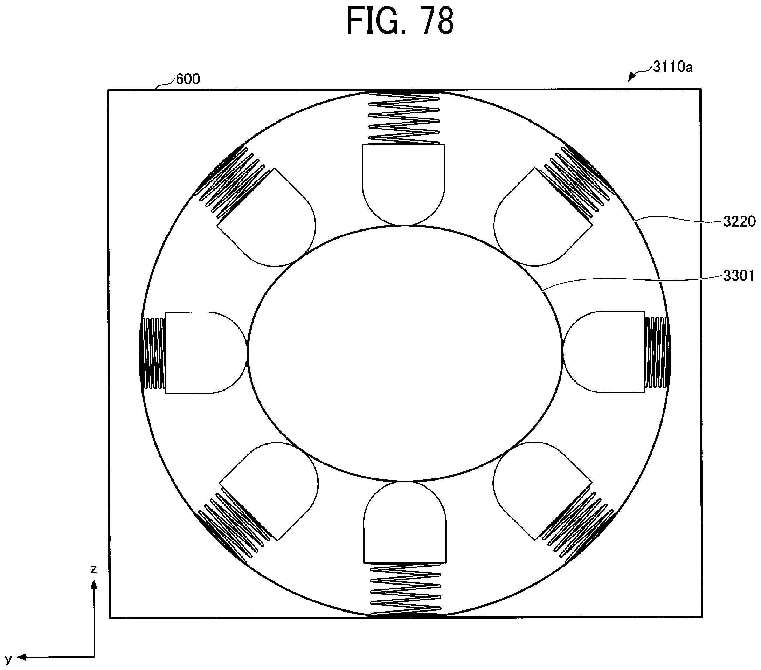

FIG. 78 is a third diagram illustrating a detailed structure of the holder 3220 in the measuring unit 3110a. As illustrated in FIG. 78, the holder 3220 has an annular shape, and the elbow of the subject 200 is placed on an inner circumferential portion 3301. A plurality of sensor modules is fixed to an outer circumferential portion of the holder 3220 via elastic members. The sensor modules are fixedly provided on the outer circumferential portion of the holder 3220 such that the ends of the sensor modules are oriented to the center of the circle.

The sensor modules illustrated in FIG. 78 are all provided such that the incident direction of the laser beam of the built-in optically pumped atomic magnetometer is substantially parallel to the x-axis direction of the measuring unit 3110a. This allows the incident direction of the laser beam to be identical to the running direction of the nerve of the subject 200, and the magnetic field generated due to the current flowing through the nerve of the subject 200 may be detected with high sensitivity.

As a result, as the elbow of the subject 200 is placed on the inner circumferential portion 3301 of the holder 3220, the biomagnetic-field detection apparatus 3100 may detect a magnetic field all around the elbow of the subject 200.

It is understood from the above description that the biomagnetic-field detection apparatus 3100 according to the fifth embodiment of the second group of embodiments includes the shielding member that covers the space around the elbow of the subject to shield electromagnetic waves. The biomagnetic-field detection apparatus 3100 includes the sensor modules including the optically pumped atomic magnetometers provided in the entire circumferential direction to detect a magnetic field generated all around the elbow of the subject in a state where the surrounding space is covered with the shielding member. In the sensor modules, the optically pumped atomic magnetometer is disposed in the direction (the direction in which the nerve running direction matches the light propagation direction) corresponding to the direction toward the end of the arm (the direction in which the nerve runs).

Thus, with the biomagnetic-field detection apparatus 3100 according to the fifth embodiment of the second group of embodiments, a magnetic field generated in the elbow of the subject may be detected in the entire circumferential direction, and image data may be displayed to make a diagnosis as to whether the nerve is normal.

ALTERNATIVE EMBODIMENT OF SECOND GROUP OF EMBODIMENTS

In the description according to the first embodiment to the fifth embodiment of the second group of embodiments described above, the optically pumped atomic magnetometer is used as a room-temperature magnetic sensor; however, a room-temperature magnetic sensor (e.g., a magneto resistive (MR) sensor) other than an optically pumped atomic magnetometer may be used.

The present invention is not limited to the configurations described here and, for example, the configuration described in the above embodiment, etc., may be combined with different components. In this aspect, modifications may be made without departing from the scope of the present invention and may be determined appropriately depending on its application form.

THIRD GROUP OF EMBODIMENTS

Next, a magnetic shield box and a biomagnetic-field measurement apparatus according to a third group of embodiments of the present invention are described.

To diagnose amyotrophic lateral sclerosis (ALS) or muscular dystrophy, the needle electromyograph is used as a diagnostic device. However, with techniques using the needle electromyograph according to a related art, a needle electrode is inserted into a muscle of the subject, which causes pain to the subject. For this reason, consideration has been given to various techniques that use no needle.

The method that uses no needle is, for example, a method for detecting a magnetic field of the live subject by using a superconducting quantum interference device (SQUID). As a reduction in an external magnetic field is desired to detect a magnetic field, a magnetic shield box (MSB) may be used to measure a magnetic field.

For example, a magnetic shield box according to a related art has an open structure (see, for example, JP-2017-152573-A). Furthermore, in a technology according to a related art, a magnetic field may be measured in a magnetic shield room, and at that time, an optically pumped atomic magnetometer is used as a feedback sensor to reduce a magnetic field in the magnetic shield room (for example, see "Boto, E., Meyer, S. S., Shah, V., Alem, O., Knappe, S., Kruger, P., Fromhold, T. M., Lim, M., Glover, P. M., Morris, P. G., Bowtell, R., Barnes, G. R., Brookes, M. J. (2017). A new generation of magnetoencephalography: Room temperature measurements using optically-pumped magnetometers. Neuroimage, 149, 404-414"). However, it is considered that the limit of the high-speed response of the OPM is approximately 200 Hz as the response speed is determined by the relaxation times T1 and T2 of the alkaline metal (Rb or Cs) sealed in the internal gas cell.

It is, however, considered that the limit of a high-speed response of the OPM is approximately 200 Hz as the response speed is determined by relaxation times T1 and T2 of an alkaline metal (Rb or Cs) that is sealed in an internal gas cell.

On the other hand, as the frequency of the magnetic field (muscle magnetic field) generated by a muscle is approximately 500 Hz, the detection of a muscle magnetic field waveform of approximately 10 msec is desirable. In a case where the OPM with a response speed limit of approximately 200 Hz is used as a feedback sensor, the feedback at a desired band is difficult. As a result, it is difficult to reduce the magnetic field in the magnetic shield box to obtain a magnetic field sufficient to detect a muscle magnetic field. With respect to the point described above, an embodiment for carrying out the present invention is described below referring to the drawings. In each drawing, the same components are denoted by the same reference numerals, and duplicated descriptions may be omitted.

FIRST EMBODIMENT OF THIRD GROUP OF EMBODIMENTS

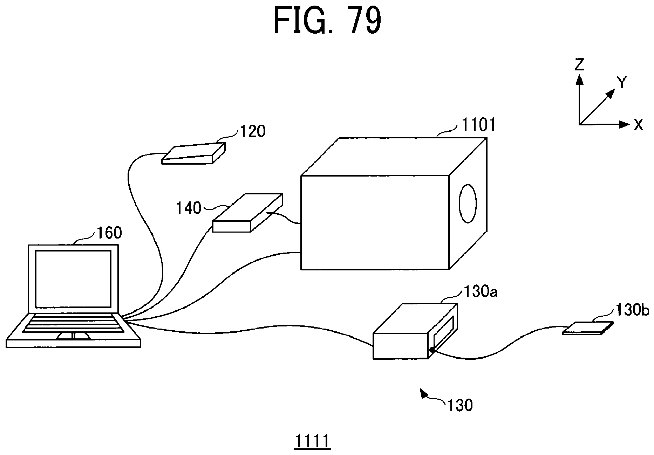

First, an external configuration of a biomagnetic-field measurement apparatus 1111 according to a first embodiment of the third group of embodiments is described. FIG. 79 is a diagram illustrating the external configuration of the biomagnetic-field measurement apparatus 1111. As illustrated in FIG. 79, the biomagnetic-field measurement apparatus 1111 includes a magnetic shield box 1101, the ultrasonic measuring device 120, the electrical stimulation device 130 including the generator 130a and the input terminal 130b, the current generating device 140, and the information processing device 160. The ultrasonic measuring device 120 and the electrical stimulation device 130 may be omitted from or included in the biomagnetic-field measurement apparatus 1111 as appropriate.

A description is primarily provided below of an example in which the measurement target of the biomagnetic-field measurement apparatus 100 is a "skeletal muscle of a hand," which is a part of a limb of the subject, more specifically, "abductor pollicis brevis of the hand." However, this is merely an example, and the measurement target of the biomagnetic-field measurement apparatus 100 is not limited to the "skeletal muscle of the hand." The measurement target of the biomagnetic-field measurement apparatus 100 may be a leg, head, etc.

The magnetic shield box 110 is a device that detects a magnetic field generated in the subject. The magnetic shield box 110 detects the magnetic field generated in the hand in a state where the subject's arm is inserted and the hand is placed at a predetermined position. The magnetic shield box 110 transmits magnetic-field data (data group on the magnetic flux density at each time) to the information processing device 160.

According to the first embodiment of the first group of embodiments, the longitudinal direction of the magnetic shield box 110 illustrated in FIG. 1 is the X-axis direction, the depth direction thereof is the Y-axis direction, and the height direction thereof is the Z-axis direction.

The ultrasonic measuring device 120 is a device that transmits and receives ultrasonic waves to measure the thickness of the fat of the hand, etc. The ultrasonic measuring device 120 transmits the measured ultrasonic data to the information processing device 160.

The measurement by the ultrasonic measuring device 120 is used to, for example, quantify the thickness of the fat on the surface layer of the abductor pollicis brevis of the hand. The ultrasonic measuring device 120 may measure the position and the depth of the fat in three dimensions. As the thickness of the fat may be estimated from the physical size, the weight, or the like, of the subject, the measurement by the ultrasonic measuring device 120 may be conducted as appropriate.

The electrical stimulation device 130 is a device that applies electrical stimulation to a predetermined site of the subject in a state where the subject's arm is inserted into the magnetic shield box 110 and the hand is placed at a predetermined position. As the electrical stimulation device 130, for example, "electromyography/evoked potential testing device MEB-9400 series Neuropack S1" manufactured by Nihon Kohden may be used.

The generator 130a of the electrical stimulation device 130 generates a voltage to be applied to an electrode of the input terminal 130b in response to an instruction from the information processing device 160. The input terminal 130b of the electrical stimulation device 130 is attached to a predetermined site of the subject to apply the voltage to the site so as to give electrical stimulation.

The information processing device 160 may process ultrasonic data transmitted from the ultrasonic measuring device 120 and calculate the thickness of the fat of a predetermined part of the hand. The information processing device 160 may also transmit an instruction to the electrical stimulation device 130 at predetermined timing to drive the electrical stimulation device 130.

The information processing device 160 transmits an instruction for driving each unit of the magnetic shield box 110 to the magnetic shield box 110. The information processing device 160 receives magnetic-field data transmitted from the magnetic shield box 110. The information processing device 160 calculates the muscle magnetic field of the skeletal muscle of the hand by using the data on the fat of the hand and the received magnetic-field data and then displays the waveform of the muscle magnetic field and numerical data calculated from the waveform.

The biomagnetic-field measurement apparatus 100 may calculate the spontaneous muscle magnetic field of the subject's skeletal muscle and display the waveform of the muscle magnetic field and the numerical data calculated from the waveform. The biomagnetic-field measurement apparatus 100 may calculate the muscle magnetic field (evoked muscle magnetic field), which is evoked when an electrical stimulus is given to the subject, of the subject's skeletal muscle and display the waveform of the muscle magnetic field and the numerical data calculated from the waveform. Thus, with the use of the biomagnetic-field measurement apparatus 100, the doctor, or the like, may properly diagnose ALS and muscular dystrophy.

For example, the ALS tends to show the sign of disability in a muscle of the hand. Therefore, with the use of the biomagnetic-field measurement apparatus 100, the doctor, or the like, detects the spontaneous muscle magnetic field or the evoked muscle magnetic field of, for example, the "abductor pollicis brevis" of the hand so as to estimate the degree of progress of the ALS.

Next, the flow of an operation to diagnose the muscle magnetic field of the skeletal muscle of the hand using the biomagnetic-field measurement apparatus 100 is described. FIGS. 2 and 3 are diagrams illustrating the flow of an operation to diagnose the muscle magnetic field of the skeletal muscle. In the example illustrated according to the present embodiment, the magnetic field of the abductor pollicis brevis is detected.

FIG. 2 illustrates a situation where the doctor, or the like, measures the thickness of the fat of the hand of a subject 200 using the ultrasonic measuring device 120. As illustrated in FIG. 2, the hand of the subject 200 is measured by using the ultrasonic measuring device 120 so that the information processing device 160 calculates the thickness of the fat of the part of the abductor pollicis brevis of the hand of the subject 200.

FIG. 3A illustrates a situation where the doctor, or the like, guides the subject 200 so as to insert the arm into the magnetic shield box 110 and place the palm at a predetermined position within the magnetic shield box 110.

Furthermore, FIG. 3A illustrates a situation where the doctor, or the like, operates the information processing device 160 so that a camera provided in the magnetic shield box 110 captures the palm. As illustrated in FIG. 3A, after the palm of the subject 200 is captured, the information processing device 160 displays image data 310.

FIG. 3B illustrates a situation where the doctor, or the like, attaches the input terminal 130b to the elbow (a site different from the palm that is the object under examination) of the subject 200. When the doctor, or the like, operates the information processing device 160 in a state where the input terminal 130b is attached, the generator 130a is driven to apply electrical stimulation to the subject 200 (e.g., the median nerve of the elbow). The magnetic shield box 1101 detects the evoked muscle magnetic field generated in the abductor pollicis brevis of the hand and transmits the magnetic-field data to the information processing device 160.

To detect the spontaneous muscle magnetic field, the doctor or the like prompts the subject 200 to perform a weak compression action. In this case, as no electrical stimulation is applied, the input terminal 130b is not attached in FIG. 3B. The magnetic shield box 110 detects the spontaneous muscle magnetic field generated in the abductor pollicis brevis of the hand in response to a weak compression action and transmits the magnetic-field data to the information processing device 160.

In a case where the biomagnetic-field measurement apparatus 100 exclusively detects the spontaneous muscle magnetic field, the biomagnetic-field measurement apparatus 100 may omit the electrical stimulation device 130.

In the case of both the detection of the evoked muscle magnetic field and the detection of the spontaneous muscle magnetic field, the information processing device 160 uses the thickness of the fat of the hand and the magnetic-field data to generate waveform data 320 on the muscle magnetic field of the abductor pollicis brevis of the hand and displays the waveform data 320 on the information processing device 160. The information processing device 160 may display the numerical data calculated from the waveform data 320 on the muscle magnetic field together with the waveform data 320 or in place of the waveform data 320.

The detection of the muscle magnetic field by the magnetic shield box 110 and the generation and the display of the waveform data 320, or the like, by the information processing device 160 continue for a predetermined time period. The doctor or the like monitors the waveform data 320 or the numerical data calculated from the waveform data 320 so as to properly diagnose ALS or muscular dystrophy of the subject 200.

Next, a system configuration of the biomagnetic-field measurement apparatus 100 is described. FIG. 4 is a diagram illustrating an example of the system configuration of the biomagnetic-field measurement apparatus 100.

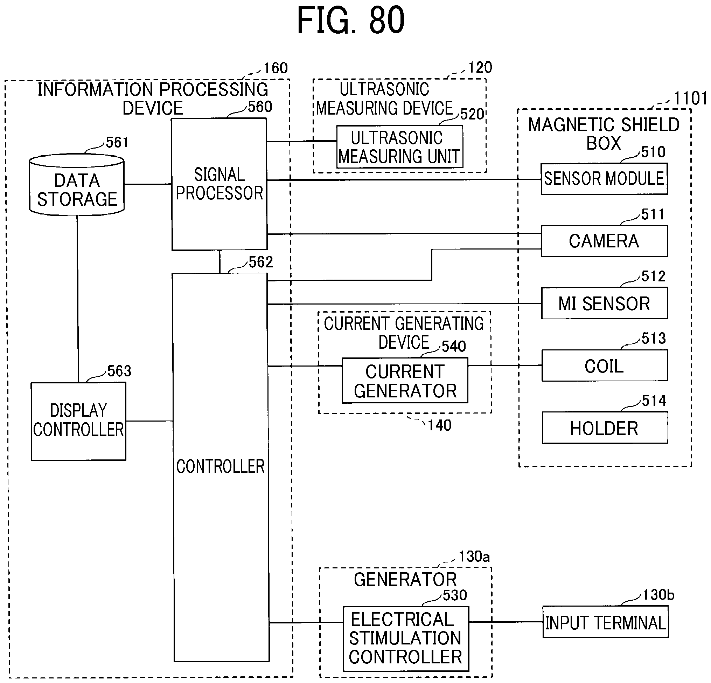

As illustrated in FIG. 80, in the biomagnetic-field measurement apparatus 1111 according to the first embodiment of the third group of embodiments, the magnetic shield box 1101 includes the sensor module 510, the camera 511, the MI sensor 512, the coil 513, and the holder 514. The sensor module 510 is a typical example of a first magnetic sensor according to the present invention. The MI sensor 512 is a typical example of a second magnetic sensor according to the present invention.

The sensor module 510 has an optically pumped atomic magnetometer and a position sensor built therein. The sensor module 510 detects the magnetic field (the muscle magnetic field of the abductor pollicis brevis) generated in the palm in a state where the end of the sensor module 510 is pressed against and is brought into contact with the abductor pollicis brevis of the hand placed at a predetermined position. The magnetic field measured by the sensor module 510 may be a spontaneous muscle magnetic field or an evoked biomagnetic field.

In the sensor module 510, the built-in position sensor detects the position of the sensor module 510 when a magnetic field is detected. The sensor module 510 transmits the detected magnetic-field data and the detected position data to a signal processor 560 of the information processing device 160.

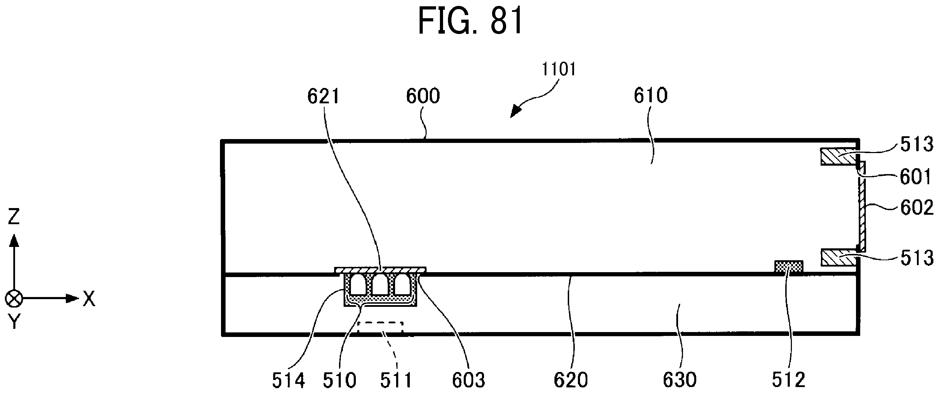

The single sensor module 510 may be provided, or the sensor modules 510 may be provided. As illustrated in FIG. 81 described later, for example, the magnetic shield box 1101 includes the three sensor modules 510 according to the present embodiment.

The camera 511 captures the palm placed at a predetermined position and transmits the image data to the signal processor 560 of the information processing device 160.

The MI sensor 512 is a solid-state magnetic sensor using a magneto-impedance element to measure a magnetic field in the magnetic shield box 110. The MI sensor 512 has a sensitivity of sub nT, a response speed of 1 kHz or more, and a size of approximately several centimeters. As the MI sensor 512, for example, MI-CB-1DH (Aich Micro Intelligent Corporation) may be used. The MI sensor 512 transmits the measured internal magnetic-field data to a controller 562 of the information processing device 160.

The coil 513 is supplied with the current determined by a current generator 540 of the current generating device 140 based on the measured value of the MI sensor 512. In other words, the current generator 540 acquires the current value calculated by the controller 562 of the information processing device 160 based on the internal magnetic-field data measured by the MI sensor 512 and controls the current flowing through the coil 513 based on the acquired current value. Accordingly, the magnetic field inside the magnetic shield box 110 is reduced so that a magnetic field allowing the detection of a muscle magnetic field may be formed inside the magnetic shield box 110.

The holder 514 is a member that holds the sensor module 510. The holder 514 holds the sensor module 510 such that the end of the sensor module 510 is pressed against and is brought into contact with the palm placed at a predetermined position.

As illustrated in FIG. 4, the ultrasonic measuring device 120 includes an ultrasonic measuring unit 520. The ultrasonic measuring unit 520 starts the measurement in response to an input instruction for starting ultrasonic measurement from the doctor, or the like, and transmits ultrasonic data to the signal processor 560 of the information processing device 160.

As illustrated in FIG. 4, the generator 130a includes an electrical stimulation controller 530. The electrical stimulation controller 530 generates the voltage to be applied to an electrode provided in the input terminal 130b in response to an instruction from the controller 562 of the information processing device 160.

As illustrated in FIG. 4, the current generating device 140 includes the current generator 540. The current generator 540 acquires the current value calculated by the controller 562 of the information processing device 160 based on the internal magnetic-field data measured by the MI sensor 512 and controls the current flowing through the coil 513 based on the acquired current value.

As illustrated in FIG. 4, the information processing device 160 includes the signal processor 560, a data storage 561, the controller 562, and a display controller 563.

The signal processor 560 calculates the thickness of the fat of the hand of the subject 200 based on the ultrasonic data transmitted from the ultrasonic measuring unit 520 and stores the thickness of the fat in the data storage 561. The signal processor 560 stores the image data transmitted from the camera 511 in the data storage 561. The signal processor 560 uses the thickness of the fat of the hand stored in the data storage 561 and the magnetic-field data and the position data transmitted from the sensor module 510 to generate the waveform data on the muscle magnetic field of the abductor pollicis brevis of the hand and stores the waveform data in the data storage 561.

The controller 562 receives a capturing instruction input by the doctor, or the like, via the display controller 563 and transmits an instruction to the camera 511. The controller 562 calculates the current value based on the internal magnetic field data measured by the MI sensor 512 and transmits the current value to the current generator 540. The controller 562 receives an instruction for starting the magnetic field measurement input by the doctor, or the like, via the display controller 563 and transmits an instruction to the electrical stimulation controller 530 and the current generator 540.

The display controller 563 notifies the controller 562 of an instruction for starting the magnetic field measurement input by the doctor or the like. The display controller 563 displays the waveform data stored in the data storage 561 in response to the notification of the instruction for starting the magnetic field measurement.

Next, an internal configuration of the magnetic shield box 110 is described. FIG. 5 is a diagram illustrating the internal configuration of the magnetic shield box 110 according to the first embodiment of the first group of embodiments. As illustrated in FIG. 5, the magnetic shield box 110 is covered with a hollow shielding member 600 that shields the external magnetic field.

The shielding member 600 may be formed by, for example, bending a permalloy flat plate (a thickness of approximately 2 mm). In order to reduce the residual magnetic field inside the shielding member 600 to such a degree that the muscle magnetic field is measurable, it is preferable that the permalloy used for the shielding member 600 has a multi-layer structure (e.g., a three-layer structure). For the shielding member 600, permalloy plates may be bonded by, for example, welding so that a magnetic field is prevented from leaking, except for the holes for passing the wires of the sensor module 510 and the MI sensor 512 and an opening 601 described later.

The internal space of the magnetic shield box 110 is divided into a first space 610 in which the palm of the inserted arm is placed and a second space 630 in which the holder 514 holding the sensor module 510 is provided.

A boundary member 620 is provided between the first space 610 and the second space 630. The boundary member 620 includes, for example, permalloy.

The boundary member 620 is a support member that supports the live subject inserted through the opening 601 and is provided between the opening 601 and at least the measurement position. As the boundary member 620, which is a support member, is located close to the site where the magnetic field is generated, the boundary member 620 may absorb the noise magnetic field (artifact) effectively due to the direct contact with the live subject. Providing the boundary member 620 allows the removal of the electromagnetic field reflected and diffused in the second space 630 and allows a reduction in the noise in the first space 610.

The shielding member 600 is provided with the opening 601 through which a site of the live subject is inserted into the first space 610 to measure a magnetic field. The opening/closing mechanism 602 is provided around the opening 601. For example, the upper arm and the forearm of the subject 200 may be inserted into the first space 610 through the opening 601. The opening 601 may be, for example, circular or elliptical.

The coil 513 is provided in the first space 610. The coil 513 includes, for example, a solenoid coil. For example, the coil 513 may be arranged in an annular shape so as to surround the opening 601. In the first space 610, the MI sensor 512 is provided.

The camera 511 and the holder 514 are provided in the second space 630. The holder 514 is fixed to the boundary member 620 while holding the sensor module 510. Here, for example, the holder 514 holds the three sensor modules 510.

An opening 603 is provided at a predetermined position on the boundary member 620, and the flexible film 621 is fixed to the opening 603. The opening 603 to which the flexible film 621 is fixed is the position (measurement position) where the palm is placed in the first space 610. The end of the sensor module 510 held by the holder 514 is in contact with the palm placed in the first space 610 via the flexible film 621.

The flexible film 621 is pressed by the sensor module 510 in the holder 514 and is therefore deformed along the surface shape of the object under examination. The flexible film 621 may include, for example, a Teflon (registered trademark) film or a magnetic shield film in which the front and back surfaces of an amorphous metallic foil are processed with a PET film (a magnetic shield film having an amorphous metallic foil sandwiched therein). For example, a KOYO MS sheet manufactured by Koyo Sangyo Co., Ltd. may be used as the flexible film 621.

Inside the shielding member 600, a magnetic field enters through the opening 601 and the holes for passing the wires of the sensor module 510 and the MI sensor 512, and a gradient residual magnetic field (i.e., a gradient magnetic field) is generated in a direction away from the opening 601 along the X-axis direction. Here, the gradient magnetic field is a magnetic field whose intensity changes depending on the location.

According to the present embodiment, in order to cancel the residual magnetic field inside the shielding member 600, the MI sensor 512 is provided inside the shielding member 600, and the coil 513 is provided around the opening 601 inside the shielding member 600. The magnetic field is detected by the MI sensor 512, the current is applied to the coil 513 based on the detection result of the MI sensor 512, and the residual magnetic field inside the shielding member 600 is canceled by the electromagnetic field generated by the coil 513. This makes it possible to reduce the residual magnetic field at the measurement position inside the shielding member 600 and to detect a weak magnetic field.

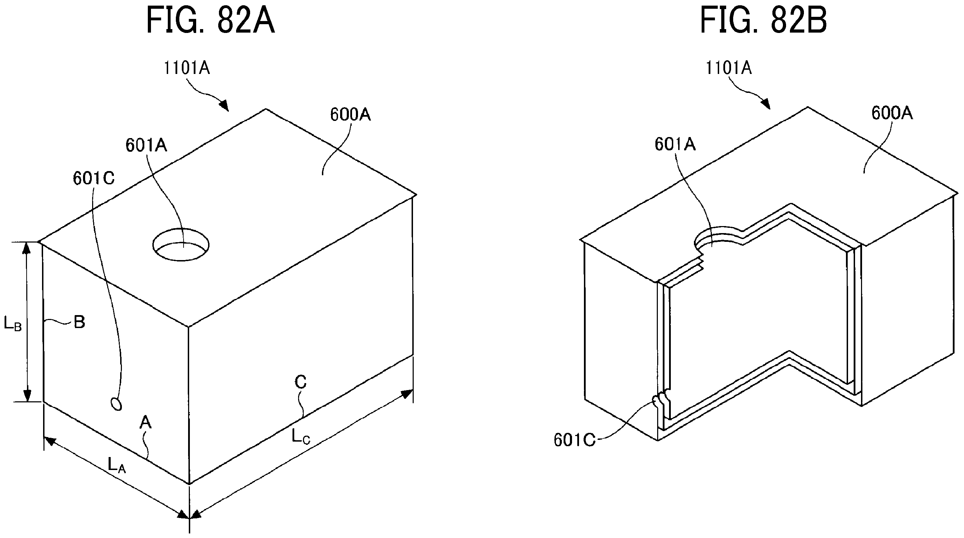

A reduction in the magnetic field in a magnetic shield box 1101A is described below in detail. FIGS. 82A and 82B are first diagrams illustrating a gradient magnetic field and illustrating the magnetic shield box 1101A used for simulation. FIG. 82A is a perspective view illustrating the external appearance of the magnetic shield box 1101A, and FIG. 82B is a perspective view illustrating a partial cross-sectional view of the magnetic shield box 1101A.

As illustrated in FIG. 82A and FIG. 82B, a shielding member 600A of the magnetic shield box 1101A has a three layer structure of permalloy, and the outer shape of the shielding member 600A is a cuboid having a side A, a side B, and a side C (the side A<the side C and the side B<the side C). Here, one of the surfaces formed by the side A and the side B is the bottom surface, the surface opposite to the bottom surface is the upper surface, and the surfaces formed by the side A and the side C or the side B and the side C are side surfaces.

For example, in the shielding member 600A, a length LA of the side A of the bottom surface=a length LB of the side B of the bottom surface=300 mm. Also, a length LC (a length in the longitudinal direction) of the side C perpendicular to the side A and the side B of the bottom surface=600 mm.

A circular opening 601A is provided on one of the side surfaces formed by the side A and the side C of the shielding member 600A, and an opening 601C for passing the wire of the sensor module 510 is provided on the bottom surface formed by the side A and the side B. Here, for example, the opening 601A has a diameter of 12 mm.

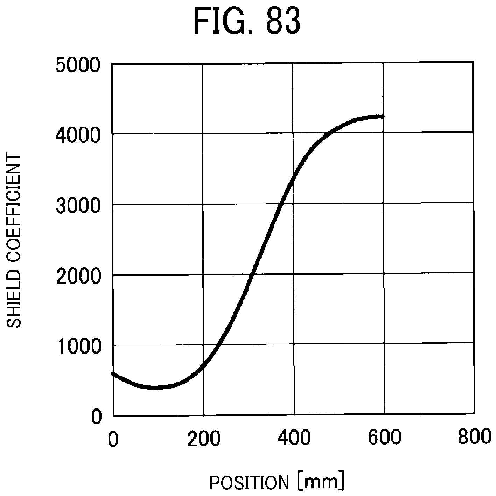

FIG. 83 is a second graph illustrating the gradient magnetic field and illustrating the simulation result of the gradient magnetic field inside the shielding member 600A illustrated in FIGS. 82A and 82B. In FIG. 83, the horizontal axis indicates a position in the longitudinal direction of the shielding member 600A (in the direction parallel to the side C), and the vertical axis indicates a shield coefficient (the magnetic field outside the shielding member 600A/the magnetic field inside the shielding member 600A).

According to the simulation result illustrated in FIG. 83, there is a magnetic field of approximately $10\mu T$ near the entrance of the shielding member 600A (near the position of 0 mm). It is understood that a larger shield coefficient is obtained at a deeper position in the shielding member 600A in the longitudinal direction thereof and a gradient magnetic field is formed.

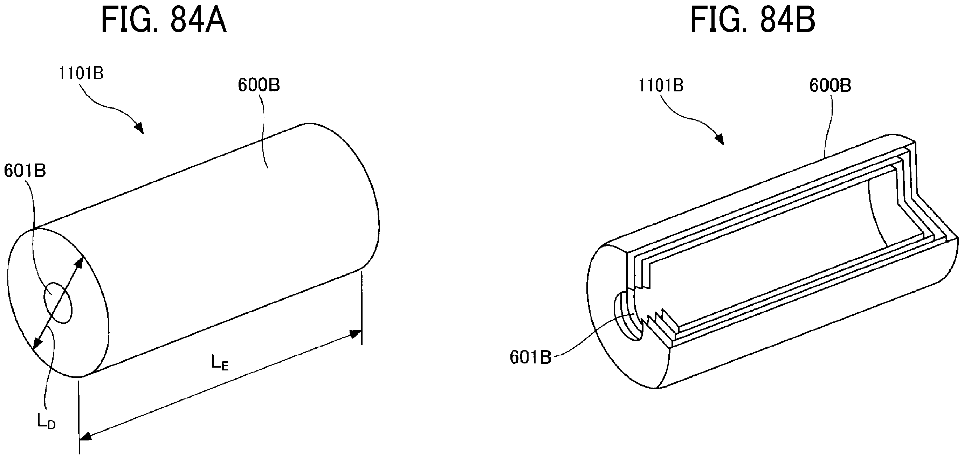

FIGS. 84A and 84B are third diagrams illustrating a gradient magnetic field and illustrating a magnetic shield box 1101B used for simulation. FIG. 84A is a perspective view of the external appearance of the magnetic shield box 1101B, and FIG. 84B is a perspective view illustrating a partial cross-section of the magnetic shield box 1101B.

As illustrated in FIG. 84A and FIG. 84B, a shielding member 600B of the magnetic shield box 1101B is a three-layer structure of permalloy, and the outer shape of the magnetic shield box 1101B is a cylindrical shape. Here, one of the surfaces having a diameter $L_D$ is the bottom surface, and the surface opposite to the bottom surface is the upper surface. The curved surface having a height $L_E$ connecting the bottom surface and the upper surface is a side surface.

For example, in the shielding member 600B, the diameter $L_D$ of the bottom surface and the upper surface<the height $L_E$. Specifically, the diameter $L_D$ of the bottom surface=150 mm, and the height $L_E$=340 mm.

The bottom surface of the shielding member 600B is provided with a circular opening 601B. For example, the opening 601B has a diameter of 50 mm.

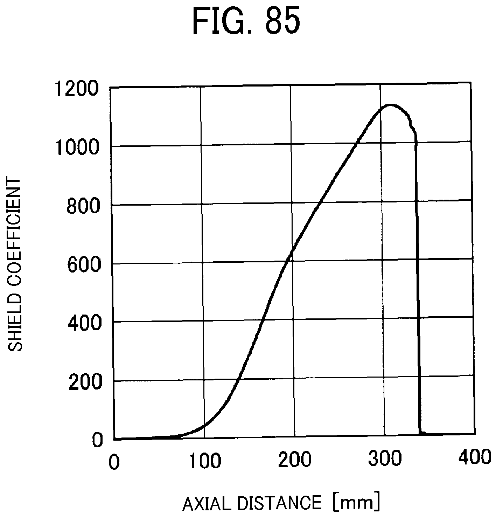

FIG. 85 is a fourth graph illustrating a gradient magnetic field and illustrating the simulation result of the gradient magnetic field inside the shielding member 600B illustrated in FIGS. 84A and 84B. In FIG. 85, the horizontal axis indicates a position in the axial direction (in the height direction) of the shielding member 600B, and the vertical axis indicates a shield coefficient (the magnetic field outside the shielding member 600B/the magnetic field inside the shielding member 600B).

According to the simulation result illustrated in FIG. 85, there is a magnetic field of approximately $10\mu T$ near the entrance of the shielding member 600B (near the position of 0 mm). It is understood that a larger shield coefficient is obtained at a deeper position in the shielding member 600B in the axial direction thereof and a gradient magnetic field is formed.

From the simulation results illustrated in FIGS. 83 and 85, it is understood that, when a shielding member having a predetermined shape is provided with an opening, a gradient magnetic field is formed inside the shielding member such that the magnetic field near the opening is largest and the magnetic field becomes smaller in accordance with an increase in the distance from the opening. Furthermore, from the simulation results in FIGS. 83 and 85, it is understood that the length of the shielding member is adjusted so as to obtain a shield coefficient of 1000 or more.

For example, when there is a magnetic field of $10\mu T$ near the opening of the shielding member, the magnetic field is approximately 1/1000 at the position where the shield coefficient is 1000, that is, a magnetic field of approximately 100nT.

Therefore, as in the magnetic shield box 1101 illustrated in FIG. 81, the coil 513 is provided near the opening 601 inside the shielding member 600, and the MI sensor 512 is provided between the opening 601 and the measurement position (the position of the sensor module 510). This allows the current generator 540 to apply the current to the coil 513 such that the magnetic field detected by the MI sensor 512 is reduced to the limit of the sensitivity of the MI sensor 512. The current applied to the coil 513 by the current generator 540 is, for example, several amperes.

As the sensitivity of the MI sensor 512 is approximately 100 pT, the MI sensor 512 may detect a magnetic field near the opening 601 and provide the feedback up to approximately 100 pT, which is the limit of the sensitivity of the MI sensor 512. As a result, the magnetic field near the opening 601 (on the inner side of the opening 601) may be reduced to approximately 100 pT.

As described above, as the coil 513 is supplied with the current determined based on the value measured by the MI sensor 512 from the current generator 540 of the current generating device 140 outside the shielding member 600, the magnetic field on the inner side of the opening 601 may be reduced.

In the shielding member 600, a gradient magnetic field is formed, which has a smaller magnetic field in accordance with an increase in the distance from the opening 601. Therefore, when the magnetic field near the opening 601 is approximately 100 pT, the magnetic field is approximately 100 fT at the position where the shield coefficient is approximately 1000.

As the sensor module 510 is provided at a position where the magnetic field has been reduced to approximately 100 fT, the sensor module 510 may detect an extremely weak magnetic field (e.g., 500 Hz at 1 pT) generated by a muscle. The sensor module 510 may be provided at a position where the shield coefficient is 1000 or more.

As the response speed of the MI sensor 512 is 1 kHz or more, the feedback at this speed makes it possible to form a magnetic field sufficient to detect an extremely weak magnetic field (e.g., 500 Hz at 1 pT) generated by a muscle. On the other hand, even when the feedback is provided by using the sensor module 510 with a response speed of approximately 200 Hz as it is conventionally conducted, it is difficult to form a magnetic field sufficient to detect an extremely weak magnetic field (e.g., 500 Hz at 1 pT) generated by a muscle.

When the ratio of the magnetic field intensity at the measurement position (the position of the sensor module 510) to the magnetic field intensity at the position of the opening 601 is B1, and the ratio of the minimum value of the magnetic field to be measured to the minimum value of the magnetic field detectable by the MI sensor 512 is B2, B1<B2 is satisfied. Thus, the magnetic field at the measurement position may be smaller than the magnetic field to be detected.

For example, at the position where the shield coefficient is 1000, B1=1/1000. As the minimum value of the magnetic field detectable by the MI sensor 512 is approximately 100 pT and the minimum value of the magnetic field to be measured is approximately 1 pT, B2=1/100. In this case, B1<B2 is satisfied.

The ratio of the magnetic field intensity at the measurement position to the magnetic field intensity at the position of the opening 601 is B1 and the ratio of the minimum value of the magnetic field detectable by the MI sensor 512 to the minimum value of the magnetic field detectable by the sensor module 510 is B3, B1<B3 is satisfied. Thus, the noise at the measurement position may be lowered as compared with the minimum value of the magnetic field detectable by the sensor module 510. That is, the maximum performance of the sensor module 510 may be achieved.

For example, B1=1/1000 at the position where the shield coefficient is 1000. As the minimum value of the magnetic field detectable by the sensor module 510 is approximately 10 fT and the minimum value of the magnetic field detectable by the MI sensor 512 is approximately 100 pT, B3=110 fT/100 pT=1.1/1000. In this case, B1<B3 is satisfied.

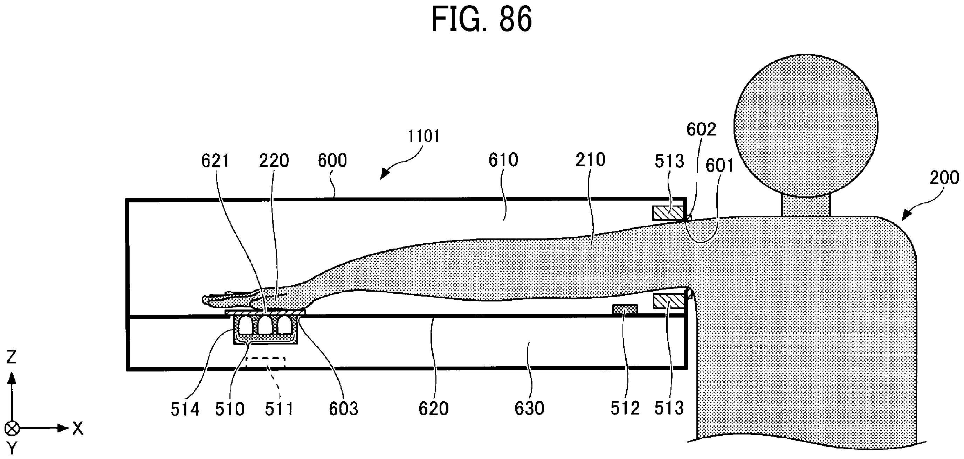

Next, the positional relationship with each part when the arm is inserted into the shielding member 600 is described. FIG. 86 is a diagram illustrating a situation where the arm 210 is inserted into the shielding member 600. As illustrated in FIG. 86, the subject 200 inserts the arm 210 through the opening 601 provided in the first space 610 to place the palm 220 at the position where the flexible film 621 is fixed.

According to the present embodiment, the sensor module 510 is arranged inside the shielding member 600 to detect the abductor pollicis brevis. Furthermore, the shielding member 600 is designed such that the most part of the arm 210 is located within the shielding member 600 and the shielding member 600 is as thin as possible.

The shielding member 600 may have, for example, a cylindrical shape having a diameter of approximately 150 mm and a length of approximately 600 mm. This is an example of the case where the diameter of the cylindrical shape is determined in accordance with the size of the typical male hand (with the palm squeezed) and the length of the cylindrical shape is determined in accordance with the length of the female arm. The shielding performance is improved when the cylindrical shape is made as thin as possible.

The shape of the shielding member 600 is not limited to a cylindrical shape and may have any shape such as a cuboid or a truncated cone shape into which the arm may be inserted.

As the opening/closing mechanism 602 provided around the opening 601 is in a closed state when the palm 220 is placed, the first space 610 is sealed. Thus, the shielding member 600 of the magnetic shield box 1101 allows the palm 220 of the subject 200 to be placed (held) inside the shielding member 600.

The camera 511 is provided at a position different from the sensor module 510 in the Y-axis direction so as to capture the palm 220 through the opening 603 in a state where the palm 220 is placed. Furthermore, in a state where the palm 220 is placed, the sensor module 510 in the holder 514 may detect the magnetic field generated in the palm 220.

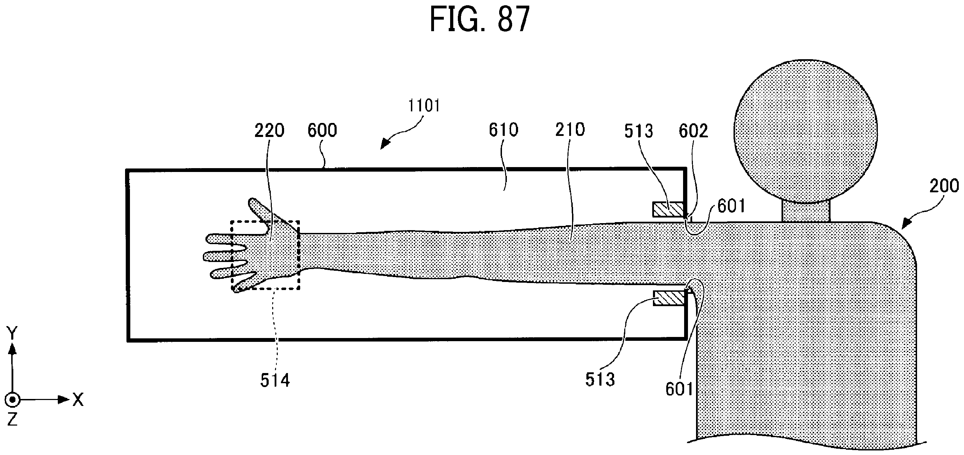

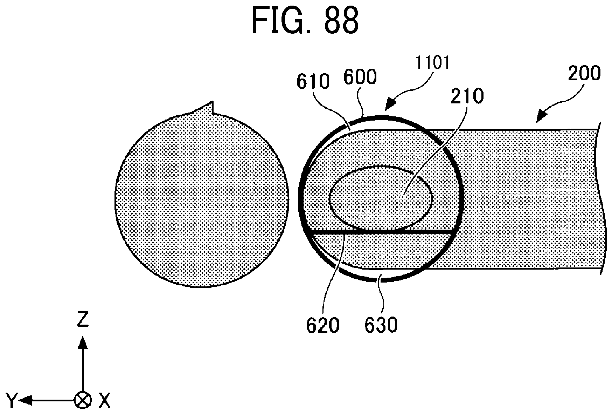

Although FIG. 86 illustrates an example in which the subject 200 is inserting the arm 210 through the opening 601 of the shielding member 600 in a state where the subject 200 is sitting, the subject 200 may insert the arm 210 through the opening 601 of the shielding member 600 in a state where the subject is lying on the back, as illustrated in FIGS. 87 and 88.

Next, a detailed configuration of the holder 514 is described. FIG. 44 is a diagram illustrating the detailed configuration of the holder 514.

For example, the sensor modules 510_1, 510_2, and 510_3 are arranged along the X-axis direction.

The sensor module 510_1 is supported by the support base 802_1 via the elastic member 801_1 (e.g., a spring). The sensor module 510_2 is supported by the support base 802_2 via the elastic member 801_2 (e.g., a spring). The sensor module 510_3 is supported by the support base 802_3 via the elastic member 801_3 (e.g., a spring). The support bases 802_1, 802_2, and 802_3 are fixed to the holder 514.

As the sensor modules 510_1 to 510_3 are supported via the elastic members 801_1 to 801_3, the ends of the sensor modules 510_1 to 510_3 may be pressed against and brought into contact with the palm 220.

The sensor module 510_1 and the sensor module 510_2 are separated by the permalloy partition wall 803. The sensor module 510_2 and the sensor module 510_3 are separated by the permalloy partition wall 804. With the provision of the permalloy partition wall between the sensor modules, the occurrence of crosstalk between the sensor modules may be prevented.

As illustrated in FIG. 44, the sensor module 510_1 includes the gas cell 1021-1 and the position sensor 1031_1. The sensor module 510_2 includes the gas cell 1021-2 and the position sensor 1031_2. The sensor module 510_3 includes the gas cell 1021-3 and the position sensor 1031_3.

The gas cell 1021-1, the gas cell 1021-2, and the gas cell 1021-3 detect the magnetic field generated in, for example, the abductor pollicis brevis. The position sensor 1031_1, the position sensor 1031_2, and the position sensor 1031_3 detect the position when the magnetic field is detected.

Next, a schematic configuration of the optically pumped atomic magnetometer included in the sensor module 510 is described. FIG. 11 is a diagram illustrating a schematic configuration of the optically pumped atomic magnetometer. As illustrated in FIG. 11, the optically pumped atomic magnetometer emits laser beam to a gas cell of rubidium atoms and detects the laser beam having passed through the gas cell by using a photodetector. The laser beam passing through the gas cell is absorbed depending on the magnitude of the magnetic field generated in a $Y_0$ axis direction or a $Z_0$ axis direction, and therefore the intensity of the laser beam detected by the photodetector decreases due to the magnetic field generated in the $Y_0$ axis direction or the $Z_0$ axis direction.

Thus, it is possible to calculate the magnitude of the magnetic field by comparing the intensity of the laser beam detected by the photodetector in a state where no magnetic field is generated and the intensity of the laser beam detected by the photodetector in a state where a magnetic field is generated. A coil is wound around the gas cell to apply an appropriate alternating current.

Thus, the optically pumped atomic magnetometer may detect a magnetic field in a direction substantially perpendicular to the incident direction (light propagation direction) of the laser beam. According to the present embodiment, the direction substantially parallel to the incident direction of the laser beam is an $X_0$ axis direction, and the directions substantially perpendicular to the incident direction of the laser beam are the $Y_0$ axis direction and the $Z_0$ axis direction.

For example, the gas cell is located away from the housing surface by approximately 6 mm, and the gas cell detects a magnetic field at this area. Hereinafter, the term "gas cell" refers to a detection position.

For example, the myoelectric potential propagates in the direction of the muscle fiber of the abductor pollicis brevis. The direction may be defined as a potential propagation direction. As illustrated in FIG. 12, a potential propagation direction P of a muscle fiber (the direction of a muscle fiber) of an abductor pollicis brevis 250 is substantially parallel to the light propagation direction (the $X_0$ axis direction) of the sensor module 510 so that a magnetic field may be measured with high accuracy.

FIG. 13 is a diagram illustrating an example of the arrangement of sensor modules 510_1, 510_2, and 510_3 in the holder 514. In the example of FIG. 13, the sensor modules 510_1, 510_2, and 510_3 are arranged in parallel in the $Y_0$ axis direction. In FIG. 13, in a state where the palm 220 is placed at a predetermined position, a muscle fiber 255 of the palm 220 runs in the $X_0$ axis direction substantially perpendicular to the YZ plane.

The potential propagating in the muscle fiber direction (the $X_0$ axis direction) may be understood in the same manner as the current, and a magnetic field is generated in the direction of rotation. For example, when the current flows through the muscle fiber 255 from the front side of the drawing plane to the back side thereof, a magnetic field is generated on the YZ plane in the direction of an arrow M. As a result, magnetic fields are generated at the positions of gas cells 1021-1 to 1021-3 in different vector directions as indicated by arrows V1 to V3. Thus, the area where a myoelectric potential occurs may be identified by detecting a magnetic field in the $Y_0$ axis direction and a magnetic field in the $Z_0$ axis direction with the sensor modules provided at multiple areas instead of the sensor module provided at a single area.

Next, the magnetic field shield characteristics of the flexible film 621 are described. FIG. 14 is a graph illustrating an example of the magnetic field shield characteristics of the flexible film 621. In FIG. 14, the horizontal axis represents a frequency, and the vertical axis represents the permeability of a magnetic field.

As illustrated in FIG. 14, the flexible film 621 secured to the boundary member 620 allows the permeability of a magnetic field having a frequency band higher than 1 [Hz] (having a filtering function to shield a magnetic field having a frequency band from 0.01 [Hz] to 1 [Hz]). Thus, the sensor modules 510_1 to 510_3 have a high sensitivity for detecting a magnetic field (100 [Hz] or more) generated due to the current flowing through the palm 220 even when the sensor modules 510_1 to 510_3 are in contact with the subject 200 via the flexible film 621.

Next, the opening/closing mechanism 602 disposed around the opening 601 in the shielding member 600 is described.

FIGS. 15A and 15B are first diagrams illustrating a function of the opening/closing mechanism 602 and illustrating a situation when the shielding member 600 is viewed in the X-axis direction. FIG. 15A illustrates a case where the opening/closing mechanism 602 is in an opened state. As illustrated in FIG. 15A, in a case where the opening/closing mechanism 602 is in an opened state, an insertion opening 1220, through which the arm of the subject 200 is inserted, has more than a certain size with respect to the opening 601 provided in the shielding member 600. This allows the subject 200 to easily insert the arm into the first space 610.

On the other hand, FIG. 15B illustrates a case where the opening/closing mechanism 602 is in a closed state. As illustrated in FIG. 15B, in a case where the opening/closing mechanism 602 is in a closed state, the insertion opening 1220 is narrower with respect to the opening 601 provided in the shielding member 600. This allows the first space 610 to be sealed in a state where the subject 200 has inserted the arm into the first space 610.

FIGS. 16A and 16B are second diagrams illustrating a function of the opening/closing mechanism 602 and illustrating a situation when the shielding member 600 is viewed in the Y-axis direction. FIG. 16A illustrates a situation where the opening/closing mechanism 602 is in an opened state and the subject 200 is inserting the palm 220.

On the other hand, FIG. 16B illustrates a situation where the opening/closing mechanism 602 is in a closed state and the subject 200 has inserted the arm. As illustrated in FIG. 16B, the opening/closing mechanism 602 holds part of the arm of the subject 200 while in a closed state.

It is known that there are three types of human muscles and, as illustrated in FIG. 17, the human muscles include skeletal muscles, cardiac muscles, and smooth muscles. Although there are conventional biomagnetic-field measurement apparatuses targeted for cardiac muscles, biomagnetic-field measurement apparatuses targeted for skeletal muscles are not known.

As only skeletal muscles allow voluntary motions and are controlled by the brain and motor nerves, skeletal muscles are primarily examined for needle electromyography used to diagnose ALS and muscular dystrophy. This is because a myoelectric waveform generated during a voluntary motion is interpreted so as to determine a disease. It is known that a skeletal muscle is an elongated cylindrical muscle cell (hereinafter referred to as muscle fiber) and a potential propagates in the fiber direction over a certain distance.

FIG. 18 is a diagram schematically illustrating the abductor pollicis brevis 250 that is an example of a skeletal muscle. As illustrated in FIG. 18, the abductor pollicis brevis 250 is a bundle of the muscle fibers 255. An overall length L1 of the abductor pollicis brevis 250 is approximately 40 mm, and the overall width thereof is approximately 20 mm. As the outer diameter of the single sensor module 510 is approximately a dozen mm, the sensor modules 510_1 and 510_2 may be arranged in parallel in the potential propagation direction P (the Xo axis direction) of the muscle fibers 255, as illustrated in for example FIG. 18.

On the other hand, as cardiac muscles and smooth muscles are planar muscles, it is difficult to define a fiber direction. Furthermore, in cardiac muscles and smooth muscles, a myoelectric potential also spreads on a surface. For these reasons, it is not practical to arrange the sensor modules 510 in the muscle fiber direction or to align an optical axis OA of the sensor modules 510_1 and 510_2 with the muscle fiber direction.

FIG. 18 schematically illustrates fat 257, a motor nerve 259, and a discharge state D.

FIG. 19 is a first table illustrating waveforms (comparative example) of needle electromyography. As illustrated in FIG. 19, a waveform of the needle electromyography typically used for the examination on the ALS, etc., is a two to three phase (the negative potential repeats twice and the positive potential once) waveform of approximately 10 msec. A waveform, which is multiphase, spiky, or the like, is detected to make a diagnosis. The frequency at which a waveform is multiphase or spiky is detected to make a diagnosis. Also, the intensity (approximately 1 mV in potential and approximately 1 pT in a magnetic field as a standard) of a waveform is detected to make a diagnosis.

FIG. 20 is a second table illustrating a waveform (comparative example) of needle electromyography. As illustrated in FIG. 20, the waveform of a fibrillation potential, a positive sharp wave, or the like, is also observed in a resting state to examine whether there is abnormal discharge.

A typical sensor module including an optically pumped atomic magnetometer includes, for example, an OPM device manufactured by QuSpin Inc. The response speed of the OPM device is defined by the relaxation times T1 and T2 of the alkaline metal rare gas in the gas cell, and the response speed is typically approximately 200 Hz.

FIGS. 21A to 21D are diagrams for examining the response speed of a typical sensor module and illustrates waveforms measureable by an OPM device with one module.

The original data in FIG. 21A is a weak compression waveform of the abductor pollicis brevis of a healthy person obtained with needle electromyography, and two to three types of motor units are detected. Distinguishing between two to three types of waveforms in FIG. 21A is desirable.

FIGS. 21B to 21D illustrate whether the two to three types of waveforms in FIG. 21A may be distinguished when a response speed is different. The waveform may be distinguished at a response speed of 1 kHz illustrated in FIG. 21B or at a response speed of 500 Hz illustrated in FIG. 21C, while it is difficult to sufficiently distinguish the waveform at a response speed of 200 Hz illustrated in FIG. 21D.

That is, in the OPM device with a response speed of 200 Hz, it is difficult to distinguish a weak compression waveform of the abductor pollicis brevis. To distinguish the weak compression waveform of the abductor pollicis brevis, a response speed higher than 200 Hz is desirable, and it is preferable to use a device having a response speed of at least approximately 500 Hz.

Next, an improvement in the accuracy of the sensor module is described. For example, a consideration is given to a case where the sensor modules 510_1 and 510_2 are arranged in parallel in the potential propagation direction P of the muscle fiber 255, as described above in FIG. 18.

FIG. 22 is a first graph illustrating an improvement in the accuracy of the sensor modules 510_1 and 510_2. As illustrated in FIG. 22, in a case where the measurement points have an interval of 5 msec, for example, signals of the sensor modules 510_1 and 510_2 in FIG. 18 are measured at different positions, and each of the signals is meaningful data. Therefore, the signals are combined to calculate high-accurate data.

Generally, the potential propagation velocity of a skeletal muscle is several tens of m/sec, and it takes several msec to propagate several cm. Therefore, when the sensor modules 510_1 and 510_2 are arranged in the potential propagation direction P, as illustrated in FIG. 18, the detection times of the sensor modules 510_1 and 510_2 are shifted by several msec, as illustrated in FIG. 22. This time is defined as T1. T1 depends on the distance between the sensor module 510_1 and the sensor module 510_2.

FIG. 23 is a second graph illustrating an improvement in the accuracy of the sensor modules 510_1 and 510_2 and schematically illustrating a case where each data set of the sensor module 510_2 in FIG. 22 is shifted in the direction of the arrow by the time T1. As illustrated in FIG. 23, although there are five data sets in the case of the measurement at an interval of 5 msec (approximately 20 msec in total), nine data sets, almost twice as many, are presented, whereby an improvement in the accuracy of measurement data is achieved. Due to potential measurements, and the like, it is known that a propagation waveform is not largely deformed.

FIG. 24 is a third diagram illustrating an improvement in the accuracy of the sensor modules 510_1 and 510_2. As illustrated in FIG. 24, in some case, a motor nerve 259 is coupled to the muscle fiber 255 in the vicinity of the center thereof in the $X_0$ axis direction, and potentials propagate in two directions P1 and P2 (opposite directions) along the muscle fiber 255 due to the firing at the coupling area (central firing).

FIG. 25 is a fourth graph illustrating an improvement in the accuracy of the sensor modules 510_1 and 510_2 and illustrating signal waveforms in the case of FIG. 24. In this case, different from the potential propagation velocity of the skeletal muscle, the correction with T1 as in FIG. 23 is not proper. Central firing may be estimated to some extent by observing a signal waveform. In the case of central firing, T2 illustrated in FIG. 25 is defined, and a shift is made by T2, as illustrated in FIG. 26. As the measurement points of two waveforms are close, there is less effect on the correction due to an increase in the measurement points as compared with the case in FIG. 23; however, an improvement in the S / N, etc., is beneficial.

As described above, the parallel arrangement of the sensor modules 510_1 and 510_2 in the potential propagation direction P of the muscle fiber 255 improves the temporal resolution or the S / N.

FIGS. 27 and 28 are diagrams illustrating another example of the arrangement of the two sensor modules 510_1 and 510_2. FIG. 27 illustrates a situation viewed from above when the muscle fiber direction is in the $X_0$ axis direction, and FIG. 28 illustrates a situation viewed in a direction perpendicular to the YZ plane. In FIG. 28, for example, when a current flows through the muscle fiber 255 from the back side of the drawing plane to the front side, a magnetic field is generated in the direction of an arrow M on the YZ plane.

In FIGS. 27 and 28, the sensor modules 510_1 and 510_2 are arranged in parallel in the $Y_0$ axis direction perpendicular to the $X_0$ axis direction (the same direction as the potential propagation direction P), which is the muscle fiber direction. As described above, an overall width L2 of the abductor pollicis brevis 250, which is the bundle of the muscle fibers 255, is approximately 20 mm.

As illustrated in FIGS. 27 and 28, the parallel arrangement of the sensor modules 510_1 and 510_2 in the $Y_0$ axis direction makes it possible to detect where the muscle fiber 255 has fired in the 20-mm width of the abductor pollicis brevis 250. In the examples of FIGS. 27 and 28, the firing position may be identified at the left edge of the drawing.

FIG. 29 is a diagram illustrating an example of the arrangement of the three sensor modules. As illustrated in FIG. 29, for example, the three sensor modules (the sensor modules 510_1 to 510_3) may be provided. In this case, it is preferable that the permalloy shielding plate 800 is sandwiched between the sensor module 510_2 and the sensor module 510_3 that are adjacent to each other in the optical axis direction. For example, the permalloy shielding plate 800 of approximately 2 mm sandwiched between the adjacent sensor modules may reduce the crosstalk.

In FIG. 29, as optical axes OA1 to OA3 (light propagation directions) of the sensor modules 510_1 to 510_3 are aligned with the potential propagation direction P (muscle fiber direction), the information on the depth direction may be obtained from the ratio of the magnetic-field data in the $Z_0$ direction to the magnetic-field data in the $Y_0$ direction.

It is preferable that a distance Lx from the sensor module 510_1 to the sensor modules 510_2 and 510_3 in the $X_0$ axis direction is smaller than a distance Ly between the sensor module 510_2 and the sensor module 510_3 in the $Y_0$ axis direction.

That is, the relationship Lx<Ly is preferable. This is because the crosstalk in the light propagation direction is larger than the crosstalk in a direction perpendicular to the light propagation direction and preventing the crosstalk is desirable. Maintaining the relationship Lx<Ly makes it possible to have the closest packing position in the layout of the sensor modules and estimate the waveform of a muscle fiber with high accuracy.

A method for calculating a distance Zk in the depth direction at the firing position by comparing the magnetic-field data in the $Z_0$ direction with the magnetic-field data in the $Y_0$ direction is described. As for the information on the depth direction, as the Biot-Savart law holds for the relation between a magnetic field and a current, the intensity is proportional to the square of the distance. The distance is estimated and the detected magnetic field intensity is multiplied by the correction coefficient based on the distance so that the magnetic field independent of the depth may be detected. It is known that the magnitude of a magnetic field in the muscular dystrophy is small, and the magnitude of a magnetic field in the ALS is large (giant motor unit (MUP)), and therefore the above-described correction is desirable for the accurate diagnosis.

FIG. 30 is a diagram illustrating a specific method for quantifying a depth direction. In FIG. 30, a magnetic field is concentrically formed around a firing position D. A perpendicular LS1 is a perpendicular to a magnetic field M2 at the position of the sensor module 510_1. A perpendicular LS2 is a perpendicular to a magnetic field M3 at the position of the sensor module 510_2. The intersection of the perpendicular LS1 and the perpendicular LS2 is the firing position D.

A perpendicular LS4 from the firing position D to a perpendicular LS3 connecting the sensor module 510_1 and the sensor module 510_2 divides the distance Ly between the sensor module 510_1 and the sensor module 510_2 into a distance Ly2 and a distance Ly3. The distance Ly2 and the distance Ly3 may be expressed by θ, ø, and Zk.

Specifically, when the ratio "Zn/Yn" of a peak value Zn to a peak value Yn in the detection data of the sensor modules 510_1 and 510_2 is obtained, $\tan ø=Z2/Y2$, $\tan θ=Z3/Y3$. Furthermore, $Ly2 \times \tan ø=Zk$, $Ly3 \times \tan θ=Zk$, $Ly2+Ly3=Ly$. As Z2/Y2, Z3/Y3, Ly2, Ly3, and Ly are all known, the distance Zk in the depth direction at the firing position may be calculated from these values.

Next, the information processing device 160 is described. FIG. 31 is a diagram illustrating an example of the hardware configuration of the information processing device 160. As illustrated in FIG. 31, the information processing device 160 includes a central processing unit (CPU) 1501, a read only memory (ROM) 1502, and a random access memory (RAM) 1503. The CPU 1501, the ROM 1502, and the RAM 1503 form what is called a computer.

The information processing device 160 includes an auxiliary storage device 1504, a display device 1505, an operating device 1506, an interface (I/F) device 1507, and a drive device 1508. The hardware devices of the information processing device 160 are coupled to one another via a bus 1509.

The CPU 1501 is an arithmetic device that executes various programs (e.g., programs (referred to as information processing programs) for implementing the signal processor 560, the controller 562, and the display controller 563 described above) installed in the auxiliary storage device 1504.

The ROM 1502 is a non-volatile memory. The ROM 1502 functions as a primary memory device that stores various programs, data, and the like, for the CPU 1501 to execute various programs installed in the auxiliary storage device 1504. Specifically, the ROM 1502 functions as a primary memory device that stores boot programs such as Basic Input/Output System (BIOS) or Extensible Firmware Interface (EFI), and the like.

The RAM 1503 is a volatile memory such as a dynamic random access memory (DRAM) or a static random access memory (SRAM). The RAM 1503 functions as a primary memory device that provides a work area that is loaded when various programs installed in the auxiliary storage device 1504 are executed by the CPU 1501.

The auxiliary storage device 1504 is an auxiliary storage device that stores various programs and information acquired when various programs are executed. For example, the data storage 561 is implemented by the auxiliary storage device 1504.

The display device 1505 is a display device that displays various types of image data (the image data 310, the waveform data 320, etc.). The operating device 1506 is an input device that allows a doctor, or the like, to input various instructions to the information processing device 160. The I/F device 1507 is a communication device that is coupled to the ultrasonic measuring device 120, the magnetic shield box 110, the electrical stimulation device 130, etc. so that the information processing device 160 communicates with each device.

The drive device 1508 is a device in which a recording medium 1510 is set. The recording medium 1510 described here includes a medium that records information optically, electrically, or magnetically, such as a CD-ROM, a flexible disk, or a magnetooptical disk. The recording medium 1510 may include a semiconductor memory that electrically records information, such as a ROM or a flash memory.

Various programs installed in the auxiliary storage device 1504 are installed when, for example, the distributed recording medium 1510 is set in the drive device 1508 and various programs recorded in the recording medium 1510 are read. Alternatively, various programs installed in the auxiliary storage device 1504 may be installed by being downloaded via the network.

Next, a functional configuration of the information processing device 160 is described in detail. FIG. 32 is a diagram illustrating details of the functional configuration of the information processing device 160.

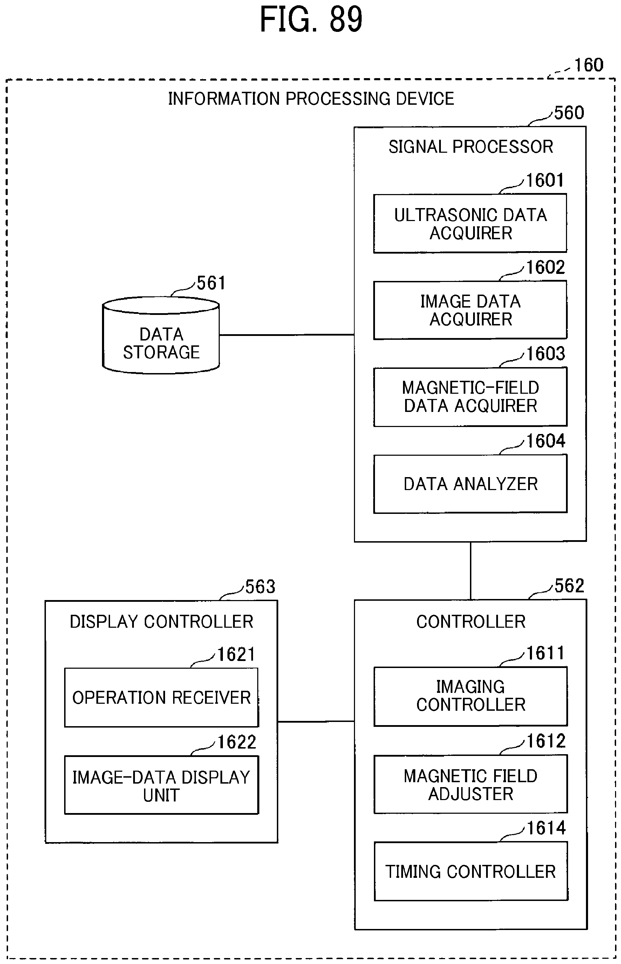

As illustrated in FIG. 89, the signal processor 560 includes the ultrasonic data acquirer 1601, the image data acquirer 1602, the magnetic-field data acquirer 1603, and the data analyzer 1604.

The ultrasonic data acquirer 1601 acquires the ultrasonic data transmitted from the ultrasonic measuring unit 520, calculates the thickness of the fat at a predetermined position in the palm of the subject 200 based on the acquired ultrasonic data, and stores the thickness of the fat in the data storage 561.

The image data acquirer 1602 acquires the image data transmitted from the camera 511 and stores the acquired image data in the data storage 561.

The magnetic-field data acquirer 1603 acquires the magnetic-field data transmitted from the sensor module 510 and stores the magnetic-field data in the data storage 561.

The data analyzer 1604 reads the magnetic-field data stored in the data storage 561, interpolates the magnetic-field data to generate waveform data, calculates the numerical data based on the waveform data, and stores the waveform data and the numerical data in the data storage 561.

As illustrated in FIG. 32, the controller 562 includes an imaging controller 1611, a magnetic field adjuster 1612, and a timing controller 1614.

The imaging controller 1611 transmits an imaging instruction to the camera 511 in response to a received imaging instruction from an operation receiver 1621 of the display controller 563.

After receiving an instruction for starting the magnetic field measurement from the operation receiver 1621 of the display controller 563, the magnetic field adjuster 1612 acquires the internal magnetic-field data measured by the MI sensor 512, calculates the current value of the current flowing through the coil 513, and transmits the current value to the current generator 540.

After receiving an instruction for starting the magnetic field measurement from the operation receiver 1621 of the display controller 563, the timing controller 1614 transmits the instruction to the electrical stimulation controller 530 at predetermined timing.

As illustrated in FIG. 32, the display controller 563 includes the operation receiver 1621 and an image-data display unit 1622.

In response to an input imaging instruction, the operation receiver 1621 notifies the imaging controller 1611 of the imaging instruction. Furthermore, in response to an instruction for starting the magnetic field measurement input from a doctor, or the like, the operation receiver 1621 notifies the magnetic field adjuster 1612 and the timing controller 1614 of the instruction.

The image-data display unit 1622 reads at least one of the waveform data and the numerical data on the muscle magnetic field stored in the data storage 561 and displays at least one of the waveform data and the numerical data.

Next, the positional relationship between the position where the electrical stimulation is applied to the subject 200 with the attached input terminal 130b of the electrical stimulation device 130 and the position where the magnetic field is detected by the sensor module 510 is described.

FIG. 36 is a diagram illustrating the positional relationship between the position where the electrical stimulation is applied and the position where the magnetic field is detected. As illustrated in FIG. 36, the input terminal 130b of the electrical stimulation device 130 is attached to a part (e.g., the elbow, which is a site different from the palm) of the arm of the subject 200.

The palm 220 is placed at a predetermined position inside the shielding member 600, and the sensor module 510 detects the magnetic field at the position. Therefore, the position where the electrical stimulation is applied is separated from the position where the magnetic field is detected by a predetermined distance. As a result, there is a time difference corresponding to the predetermined distance from when the electrical stimulus is applied until the sensor module 510 detects the magnetic field.

Next, a diagnostic operation when the doctor or the like performs a diagnosis based on the muscle magnetic field of the skeletal muscle of the subject 200 using the biomagnetic-field measurement apparatus 100 is described. FIG. 34 is a diagram illustrating the flow of an operation to make a diagnosis based on the muscle magnetic field of the skeletal muscle. Here, an example of detecting the muscle magnetic field of the abductor pollicis brevis of the hand is described.

At Step S1901, the doctor, or the like, uses the ultrasonic measuring device 120 to perform the ultrasonic measurement of the palm of the subject 200. Thus, the ultrasonic data is transferred from the ultrasonic measuring device 120 to the information processing device 160.

At Step S1902, the ultrasonic data acquirer 1601 of the information processing device 160 processes the ultrasonic data on the subject 200 and calculates the thickness of the fat at the position corresponding to the abductor pollicis brevis of the hand of the subject 200.

At Step S1903, the doctor or the like prompts the subject 200 to insert the arm into the shielding member 600 and place the hand of the subject 200 at a predetermined position. It is preferable to form a guide inside the shielding member 600 so that the hand of the subject 200 is in a generally appropriate position. This allows the abductor pollicis brevis of the subject 200 to be guided roughly to the position of the sensor module. It is preferable to lightly fix the hand of the subject 200 with an auxiliary guide in the above state.

At Step S1904, the doctor, or the like, attaches the input terminal 130b of the electrical stimulation device 130 to the arm of the subject 200.

At Step S1905, the doctor, or the like, operates the information processing device 160 to input an imaging instruction and drive the camera 511. Thus, the camera 511 captures the palm of the subject 200 placed at the predetermined position and transmits the image data to the information processing device 160.

At Step S1906, the image data acquirer 1602 of the information processing device 160 acquires the image data on the palm of the subject 200.

At Step S1907, the doctor or the like operates the information processing device 160 to input an instruction for starting the magnetic field measurement.

At Step S1908, the magnetic field adjuster 1612 acquires the internal magnetic-field data measured by the MI sensor 512 and calculates the current value. The current generator 540 applies the current with the calculated current value to the coil 513 to reduce the magnetic field inside the shielding member 600.

As described above, the magnetic field at the position of the sensor module 510 inside the shielding member 600 may be reduced to, for example, approximately 100 fT by using the gradient magnetic field. Thus, an extremely weak magnetic field (e.g., 500 Hz at 1 pT) generated by a skeletal muscle may be detected.

At Step S1909, each unit of the biomagnetic-field measurement apparatus 100 performs a magnetic-field detection process. Details of the magnetic-field detection process are given later.

At Step S1910, the image-data display unit 1622 displays the muscle magnetic-field data on the skeletal muscle of the subject 200. For example, the information processing device 160 displays the waveform data 320 on the muscle magnetic field of the abductor pollicis brevis illustrated in FIG. 3B.

At Step S1911, the doctor or the like diagnoses the muscle magnetic field of the skeletal muscle of the subject 200 based on the data obtained at Step S1909.

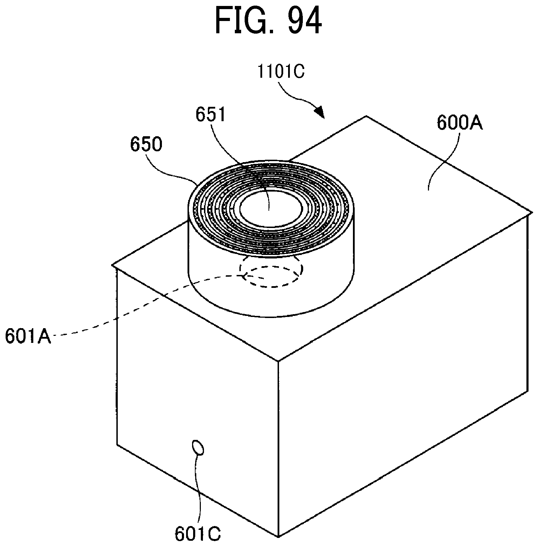

Next, the details of the magnetic-field detection process (Step S1910) are described. FIG. 94 is a flowchart illustrating the flow of the magnetic-field detection process.

At Step S2001, the doctor, or the like, prompts the subject 200 to make a voluntary motion (weak compression action). Here, the electrical stimulation device 130 stops electrical stimulation to the subject 200.

At Step S2002, the sensor module 510 starts to detect the magnetic field during the voluntary motion.

At Step S2003, while monitoring the detection result of the sensor module 510, the doctor, or the like, guides the subject 200 in voice so as to have an appropriate compressed state so that the appropriate waveform is output.

At Step S2004, the sensor module 510 continuously detects the muscle magnetic field of the abductor pollicis brevis during the voluntary motion for approximately one minute.

At Step S2005, the sensor module 510 stops detecting the magnetic field, and the magnetic-field data acquirer1603 acquires the data on the muscle magnetic field of the abductor pollicis brevis.

At Step S2006, the magnetic-field data acquirer1603 stores the acquired data on the muscle magnetic field of the abductor pollicis brevis together with the position data in the data storage 561.

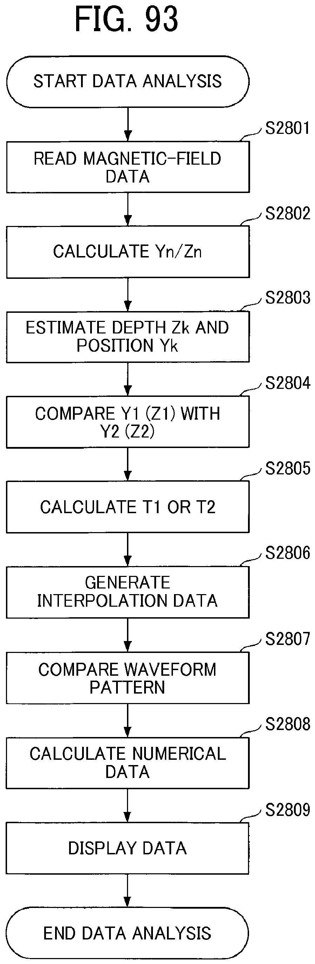

Next, data analysis is described. FIG. 93 is a flowchart illustrating an example of the flow of data analysis. It is assumed that the sensor modules are arranged as illustrated in FIG. 29.

At Step S2101, the data analyzer 1604 reads the magnetic-field data from the data storage 561. As each of the sensor modules 510_1 to 510_3 has data on the two axes, the data analyzer 1604 read six data sets in total. Here, each data is described as Yn, Zn. Data on the sensor modules adjacent in the Y direction are denoted by Y2 and Y3, and data on the sensor modules adjacent in the X-axis direction are denoted by Y1 and Y2.

At Step S2102, the data analyzer 1604 calculates Yn/Zn for all the data read at Step S2101. Information on a depth direction may be obtained from the values of Yn/Zn, as described above.

At Step S2103, the data analyzer 1604 refers to the ultrasonic-measurement form data (i.e., data on the thickness of fat at the position corresponding to the abductor pollicis brevis of the hand of the subject 200) obtained at Step S1902 to estimate the depth Zk and a position Yk. After Zk is determined, the depth may be corrected according to the Biot-Savart law.

At Step S2104, the data analyzer 1604 compares Y1 (Z1) with Y2 (Z2). The principle of the processes from Step S2104 to Step S2106 is as described above referring to FIGS. 23 and 26.

At Step S2105, the data analyzer 1604 calculates the amount of time gap (T1 or T2) illustrated in FIG. 22 or 25 based on the comparison result at Step S2104.

At Step S2106, the data analyzer 1604 performs data shift based on the values of T1 and T2 calculated at Step S2105, generates interpolation data, and stores, in the data storage 561, the waveform data for which the interpolation data has been generated. Due to the interpolation, the measurement data of 200 Hz (a sampling rate of 5 msec) may be changed into, apparently, the waveform data of 400 Hz (a sampling rate of 2.5 msec).

At Step S2107, the data analyzer 1604 compares the waveform data, for which the interpolation data has been generated at Step S2106, with the typical waveform pattern previously stored in the data storage 561.

At Step S2108, the data analyzer 1604 calculates numerical data for four items, e.g., multiphase/single phase, waveform length, waveform amplitude (intensity), and frequency, based on the comparison result at Step S2107 and stores the numerical data in the data storage 561.

At Step S2109, the image-data display unit 1622 causes the information processing device 160 to display at least one of the waveform data and the numerical data stored in the data storage 561.

As described above, in the magnetic shield box 1101 according to the present embodiment, the MI sensor 512 and the coil 513 are provided inside the shielding member 600 in order to cancel the residual magnetic field inside the shielding member 600. Specifically, the MI sensor 512 is disposed between the opening 601 and the measurement position (the position of the sensor module 510) inside the shielding member 600, and the coil 513 is disposed between the opening 601 and the MI sensor 512.

Then, the magnetic field is detected by the MI sensor 512, the current determined based on the value measured by the MI sensor 512 is supplied to the coil 513 from outside the shielding member 600, and the residual magnetic field inside the shielding member 600 is canceled by the electromagnetic field generated by the coil 513. Specifically, the magnetic field near the opening 601 is reduced to approximately 100 pT, which is the limit of the sensitivity of the MI sensor 512.

In the shielding member 600, a gradient magnetic field is formed, which has a smaller magnetic field in accordance with an increase in the distance from the opening 601. Therefore, when the magnetic field near the opening 601 is approximately 100 pT, the magnetic field is approximately 100 fT at the position where the shield coefficient is approximately 1000 (the position far from the opening 601).

As the sensor module 510 is provided at a position where the magnetic field has been reduced to approximately 100 fT, the sensor module 510 may detect an extremely weak magnetic field (e.g., 500 Hz at 1 pT) generated by a muscle.

As the response speed of the MI sensor 512 is 1 kHz or more, the feedback at this speed makes it possible to form a magnetic field sufficient to detect an extremely weak magnetic field (e.g., 500 Hz at 1 pT) generated by a muscle and to measure a muscle magnetic field with less noise and high accuracy.

As the sensor modules 510 are provided in at least one of the direction parallel to and the direction perpendicular to the potential propagation direction P of a muscle fiber (the direction of a muscle fiber) of the abductor pollicis brevis 250, high-accuracy magnetic field measurement is achieved.

## SECOND EMBODIMENT OF THIRD GROUP OF EMBODIMENTS

In the example described according to a second embodiment of the third group of embodiments, a mechanism is provided at the opening of the shielding member to vary the opening area. In the second embodiment of the third group of embodiments, the description of the same components as those in the above-described embodiment may be omitted.

Generally, to provide the opening through which the arm is inserted into the shielding member, it is convenient to make the opening as narrow as possible to prevent the external noise from entering. However, it is desirable to make the opening in accordance with the subject having the largest arm so that all the subjects are able to use the shielding member. As the body size varies depending on individual differences, it is desirable to make the size of the opening optimal for the subject. According to the embodiment, a mechanism is provided at the opening of the shielding member to vary the opening area. The mechanism at the opening of the shielding member to vary the opening area is described below in detail.

FIG. 94 is a perspective view illustrating a magnetic shield box 1101C according to the second embodiment of the third group of embodiments. As illustrated in FIG. 94, the magnetic shield box 1101C is different from the magnetic shield box 1101A (see FIGS. 82A and 82B) in that a chimney structure 650 is provided at the opening 601A.

The chimney structure 650 is a mechanism that varies the opening area of the opening 601A. The chimney structure 650 is an annular member provided with an insertion hole 651 in the center thereof to insert the arm. The chimney structure 650 includes a flexible material. The chimney structure 650 is attached around the opening 601A in a detachable manner.

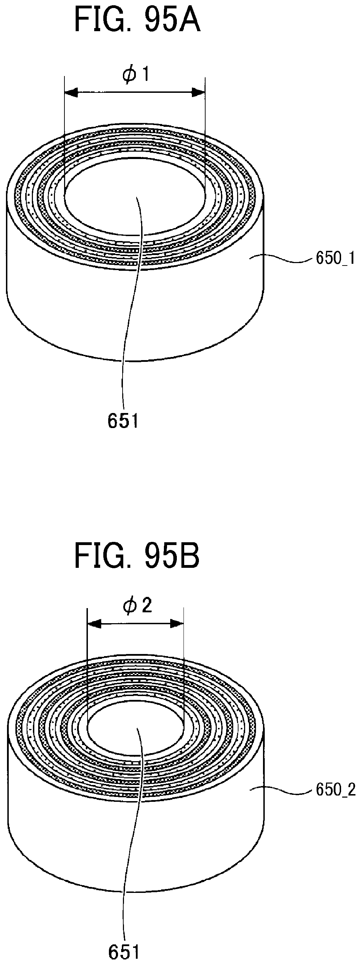

For example, a chimney structure 650_1 having the insertion hole 651 with a relatively large diameter ø1, as illustrated in FIG. 95A, and a chimney structure 650_2 having the insertion hole 651 with a relatively small diameter ø2, as illustrated in FIG. 95B, are prepared.

The chimney structure 650_1 or 650_2 is attached to the opening 601A in accordance with the size of the subject's arm to reduce the gap between the arm of the subject and the inner wall of the insertion hole 651 so that the noise entering the inside of the shielding member 600A from outside may be reduced. As a result, the shielding performance of the magnetic shield box 1101C may be improved. The three or more types of chimney structures 650 may be prepared.

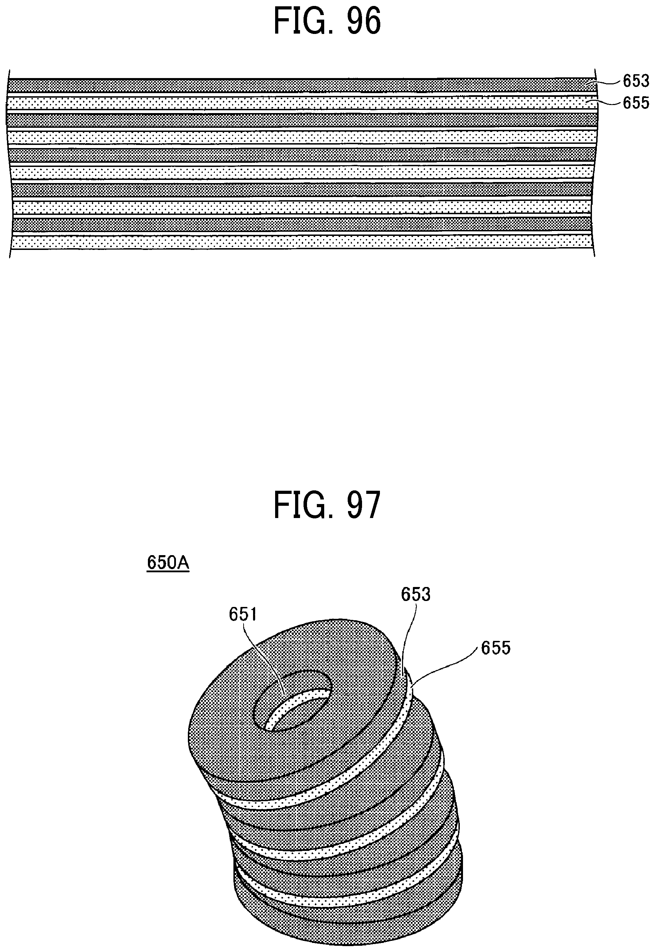

FIG. 96 is a diagram illustrating the chimney structure 650 before processing. For example, the chimney structure 650 is an annular structure that is formed by deforming a laminated product in which strips of flexible amorphous metallic foil 653 and strips of elastic member 655 are laminated, as illustrated in FIG. 96.

The amorphous metallic foil 653 has a thickness of, for example, several tens of $\mu$m. As the amorphous metallic foil 653, for example, FINEMET (registered trademark) manufactured by Hitachi Metals, Ltd. may be used. The elastic member 655 includes, for example, a polyethylene terephthalate film or a polycarbonate film having a thickness of approximately 50 $\mu$m. The amorphous metallic foil 653 and the elastic member 655 are sequentially laminated to form a flexible structure in which the amorphous metallic foil 653 is held between the elastic members 655 as the base material.

The conventional shielding member uses a crystalline metallic material, such as permalloy, and is hard. On the other hand, the amorphous metallic foil 653 has high permeability and high shielding performance regardless of its thinness. The amorphous metallic foil 653 is rapidly cooled from a high-temperature state where the amorphous metallic foil 653 is amorphous so that the amorphous metallic foil 653 is hardened without being crystallized while maintaining the amorphous state. The rapid cooling may allow the amorphous metallic foil 653 to have a thinness of several tens of $\mu$m.

As the opening 601A is covered with the chimney structure 650 that is formed by annularly deforming the laminated product in which strips of the flexible amorphous metallic foil 653 and strips of the elastic member 655 are laminated, the shape of the insertion hole 651 may be made large or small. For example, the opening area may be varied such that the shape of the insertion hole 651 is enlarged to insert the arm and the insertion hole 651 is sealed to prevent the magnetic field from entering during the measurement, whereby the shielding performance during the measurement may be improved.

As described above, the chimney structures 650 having the insertion holes 651 with different sizes are prepared and selectively attached in accordance with the subject to produce the same effect as the variable shape of the opening.

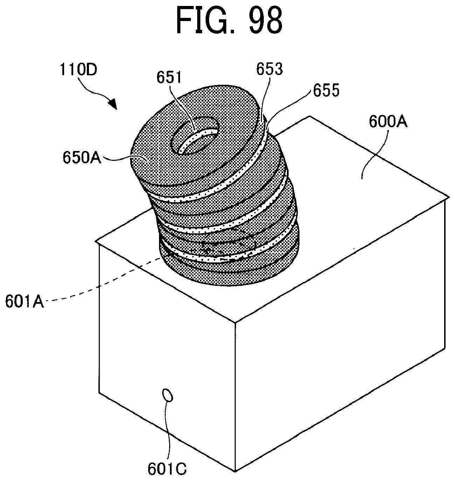

FIGS. 97 and 98 are diagrams illustrating another example of a chimney structure. As illustrated in FIG. 97, an accordion chimney structure 650A may be used. In a magnetic shield box 110D illustrated in FIG. 98, as the accordion chimney structure 650A is provided at the opening 601A, the chimney structure 650A may be deformed in accordance with the form of the arm when the arm is inserted into the insertion hole 651. Thus, the subject's physical stress may be reduced, and individual differences among the subjects may be eliminated.

The accordion chimney structure 650A may be formed by, for example, making a plurality of pairs of laminated products of the amorphous metallic foil 653 and the elastic member 655, fixing one side of the laminated product in each pair, and making the other side of the laminated product unfixed.

THIRD EMBODIMENT OF THIRD GROUP OF EMBODIMENTS

According to a third embodiment of the third group of embodiments, an example of the variations of the shape of a shielding member is described. In the third embodiment of the third group of embodiments, the descriptions of the same components as those in the above-described embodiment may be omitted.

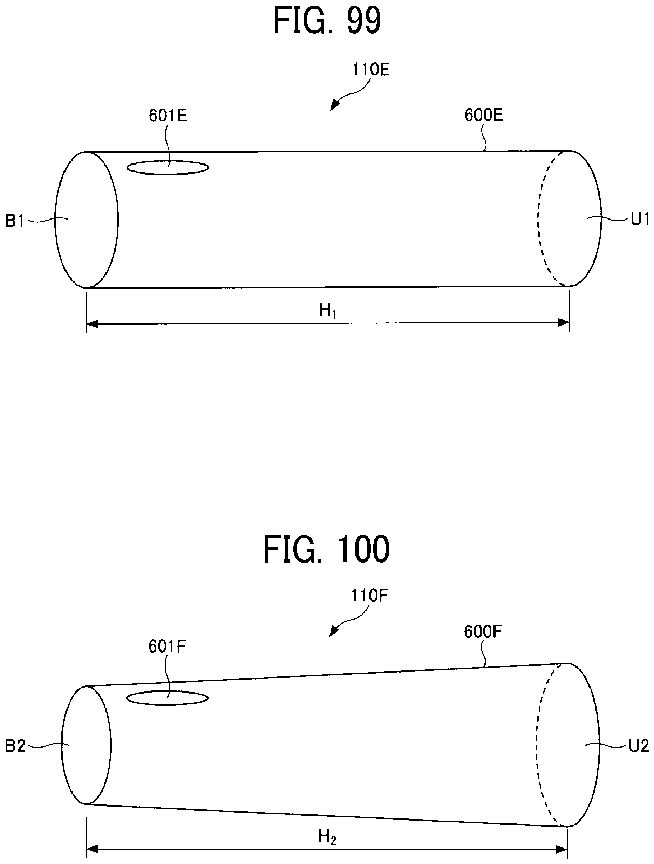

FIG. 99 illustrates a first variation of the shape of a shielding member 600E. As in a magnetic shield box 110E illustrated in FIG. 99, an opening 601E may be provided on the side surface of the cylindrical shielding member 600E.

The shielding member 600E is a cylindrical shape in which, when one surface of the shielding member 600E is a bottom surface B1 and the surface opposite to the bottom surface B1 is an upper surface U1, a height $H_1$ (the distance between the bottom surface B1 and the upper surface U1) is longer than the diameters of the bottom surface B1 and the upper surface U1. The opening 601E is provided near the bottom surface B1. The diameter of the bottom surface B1 is equal to the diameter of the upper surface U1.

In the magnetic shield box 110E, as the height $H_1$ of the shielding member 600E is long, a high shielding performance may be maintained at the position (the measurement position) far from the opening 601E.

FIG. 100 illustrates a second variation of the shape of a shielding member 600F. As in a magnetic shield box 110F illustrated in FIG. 100, an opening 601F may be provided on the side surface of the shielding member 600F having a truncated cone shape with a hollow.

The shielding member 600F has a hollow truncated cone shape in which, when one surface of the shielding member 600F is a bottom surface B2 and the surface opposite to the bottom surface B2 is an upper surface U2, a height $H_2$ (the distance between the bottom surface B2 and the upper surface U2) is longer than the diameters of the bottom surface B2 and the upper surface U2. The opening 601F is provided near the bottom surface B2. The diameter of the bottom surface B2 is smaller than the diameter of the upper surface U2.

In the magnetic shield box 110F, as the height $H_2$ of the shielding member 600F is long, a high shielding performance may be maintained at the position (the measurement position) far from the opening 601F. Furthermore, as there is a wide space at the position far from the opening 601F, a sufficient space for providing the sensor module 510, etc., may be ensured in the back while a high shielding performance is maintained.

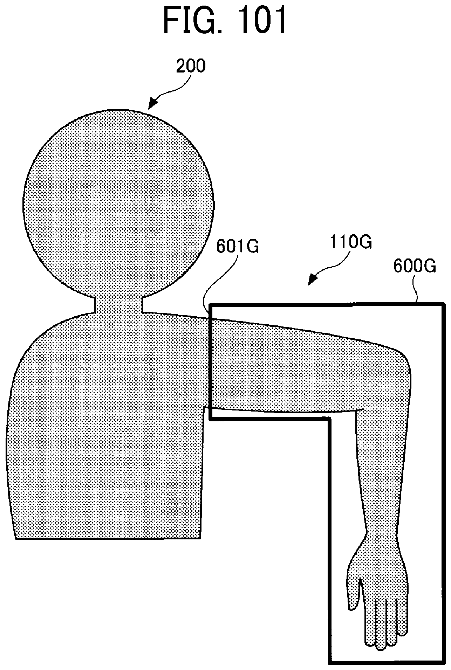

FIG. 101 illustrates a third variation of the shape of a shielding member 600G. As in a magnetic shield box 110G illustrated in FIG. 101, an opening 601G may be provided on the bottom surface of the hollow L-shaped shielding member 600G.

The shielding member 600G is a hollow L-shape having a circular or rectangular cross-sectional shape and, for example, the circular opening 601G is provided on the bottom surface. The area of the cross-sectional surface may be constant or may vary depending on the position. For example, when a wide space is formed at the position far from the opening 601G, a sufficient space for providing the sensor module 510, etc., may be ensured in the back while a high shielding performance is maintained.

In the magnetic shield box 110G, the L-shaped shielding member 600G may improve the shielding performance, as compared to the linear shape. Furthermore, the L-shaped shielding member 600G allows the limb of the subject 200 to be held in an easy position.

Among skeletal muscles, arms and legs are flexible, and therefore an L-shaped shielding member may be produced. On the other hand, it is difficult to produce an L-shaped shielding member for muscle magnetic fields in the cardiac muscle and the smooth muscle, nerve signals of the head, etc.

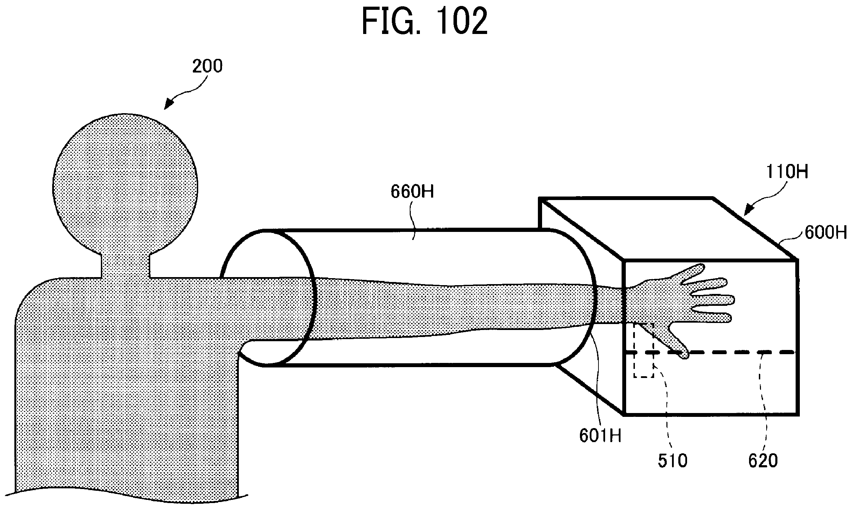

FIG. 102 illustrates a fourth variation of the shape of a shielding member 600H. As in a magnetic shield box 110H illustrated in FIG. 102, an auxiliary shielding member 660H may be attached to or detachable from an opening 601H of the shielding member 600H. The shielding member 600H may include, for example, permalloy. As the auxiliary shielding member 660H, for example, FINEMET (registered trademark) manufactured by Hitachi Metals, Ltd. may be used.

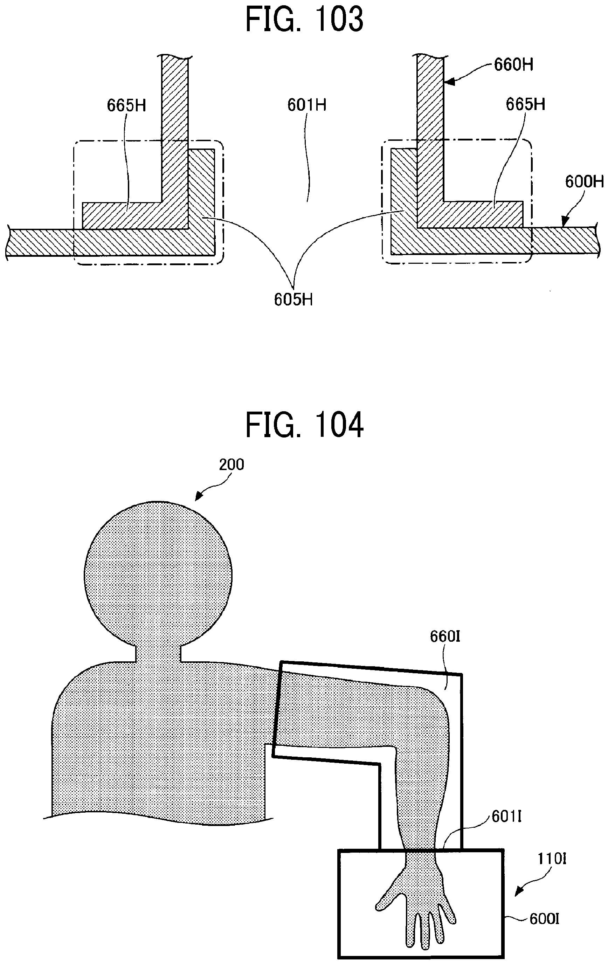

FIG. 103 is a diagram illustrating a structure with which the shielding member 600H and the auxiliary shielding member 660H are attached to or detached from each other. For example, as illustrated in FIG. 103, the circumstance of the opening 601H of the shielding member 600H is bent outward to form a bent portion 605H. The end of the auxiliary shielding member 660H is bent outward to form a bent portion 665H. The end of the auxiliary shielding member 660H is engaged with the outer peripheral side of the bent portion 605H of the shielding member 600H with such a force that the end of the auxiliary shielding member 660H is attached or detached.

As described above, the auxiliary shielding member 660H is attached to and detached from the opening 601H of the shielding member 600H so that it is easy for the subject 200 to insert the arm. At the joint portion (the portion indicated by the alternate long and short dash line in FIG. 103) between the shielding member 600H and the auxiliary shielding member 660H, the thickness of the shielding members is increased, and therefore the shielding performance may be maintained.

FIG. 104 illustrates a fifth variation of the shape of a shielding member 600I. As in a magnetic shield box 110I illustrated in FIG. 104, an L-shaped auxiliary shielding member 660I is attached to and detachable from an opening 601I of the shielding member 600I. The shielding member 600I may include, for example, permalloy. As the auxiliary shielding member 660I, for example, FINEMET (registered trademark) manufactured by Hitachi Metals, Ltd. may be used. A specific attaching structure may be the same as that in FIG. 103, for example.

FOURTH EMBODIMENT OF THIRD GROUP OF EMBODIMENTS

In an example described according to a fourth embodiment of the third group of embodiments, a high-permeability structure is provided inside the shielding member. In the fourth embodiment of the third group of embodiments, the descriptions of the same components as those in the above-described embodiment may be omitted.

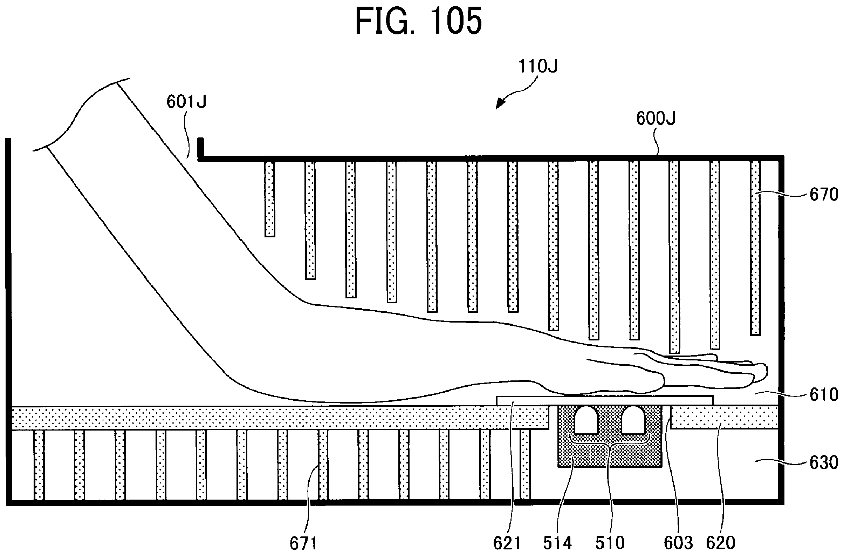

FIG. 105 is a schematic diagram illustrating an example of the internal configuration of a magnetic shield box 110J according to the fourth embodiment of the third group of embodiments.

As illustrated in FIG. 105, in the first space 610 of the magnetic shield box 110J, a plurality of flexible structures 670 hangs down at a predetermined interval from the inner wall of a shielding member 600J opposed to the boundary member 620 in substantially a vertical direction toward the boundary member 620. That is, in the first space 610, the flexible structures 670 is arranged substantially perpendicular to the magnetic field in the space between the vicinity of an opening 601J and the measurement position.

The length of each of the structures 670 is adjusted to have a length so as to be in contact with the hand or the arm of the subject 200. The structure 670 may move like a curtain due to the touch with the hand or the arm of the subject 200. As the shape (the thickness, etc.) of the hand or the arm of the subject 200 varies, the arm of the subject 200 may be in touch with all or part of the structure 670.

In the second space 630 of the magnetic shield box 110J, a plurality of flexible structures 671 hangs down at a predetermined interval from the lower surface side of the boundary member 620 in substantially a vertical direction toward the inner wall of the shielding member 600J opposed to the boundary member 620. The lower end of each of the structures 671 is in contact with the inner wall of the shielding member 600J.

The structures 670 and 671 include a high-permeability material. In this description, the high-permeability material refers to a material having a permeability of 1E-4 [H/m] or more.

The high-permeability material may be, for example, a laminated product of the flexible amorphous metallic foil 653 and the elastic member 655 illustrated in FIG. 96. As the amorphous metallic foil 653, for example, FINEMET (registered trademark) manufactured by Hitachi Metals, Ltd. may be used. The elastic member 655 includes, for example, a polyethylene terephthalate film or a polycarbonate film having a thickness of approximately 50 $\mu$m.

As described above, the structures 670 and 671 including a high-permeability material and provided inside the shielding member 600J of the magnetic shield box 110J may improve the shielding performance. Especially, the structure 670 including a high-permeability material and arranged substantially perpendicular to the magnetic field in the space between the vicinity of the opening 601J and the measurement position in the first space 610 is preferable in that it is possible to reduce the magnetic field entering through the opening 601J.

As the structures 670 and 671 including a high-permeability material are provided inside the shielding member 600J, the gradient magnetic field formed inside the shielding member 600J may be predominantly controlled in design. By predominantly controlling the gradient magnetic field in design, there may be more options for the specification of the feedback sensor. In other words, by increasing the gradient in the gradient magnetic field, it is possible to use, as a feedback sensor, a sensor with a lower detection sensitivity as compared with the MI sensor 512.

It is preferable that the structure 670 hangs down in substantially a vertical direction with respect to the gradient magnetic field formed in the space from the opening 601J to the measurement position where the sensor module 510 is provided. This is because the thickness of the structure 670 may be made as thin as possible and the shielding performance may be improved.

When the subject 200 comes into contact with the structure 670, the structure 670 flexibly changes its form like a curtain so that the structure 670 may be changed into a shape that matches the subject 200. The gap between the subject 200 and the structure 670 may be reduced to prevent noise entering during the measurement, and the shielding performance may be improved.

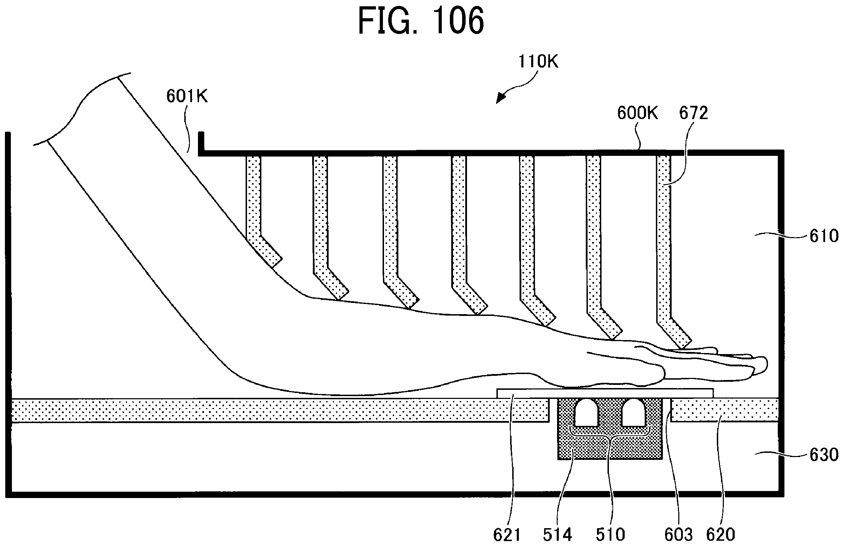

FIG. 106 is a schematic diagram illustrating another example of the internal configuration of a magnetic shield box 110K according to the fourth embodiment of the third group of embodiments.

As illustrated in FIG. 106, in the first space 610 of the magnetic shield box 110K, a plurality of flexible structures 672 hangs down at a predetermined interval from the inner wall of a shielding member 600K opposed to the boundary member 620 in substantially a vertical direction toward the boundary member 620. In the second space 630, there is no structure including a high-permeability material.

The length of each of the structures 672 is adjusted to be long so as to increase the probability of the contact with the hand or the arm of the subject 200. When the structure 672 is touched with the hand or the arm of the subject 200, the structure 672 moves like a curtain. As the structure 672 is flexible, the end of the structure 672 may be bent in accordance with the hand or the arm of the subject 200 due to the touch with the hand or the arm of the subject 200. The structure 672 may include a high-permeability material similar to that of the structure 670.

As described above, in the magnetic shield box 110K, the length of each of the structures 672 is adjusted to be long, and the probability of the contact with the hand or the arm of the subject 200 is increased; thus, the shielding performance may be further increased as compared to the magnetic shield box 110J. Other advantageous effects are the same as those of the magnetic shield box 110J.

In the second space 630, a structure including a high-permeability material may be provided as appropriate.

FIFTH EMBODIMENT OF THIRD GROUP OF EMBODIMENTS

In an example described according to a fifth embodiment of the third group of embodiments, an auxiliary member is attached to the subject 200 to prevent the magnetic field from entering through the opening of the shielding member. In the fifth embodiment of the third group of embodiments, the description of the same components as those in the above-described embodiment may be omitted.

FIGS. 48A and 48B are diagrams illustrating the auxiliary member 680 according to the fifth embodiment of the first group of embodiments. FIG. 48A illustrates a state where the auxiliary member 680 is attached to the subject 200, and FIG. 48B illustrates the auxiliary member 680.

As illustrated in FIG. 48A, the auxiliary member 680 may be attached to an upper arm portion 210U of the subject 200. For example, the subject 200 first wears the auxiliary member 680 around the upper arm portion 210U and then wears the magnetic shield box 110.

The auxiliary member 680 includes, for example, a ring-shaped sponge member produced by knitting chemical fibers containing an amorphous metal, which is shield material. The upper arm portion 210U of the subject 200 is insertable into the inner side of the ring.

As the auxiliary member 680 is attached to the upper arm portion 210U of the subject 200, the magnetic field may be prevented from entering through the opening 601 of the shielding member 600 so that the residual magnetic field inside the shielding member 600 may be reduced.

The auxiliary member 680 is deformable so that the auxiliary member 680 is attachable to the upper arm portion 210U having a different shape depending on the sex or the individual difference. As the auxiliary member 680 is deformed when the subject 200 having the auxiliary member 680 attached to the upper arm portion 210U moves, the subject 200 feels less tired during the measurement, or the like.

SIXTH EMBODIMENT OF FIRST GROUP OF EMBODIMENTS

In a sixth embodiment of the first group of embodiments, an example of a magnetic shield box 110c that detects a magnetic field for a leg 230 of the subject 200 is described. In the sixth embodiment of the first group of embodiments, the description of the same components as those in the above-described embodiment may be omitted.

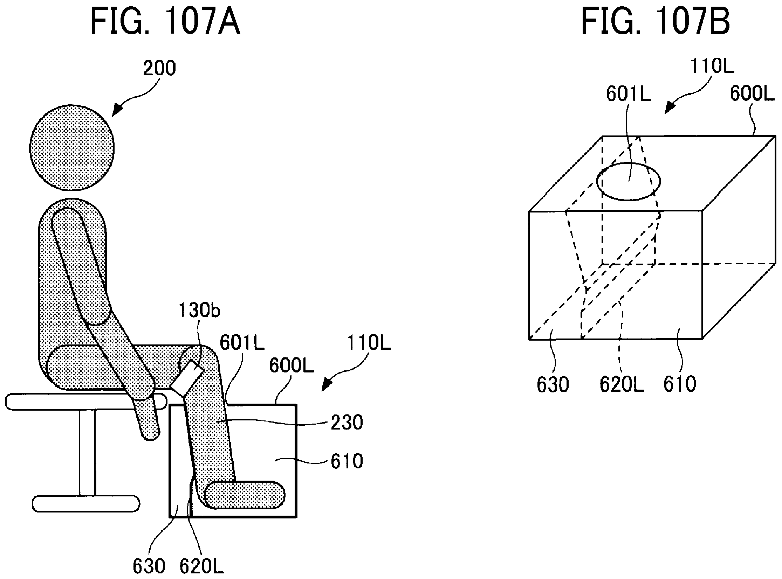

FIGS. 107A and 49B are diagrams illustrating the magnetic shield box 110c according to the sixth embodiment of the first group of embodiments. FIG. 107A illustrates a state where the magnetic shield box 110c is attached to the subject 200, and FIG. 107B illustrates the magnetic shield box 110c. In FIGS. 107A and 49B, although the sensor module 510, the MI sensor 512, and the like, are not illustrated, they may be appropriately placed at a position suitable for the magnetic field measurement of the leg 230 of the subject 200.

As illustrated in FIGS. 107A and 49B, a shielding member 600L of the magnetic shield box 110L is a cuboid and has an opening 601L, through which the leg 230 is insertable, on the top surface of the shielding member 600L. A boundary member 620L provided between the first space 610 and the second space 630 is bent in such a shape that the leg 230 may be easily inserted.

The configuration of the biomagnetic-field measurement apparatus except for the magnetic shield box 110L may be the same as the biomagnetic-field measurement apparatus 100 illustrated in, for example, FIG. 1.

In the biomagnetic-field measurement apparatus including the magnetic shield box 110L, for example, for the measurement of the magnetic field around the sural nerve of the leg 230, the subject 200 first sits on a chair and inserts the leg 230 into the shielding member 600L through the opening 601L. Then, the input terminal 130b is attached to the thigh (which may be a site located outside the shielding member 600L and different from the leg 230), or the like, of the subject 200. This makes it possible to apply electrical stimulation to the sural nerve and to measure a magnetic field around the sural nerve.

As described above, the site of the live subject targeted for the detection of a magnetic field is not limited to the hand of the subject and may be a leg that is a part of a limb of the subject 200.

In the case of muscular dystrophy, etc., a predictor is unlikely to appear in a muscle of the hand, and a predictor may appear in the leg. In that case, it is desirable to diagnose the muscle of the leg and, as described in the present embodiment, it is effective to use the technique for mearing a magnetic field around the sural nerve.

Although the shielding member 600L is a cuboid in the example of FIGS. 107A and 49B, the shielding member 600L may have any shape such as a cylindrical shape or a truncated cone shape.

SEVENTH EMBODIMENT OF THIRD GROUP OF EMBODIMENTS

According to a seventh embodiment of the third group of embodiments, an example of a magnetic shield box 110M in which a head 240 of the subject 200 is the target for which a magnetic field is detected is described. In the seventh embodiment of the third group of embodiments, the description of the same components as those in the above-described embodiment may be omitted.

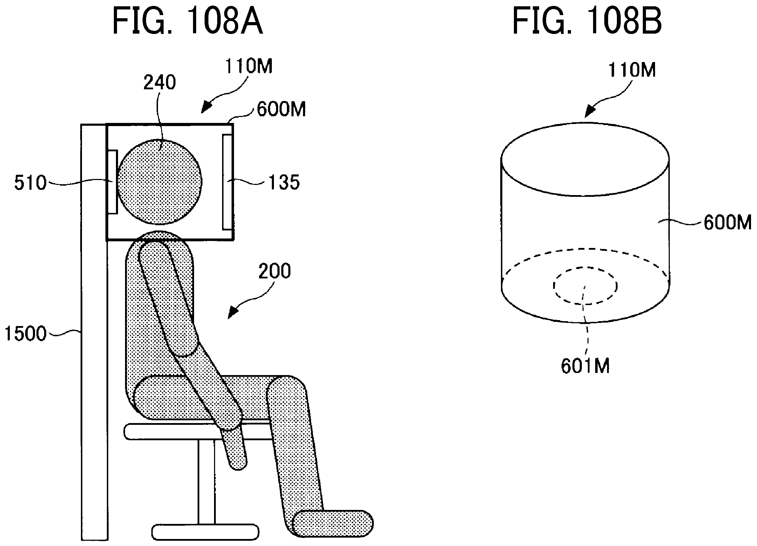

FIGS. 108A and 108B are diagrams illustrating the magnetic shield box 110M according to the seventh embodiment of the third group of embodiments. FIG. 108A illustrates a state where the magnetic shield box 110M is attached to the subject 200, and FIG. 108B illustrates the magnetic shield box 110M. Although not illustrated in FIGS. 108A and 108B, the MI sensor 512, and the like, may be appropriately provided at a position suitable for the measurement of a magnetic field of the head 240 of the subject 200.

As illustrated in FIGS. 108A and 108B, a shielding member 600M of the magnetic shield box 110M is cylindrical, and an opening 601M through which the head 240 is insertable is provided on the bottom surface. To allow the head 240 to be inserted, the periphery of the opening 601M includes a deformable flexible material.

The diameters of the bottom surface and the upper surface of the shielding member 600M may be, for example, approximately 30 cm. The height (the distance between the bottom surface and the upper surface) of the shielding member 600M may be, for example, approximately 40 cm. The diameter of the opening 601M may be, for example, approximately 15 cm.

The configuration of the biomagnetic-field measurement apparatus except for the magnetic shield box 110M may be the configuration of the biomagnetic-field measurement apparatus 1111 illustrated in FIG. 79, for example, including a visual stimulation device 135 instead of the electrical stimulation device 130. The visual stimulation device 135 is a device that induces a biomagnetic field (e.g., a screen that may display an image). The visual stimulation device 135 is placed at a position where the subject 200 inside the shielding member 600M may view the visual stimulation device 135.

To measure a magnetic field of the head 240 with the biomagnetic-field measurement apparatus including the magnetic shield box 110M, the subject 200 first sits on the chair and inserts the head 240 into the shielding member 600M through the opening 601M. The shielding member 600M is supported by, for example, a supporting pillar 1500. The visual stimulation device 135 displays an image to apply stimulation to the subject 200 so that the magnetic field of the head 240 may be measured.

As described above, the site of the live subject for which a magnetic field is detected is not limited to the hand of the subject 200 and may be the head of the subject 200. As the visual stimulation device 135 is disposed in the shielding member 600M, it is possible to give a realistic stimulus to the subject 200. As the magnetic shield box 110M is disposed around the head 240, the subject 200 can freely move the lower body, etc.

In the example of FIGS. 108A and 108B, the shielding member 600M is cylindrical; however, the shielding member 600M may have any shape, such as cuboid or truncated cone shape. The use of the magnetic shield box 110M enables the measurement of a magnetic field of the head 240 in a state where the subject 200 is standing as well as in a state where the subject 200 is sitting on the chair.

EIGHTH EMBODIMENT OF THIRD GROUP OF EMBODIMENTS

According to an eighth embodiment of the third group of embodiments, an example of a magnetic shield box 110N including no sensor module is described. In the eighth embodiment of the third group of embodiments, the description of the same components as those in the above-described embodiment may be omitted.

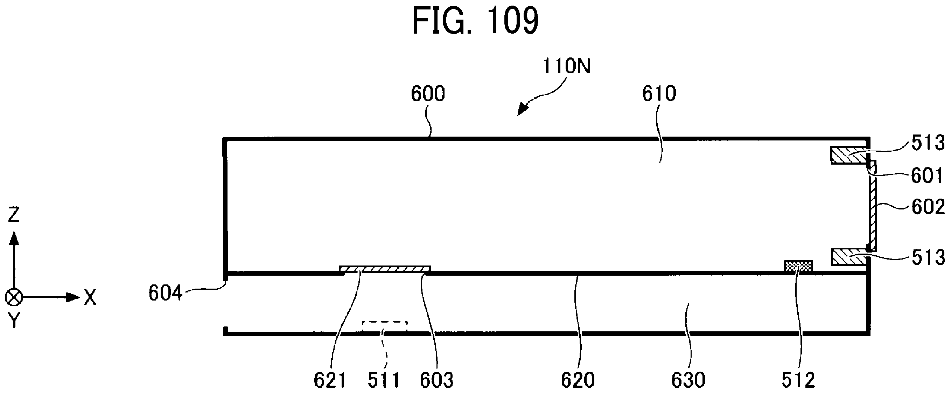

FIG. 109 is a diagram illustrating an example of the internal configuration of the magnetic shield box 110N according to the eighth embodiment of the third group of embodiments. As illustrated in FIG. 109, the magnetic shield box 110N is different from the magnetic shield box 1101 (see FIG. 81) in that the sensor module 510 is not provided and the shielding member 600 is provided with an insertion hole 604.

The insertion hole 604 is a hole through which the sensor module 510 is inserted into a measurement position (under the opening 603) in the second space 630.

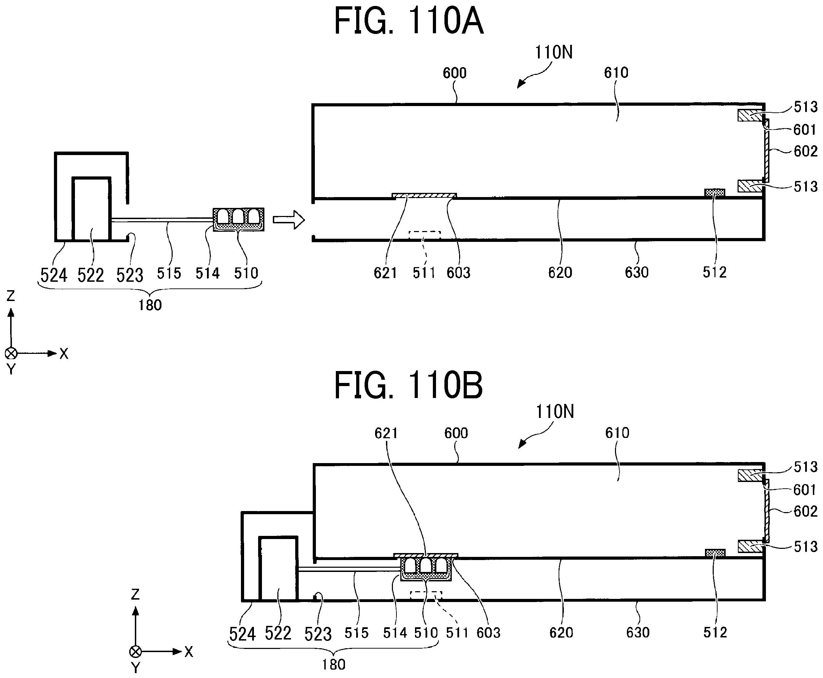

FIGS. 110A and 110B are diagrams illustrating the coupling between the magnetic shield box 110N and a module support device 180. FIG. 110A illustrates a situation before the module support device 180 is coupled to the magnetic shield box 110N, and FIG. 110B illustrates a situation after the module support device 180 is coupled to the magnetic shield box 110N.

As illustrated in FIGS. 110A and 110B, the magnetic shield box 110N may be coupled to the module support device 180 that is prepared separately from the magnetic shield box 110N.

The module support device 180 includes the at least one sensor module 510 held by the holder 514 and a drive 522 that adjusts the position of the holder 514 in the Z-axis direction. The drive 522 is fixedly mounted in a shielding member 524 including permalloy. The holder 514 holding the sensor module 510 is coupled to the drive 522 with the lever 515 through an opening 523 provided in the shielding member 524.

The module support device 180 is moved in the direction of the arrow in FIG. 110A so that the holder 514 holding the sensor module 510 is inserted into the second space 630 provided in the shielding member 600 of the magnetic shield box 110N.

As illustrated in FIG. 110B, after the holder 514 is inserted into the second space 630, the position of the holder 514 is adjusted in the Z-axis direction by the drive 522, whereby the end of the sensor module 510 held by the holder 514 may be brought into contact with the flexible film 621. The opposing surfaces of the shielding member 600 and the shielding member 524 are in close contact with each other so that the shielding performance inside the second space 630 may be maintained.

As described above, the magnetic shield box may omit a sensor module. The magnetic shield box may be provided with an insertion hole through which a sensor module is inserted from outside.

Although the embodiments have been described above in detail, there is no limitation on the above-described embodiments, and various modifications and substitutions may be made to the above-described embodiments without departing from the range described in the scope of claims.

In the example according to the first embodiment to the eighth embodiment of the third group of embodiments described above, for example, an optically pumped atomic magnetometer is used to measure a biomagnetic field. According to the first embodiment to the eighth embodiment of the third group of embodiments, however, a room-temperature magnetic sensor (e.g., MR (magneto resistive) sensor or a TMR (tunnel magneto resistance) sensor) other than an optically pumped atomic magnetometer may be used.

For example, although the MI sensor is used as a magnetic sensor for the feedback in the example described above according to the first embodiment to the eighth embodiment of the third group of embodiments, a solid magnetic sensor (e.g., a magneto resistive (MR) sensor, a tunnel magneto resistance (TMR) sensor) other than the MI sensor may be used.

According to the first embodiment to the eighth embodiment of the third group of embodiments described above, the position of the sensor module is adjustable in the shielding member.

Numerous additional modifications and variations are possible in light of the above teachings. It is therefore to be understood that within the scope of the appended claims, the disclosure of the present disclosure may be practiced otherwise than as specifically described herein. For example, elements and/or features of different illustrative embodiments may be combined with each other and/or substituted for each other within the scope of this disclosure and appended claims.

**Claims**

1.  A biomagnetic-field measurement apparatus (100) configured to measure a magnetic field of a site of a live subject, the biomagnetic-field measurement apparatus comprising a plurality of sensor modules (510) disposed at a measurement position of the site, each of the plurality of sensor modules (510) including an optically pumped atomic magnetometer.

2.  The biomagnetic-field measurement apparatus (100) according to claim 1, wherein
the site is a muscle, and
each of the plurality of sensor modules (510) is arranged parallel to a muscle fiber direction of the muscle.

3. The biomagnetic-field measurement apparatus (100) according to claim 1 or 2, wherein
the site is a muscle, and
each of the plurality of sensor modules (510) is arranged perpendicular to a muscle fiber direction of the muscle.

4. The biomagnetic-field measurement apparatus (100) according to claim 2 or 3, wherein the optically pumped atomic magnetometer is disposed such that the muscle fiber direction of the muscle is parallel to a light propagation direction of the optically pumped atomic magnetometer.

5. The biomagnetic-field measurement apparatus (100) according to any one of claims 1 to 4, further comprising:

   a hollow shielding member (600) configured to shield an external magnetic field; and
   an electrical stimulation device (130) configured to apply electrical stimulation to a subject (200) via an electrode disposed outside the shielding member (600).

6. The biomagnetic-field measurement apparatus (100) according to claim 5, wherein the electrical stimulation device (130) is configured to apply electrical stimulation to a median nerve of the subject (200).

7. The biomagnetic-field measurement apparatus (100) according to any one of claims 1 to 6, further comprising a cobalt-based amorphous metallic foil (653) sandwiched between each adjacent pair of the plurality of sensor modules (510).

8. A biomagnetic-field measurement method for measuring a magnetic field of a site of a live subject, the biomagnetic-field measurement method comprising:

   measuring magnetic field waveforms due to an eliciting stimulus multiple times by using a plurality of sensor modules (510) including an optically pumped atomic magnetometer;
   integrating the magnetic field waveforms with regard to the plurality of sensor modules (510);
   calculating a time gap T of the integrated magnetic field waveforms with regard to each adjacent pair of the plurality of sensor modules (510);
   measuring a spontaneous magnetic field waveform at a position identical to a position where the magnetic field waveforms due to the eliciting stimulus are measured by using the plurality of sensor modules (510);
shifting the spontaneous magnetic field waveform measured by one of the adjacent pair of the plurality of sensor modules (510) on a time axis by the time gap T relative to the spontaneous magnetic field waveform measured by another one of the adjacent pair of the plurality of sensor modules (510); and
combining the spontaneous magnetic field waveform measured by the one of the adjacent pair of the plurality of sensor modules (510) with the spontaneous magnetic field waveform measured by the another one of the adjacent pair of the plurality of sensor modules (510).

9. The biomagnetic-field measurement apparatus (100) according to claim 1, further comprising:

   a shielding member (600) configured to hold a part of a limb of a subject (200) inside the shielding member (600) and shield an electromagnetic wave; and
   a room-temperature magnetic sensor disposed in a direction corresponding to a longitudinal direction of the limb of the subject (200) when the part of the limb of the subject (200) is held inside the shielding member (600).

10. The biomagnetic-field measurement apparatus (100) according to claim 9, further comprising:

    a sensor module (510) including the room-temperature magnetic sensor;
    a holder (514) configured to hold the sensor module (510) such that an end of the sensor module (510) is pressed against and is brought into contact with the part of the limb of the subject (200); and
    a moving unit configured to move the holder (514) relative to the part of the limb of the subject (200) along a longitudinal direction of the limb of the subject (200).

11. The biomagnetic-field measurement apparatus (100) according to claim 10, wherein the sensor module (510) further includes a position sensor (1031) configured to detect a position of the sensor module (510) when the room-temperature magnetic sensor detects a magnetic field generated in the part of the limb of the subject (200).

12. The biomagnetic-field measurement apparatus (100) according to claim 10, wherein
the room-temperature magnetic sensor is an optically pumped atomic magnetometer, and
the optically pumped atomic magnetometer is disposed in the sensor module (510) such that a direction toward an end of the limb of the subject (200) is substantially parallel to a light propagation direction of the optically pumped atomic magnetometer.

13. The biomagnetic-field measurement apparatus (100) according to claim 12, wherein
the sensor module (510) includes a plurality of sensor modules (510),
the holder (514) holds the plurality of sensor modules (510), and
each of the plurality of sensor modules (510) is disposed in the holder (514) such that $X>Y$ is satisfied, where the light propagation direction of the optically pumped atomic magnetometer is $X_0$, a direction substantially perpendicular to the light propagation direction is $Y_0$, a distance between each adjacent pair of the plurality of sensor modules (510) in a direction parallel to $X_0$ is X, and a distance between each adjacent pair of the plurality of sensor modules (510) in a direction parallel to $Y_0$ is Y.

14. A magnetic shield box (110) comprising:

a hollow shielding member (600) configured to shield an external magnetic field, the shielding member (600) provided with an opening (601) for allowing insertion of a site of a live subject for which a magnetic field is measured;
a first magnetic sensor disposed at a measurement position of the site inside the shielding member (600);
a second magnetic sensor disposed between the opening (601) and the measurement position inside the shielding member (600); and
a coil (513) disposed between the opening (601) and the second magnetic sensor inside the shielding member (600), the coil (513) configured to be supplied with a current determined based on a value measured by the second magnetic sensor from outside the shielding member (600) to reduce a magnetic field inside the shielding member (600).

15. The magnetic shield box (110) according to claim 14, wherein a gradient magnetic field is formed from the opening (601) to the measurement position, the gradient magnetic field satisfying B1<B2, where a ratio of a magnetic field intensity at the measurement position to a magnetic field intensity at a position of the opening (601) is B1 and a ratio of a minimum value of a magnetic field to be measured to a minimum value of a magnetic field detectable by the second magnetic sensor is B2.

# FIG. 1

# FIG. 2

# FIG. 3A

# FIG. 3B

FIG. 4

# FIG. 5

EP 3 646 782 A1

# FIG. 6

EP 3 646 782 A1

# FIG. 7

EP 3 646 782 A1

FIG. 8

# FIG. 9

FIG. 10

EP 3 646 782 A1

# FIG. 11

$Z_0$

$Y_0$

$X_0$

PHOTODETECTOR

RUBIDIUM ATOM

IN CASE WHERE
NO MAGNETIC
FIELD IS
GENERATED

COIL

GAS CELL

COIL

LASER BEAM

INTENSITY

0
MAGNETIC FIELD

MAGNETIC
FIELD

MAGNETIC
FIELD

# FIG. 12

# FIG. 13

# FIG. 14

FIG. 15A

FIG. 15B

EP 3 646 782 A1

FIG. 16A

FIG. 16B

## FIG. 17

|  | SKELETAL MUSCLE | CARDIAC MUSCLE | SMOOTH MUSCLE |
|---|---|---|---|
| DISTRIBUTION | DISTRIBUTED IN MOTOR AND MUSCULAR SYSTEMS | HEART | DIGESTIVE SYSTEM, RESPIRATORY SYSTEM, AND BLOOD VESSEL |
| MUSCLE CELL | ELONGATED CYLINDRICAL SHAPE | BRANCHING WHILE COUPLING TO ADJACENT CELL VIA INTERCALATED DISC | ELONGATED SPINDLE SHAPE |
| NERVE CONTROL | MOTILE FIBER OF CRANIAL NERVE AND SPINAL NERVE | AUTONOMIC NERVE (SYMPATHETIC NERVE AND PARASYMPATHETIC NERVE) | AUTONOMIC NERVE (SYMPATHETIC NERVE AND PARASYMPATHETIC NERVE) |
| VOLUNTARY MOTION | POSSIBLE | IMPOSSIBLE | IMPOSSIBLE |

# FIG. 18

# FIG. 19

| | ABNORMAL | NORMAL | REMARK ON FACTOR |
|---|---|---|---|
| 1. SINGLE PHASE | | | NEUROLOGICAL FACTOR (ALS)<br>・NERVE CAUSES DELAY |
| 2. LENGTH | 1-2msec | 10msec | MUSCLE-FIBER FACTOR (MUSCULAR DYSTROPHY)<br>・SPIKYNESS DUE TO WEAKER MUSCLE FIBER |
| 3. INTENSITY | 10pT | 1pT | NEUROLOGICAL DECLINE (DUE TO RECRUITMENT) (ALS)<br>COMPLETE COMPARISON IS DIFFICULT DUE<br>TO DEPENDENCY ON DISTANCE FROM NEEDLE |
| 4. FREQUENCY | 5Hz | 20Hz | MUSCLE-FIBER FACTOR (MUSCULAR DYSTROPHY)<br>・DUE TO MUSCLE FIBER DECLINE |

EP 3 646 782 A1

# FIG. 20

| | WAVEFORM | IMAGE | REMARK ON FACTOR |
|---|---|---|---|
| 1. NORMAL | | | FLAT WAVEFORM |
| 2. Fib | | | MUSCLE FIBER SPONTANEOUS WAVEFORM (MUSCULAR DYSTROPHY) |
| 3. PSW | | | DUE TO MUSCLE FIBER BREAKING (MUSCULAR DYSTROPHY) |
| 4. Fac | | | CONVULSIVE SPONTANEOUSNESS (ALS, ETC.) (NEUROLOGICAL FACTOR) |

EP 3 646 782 A1

# FIG. 21A

ORIGINAL DATA
(RIGHT FINGER MUSCLE ATROPHY, NeedleEMG)

MEASUREMENT TIME [ms]

# FIG. 21B

1kHz

MEASUREMENT TIME [ms]

FIG. 21C

FIG. 21D

# FIG. 22

# FIG. 23

# FIG. 24

# FIG. 25

# FIG. 26

# FIG. 27

# FIG. 28

EP 3 646 782 A1

# FIG. 29

79

# FIG. 30

# FIG. 31

INFORMATION PROCESSING DEVICE — 160

| 1501 | 1502 | 1503 | 1504 |
|------|------|------|------|
| CPU | ROM | RAM | AUXILIARY STORAGE DEVICE |

— 1509

| 1505 | 1506 | 1507 | 1508 |
|------|------|------|------|
| DISPLAY DEVICE | OPERATING DEVICE | I/F DEVICE | DRIVE DEVICE |

— 1510

RECORDING MEDIUM

# FIG. 32

INFORMATION PROCESSING DEVICE ⌒160

SIGNAL PROCESSOR ⌒560

ULTRASONIC DATA ACQUIRER ⌒1601

IMAGE DATA ACQUIRER ⌒1602

MAGNETIC-FIELD DATA ACQUIRER ⌒1603

DATA ANALYZER ⌒1604

SOUND WAVE CONVERTER ⌒1605

DATA STORAGE ⌒561

DISPLAY CONTROLLER ⌒563

OPERATION RECEIVER ⌒1621

IMAGE-DATA DISPLAY UNIT ⌒1622

CONTROLLER ⌒562

IMAGING CONTROLLER ⌒1611

MAGNETIC FIELD ADJUSTER ⌒1612

TIMING CONTROLLER ⌒1614

# FIG. 33

# FIG. 34

```
      ┌─────────────────────┐
      │  START DIAGNOSTIC   │
      │     OPERATION       │
      └─────────────────────┘
                 │
                 ▼              ╱S1901
      ╱─────────────────────╲
      │ PERFORM ULTRASONIC  │
      │    MEASUREMENT      │
      ╲─────────────────────╱
                 │
                 ▼              ╱S1902
      ┌─────────────────────┐
      │  PROCESS ULTRASONIC │
      │        DATA         │
      └─────────────────────┘
                 │
                 ▼              ╱S1903
      ╱─────────────────────╲
      │    PLACE PALM AT     │
      │PREDETERMINED POSITION│
      ╲─────────────────────╱
                 │
                 ▼              ╱S1904
      ╱─────────────────────╲
      │ ATTACH INPUT TERMINAL OF │
      │ ELECTRICAL STIMULATION   │
      │DEVICE TO ARM OF SUBJECT  │
      ╲─────────────────────╱
                 │
                 ▼              ╱S1905
      ╱─────────────────────╲
      │     CAPTURE PALM     │
      ╲─────────────────────╱
                 │
                 ▼              ╱S1906
      ┌─────────────────────┐
      │  PROCESS IMAGE DATA │
      └─────────────────────┘
                 │
                 ▼              ╱S1907
      ╱─────────────────────╲
      │ GIVE INSTRUCTION FOR │
      │   STARTING MAGNETIC  │
      │  FIELD MEASUREMENT   │
      ╲─────────────────────╱
                 │
                 ▼              ╱S1908
      ┌─────────────────────┐
      │  ADJUST MAGNETIC FIELD │
      └─────────────────────┘
                 │
                 ▼              ╱S1909
      ╱─────────────────────╲
      │    ALIGN POSITION    │
      ╲─────────────────────╱
                 │
                 ▼              ╱S1910
      ┌┃───────────────────┃┐
      │┃  MAGNETIC-FIELD   ┃│
      │┃ DETECTION PROCESS ┃│
      └┃───────────────────┃┘
                 │
                 ▼              ╱S1911
      ┌─────────────────────┐
      │  PROCESS IMAGE DATA │
      └─────────────────────┘
                 │
                 ▼              ╱S1912
      ╱─────────────────────╲
      │    MAKE DIAGNOSIS    │
      ╲─────────────────────╱
                 │
                 ▼
      ┌─────────────────────┐
      │   END DIAGNOSTIC    │
      │     OPERATION       │
      └─────────────────────┘
```

# FIG. 35

```
        ┌─────────────────────┐
        │  START POSITIONAL   │
        │     ALIGNMENT       │
        └─────────────────────┘
                   │
                   ▼              S2001
        ┌─────────────────────┐
        │  APPLY ELECTRICAL   │
        │    STIMULATION      │
        └─────────────────────┘
                   │
                   ▼              S2002
        ┌─────────────────────┐
        │ ACQUIRE MAGNETIC-   │
        │    FIELD DATA       │
        └─────────────────────┘
                   │
                   ▼              S2003
        ┌─────────────────────┐
        │  STORE MAGNETIC-    │
        │    FIELD DATA       │
        └─────────────────────┘
                   │
                   ▼              S2004
        ┌─────────────────────┐
        │ GENERATE WAVEFORM   │
        │       DATA          │
        └─────────────────────┘
                   │
                   ▼              S2005
        ┌─────────────────────┐
        │ DISPLAY WAVEFORM    │
        │       DATA          │
        └─────────────────────┘
                   │
                   ▼              S2006
        ┌─────────────────────┐
        │ CONVERT WAVEFORM    │
        │ DATA INTO SOUND DATA│
        └─────────────────────┘
                   │
                   ▼              S2007
        ╱─────────────────────╲
        │    ALIGN POSITION    │
        ╲─────────────────────╱
                   │
                   ▼
        ┌─────────────────────┐
        │       RETURN        │
        └─────────────────────┘
```

EP 3 646 782 A1

## FIG. 36A

## FIG. 36B

## FIG. 36C

# FIG. 37

START MAGNETIC-FIELD
DETECTION PROCESS

S2101
PROMPT SUBJECT TO MAKE
VOLUNTARY MOTION

S2102
DETECT MAGNETIC FIELD
WITH SENSOR MODULE

S2103
GUIDE APPROPRIATE
COMPRESSED STATE

S2104
CONTINUOUSLY DETECT
MAGNETIC FIELD WITH
SENSOR MODULE

S2105
ACQUIRE MUSCLE
MAGNETIC-FIELD DATA

S2106
STORE MUSCLE
MAGNETIC-FIELD DATA

RETURN

# FIG. 38

START DATA ANALYSIS

↓ /S2201

READ MAGNETIC-FIELD DATA

↓ /S2202

CALCULATE Yn/Zn

↓ /S2203

ESTIMATE DEPTH Zk AND POSITION Yk

↓ /S2204

COMPARE Y1 (Z1) WITH Y2 (Z2)

↓ /S2205

CALCULATE T1 OR T2

↓ /S2206

GENERATE INTERPOLATION DATA

↓ /S2207

COMPARE WAVEFORM PATTERN

↓ /S2208

CALCULATE NUMERICAL DATA

↓ /S2209

DISPLAY DATA

↓

END DATA ANALYSIS

# FIG. 39A

# FIG. 39B

## FIG. 40A

## FIG. 40B

# FIG. 41A

# FIG. 41B

# FIG. 42

| | ESTIMATED WAVEFORM | DISPLAY (RANGE) | DETECTION ALGORITHM |
|---|---|---|---|
| 1. SINGLE PHASE | | SINGLE PHASE OR MULTIPHASE | MAKE DETERMINATION WITH STANDARD DEVIATION OF $\alpha 2$ TO $\alpha 7$ AS INDEX |
| 2. LENGTH | 1-2msec | 1msec~20msec | MAKE DETERMINATION WITH $\alpha 1$, $\alpha 2$, AND $\alpha 3$ |
| 3. INTENSITY | 10pT | 1pT~10pT | MAKE DETERMINATION WITH $\alpha 0$ |
| 4. FREQUENCY | 5Hz | 1Hz~20Hz | DISPLAY APPEARANCE FREQUENCY OF IDENTICAL MOTION UNIT AS FREQUENCY |

EP 3 646 782 A1

# FIG. 43

EP 3 646 782 A1

# FIG. 44

# FIG. 45

510_7　　　　　510_8　　　　　510_9　　OA

Ly

Lx

510_4　　　　　510_5　　　　　510_6

510_1　　　　　510_2　　　　　510_3　　　　　P

Y

Z　X

# FIG. 46

# FIG. 47

EP 3 646 782 A1

FIG. 48B

FIG. 48A

# FIG. 49A

# FIG. 49B

# FIG. 50

1011

# FIG. 51

# FIG. 52A

# FIG. 52B

# FIG. 53

# FIG. 54

INFORMATION PROCESSING DEVICE 1400

- DATA STORAGE 541
- SIGNAL PROCESSOR 5400
- CONTROLLER 542
- DISPLAY CONTROLLER 543

ULTRASONIC MEASURING DEVICE 120
- ULTRASONIC MEASURING UNIT 520

MEASURING UNIT 110a
- SENSOR MODULE 510
- CAMERA 511
- MI SENSOR 512

DRIVE
- CURRENT GENERATOR 519
- COIL 513
- MOVING UNIT 521
- HOLDER 514
- DETECTOR 517

110b

GENERATOR 130a
- ELECTRICAL STIMULATION CONTROLLER 530
- INPUT TERMINAL 130b

EP 3 646 782 A1

# FIG. 55

EP 3 646 782 A1

# FIG. 56

EP 3 646 782 A1

# FIG. 57

EP 3 646 782 A1

FIG. 58A

FIG. 58B

# FIG. 59

# FIG. 60

INFORMATION PROCESSING DEVICE 1400

CPU 1501

ROM 1502

RAM 1503

AUXILIARY STORAGE DEVICE 1504

1509

DISPLAY DEVICE 1505

OPERATING DEVICE 1506

I/F DEVICE 1507

DRIVE DEVICE 1508

RECORDING MEDIUM 1510

EP 3 646 782 A1

# FIG. 61

INFORMATION PROCESSING DEVICE 1400

SIGNAL PROCESSOR 5400

ULTRASONIC DATA ACQUIRER 1601

IMAGE DATA ACQUIRER 1602

MAGNETIC-FIELD DATA ACQUIRER 1603

CURRENT DISTRIBUTION CALCULATOR 1606

DATA STORAGE 541

DISPLAY CONTROLLER 543

OPERATION RECEIVER 1621

CURRENT-DISTRIBUTION DISPLAY UNIT 1623

CONTROLLER 542

IMAGING CONTROLLER 1611

MAGNETIC FIELD ADJUSTER 1612

POSITION CONTROLLER 1613

TIMING CONTROLLER 1614

# FIG. 62

# FIG. 63

# FIG. 64

START DIAGNOSTIC
OPERATION

↓ S2301

PERFORM ULTRASONIC
MEASUREMENT

↓ S2302

PROCESS ULTRASONIC
DATA

↓ S2303

PLACE PALM AT
PREDETERMINED POSITION

↓ S2304

ATTACH INPUT TERMINAL
OF ELECTRICAL
STIMULATION DEVICE
TO ARM OF SUBJECT

↓ S2305

CAPTURE PALM

↓ S2306

PROCESS IMAGE DATA

↓ S2307

GIVE INSTRUCTION FOR
STARTING CURRENT
DISTRIBUTION GENERATION

↓ S2308

ADJUST MAGNETIC FIELD

↓ S2309

MAGNETIC-FIELD
DETECTION PROCESS

↓ S2310

CURRENT-DISTRIBUTION
CALCULATION PROCESS

↓ S2311

PROCESS IMAGE DATA

↓ S2312

MAKE DIAGNOSIS

↓

END DIAGNOSTIC
OPERATION

# FIG. 65

START MAGNETIC-FIELD
DETECTION PROCESS

S2401
MEASURE POSITION OF
SENSOR MODULE

S2402
START TO DETECT
MAGNETIC FIELD

S2403
APPLY ELECTRICAL
STIMULATION

S2404
STOP DETECTING MAGNETIC
FIELD

S2405
SYNCHRONOUSLY ADD
MAGNETIC-FIELD DATA

S2406
IS MEASUREMENT
POSITION TO BE CHANGED
?

Yes

S2407
MOVE HOLDER IN NERVE
RUNNING DIRECTION

No

S2408
MOVE HOLDER TO
RETRACTED POSITION

RETURN

# FIG. 66A

# FIG. 66B

# FIG. 66C

# FIG. 67

START CURRENT-DISTRIBUTION CALCULATION PROCESS

S2501
READ SYNCHRONOUSLY ADDED DATA AT EACH POSITION

S2502
$T=T_0$

S2503
READ ULTRASONIC DATA

S2504
EXECUTE INVERSE PROBLEM ESTIMATION ON POSITION AT TIME T BY USING MAGNETIC-FIELD DATA AND ULTRASONIC DATA

S2505
HAS PROCESS COMPLETED?

No

S2506
$T=T+t$

Yes

S2507
STORE CURRENT DISTRIBUTION DATA

RETURN

# FIG. 68A

CURRENT DISTRIBUTION

▦ HIGH

☐ LOW

2310

# FIG. 68B

CURRENT DISTRIBUTION

▦ HIGH

☐ LOW

2320

# FIG. 69

EP 3 646 782 A1

FIG. 70

# FIG. 71

LIGHT PROPAGATION
DIRECTION

2501_2    2501_3

2501_1

X    2511_2    2511_3

2511_1

Y    2521_2    2521_3

2521_1

NERVE RUNNING
DIRECTION

x

y

# FIG. 72A

# FIG. 72B

# FIG. 73

2800

# FIG. 74

2800

# FIG. 75

CURRENT DISTRIBUTION

▨ HIGH

☐ LOW

3000

# FIG. 76

3100

# FIG. 77

# FIG. 78

# FIG. 79

1111

# FIG. 80

EP 3 646 782 A1

FIG. 81

FIG. 82A

FIG. 82B

# FIG. 83

# FIG. 84A

# FIG. 84B

EP 3 646 782 A1

# FIG. 85

# FIG. 86

EP 3 646 782 A1

## FIG. 87

EP 3 646 782 A1

# FIG. 88

# FIG. 89

# FIG. 90

# FIG. 91

```
        ( START DIAGNOSTIC )
        (    OPERATION    )
                |
                |                        S2601
        PERFORM ULTRASONIC
           MEASUREMENT
                |
                |                        S2602
        PROCESS ULTRASONIC DATA
                |
                |                        S2603
          PLACE PALM AT
      PREDETERMINED POSITION
                |
                |                        S2604
       ATTACH INPUT TERMINAL OF
        ELECTRICAL STIMULATION
       DEVICE TO ARM OF SUBJECT
                |
                |                        S2605
           CAPTURE PALM
                |
                |                        S2606
         PROCESS IMAGE DATA
                |
                |                        S2607
        GIVE INSTRUCTION FOR
          STARTING MAGNETIC
        FIELD MEASUREMENT
                |
                |                        S2608
        ADJUST MAGNETIC FIELD
                |
                |                        S2609
         MAGNETIC-FIELD
        DETECTION PROCESS
                |
                |                        S2610
         PROCESS IMAGE DATA
                |
                |                        S2611
          MAKE DIAGNOSIS
                |
        ( END DIAGNOSTIC )
        (   OPERATION   )
```

# FIG. 92

START MAGNETIC-FIELD
DETECTION PROCESS

PROMPT SUBJECT TO MAKE
VOLUNTARY MOTION — S2701

DETECT MAGNETIC FIELD
WITH SENSOR MODULE — S2702

GUIDE APPROPRIATE
COMPRESSED STATE — S2703

CONTINUOUSLY DETECT
MAGNETIC FIELD WITH
SENSOR MODULE — S2704

ACQUIRE MUSCLE
MAGNETIC-FIELD DATA — S2705

STORE MUSCLE
MAGNETIC-FIELD DATA — S2706

RETURN

# FIG. 93

```
START DATA ANALYSIS
         │
         ▼                          S2801
READ MAGNETIC-FIELD
       DATA
         │
         ▼                          S2802
   CALCULATE Yn/Zn
         │
         ▼                          S2803
ESTIMATE DEPTH Zk AND
    POSITION Yk
         │
         ▼                          S2804
 COMPARE Y1 (Z1) WITH
      Y2 (Z2)
         │
         ▼                          S2805
   CALCULATE T1 OR T2
         │
         ▼                          S2806
     GENERATE
 INTERPOLATION DATA
         │
         ▼                          S2807
 COMPARE WAVEFORM
     PATTERN
         │
         ▼                          S2808
 CALCULATE NUMERICAL
       DATA
         │
         ▼                          S2809
    DISPLAY DATA
         │
         ▼
   END DATA ANALYSIS
```

# FIG. 94

# FIG. 95A

$\phi 1$

650_1

651

# FIG. 95B

$\phi 2$

650_2

651

# FIG. 96

653
655

# FIG. 97

650A

653

651

655

# FIG. 98

# FIG. 99

# FIG. 100

# FIG. 101

FIG. 102

EP 3 646 782 A1

# FIG. 103

# FIG. 104

# FIG. 105

EP 3 646 782 A1

# FIG. 106

# FIG. 107A

# FIG. 107B

EP 3 646 782 A1

FIG. 108B

FIG. 108A

FIG. 109

EP 3 646 782 A1

# FIG. 110A

# FIG. 110B

EP 3 646 782 A1

Europäisches
Patentamt

European
Patent Office

Office européen
des brevets

# EUROPEAN SEARCH REPORT

Application Number

EP 19 20 1512

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BROSER PHILIP J ET AL: "Optically Pumped Magnetometers for Magneto-Myography to Study the Innervation of the Hand", IEEE TRANSACTIONS ON NEURAL SYSTEMS AND REHABILITATIONENGINEERING, IEEE SERVICE CENTER, NEW YORK, NY, US, vol. 26, no. 11, 24 September 2018 (2018-09-24), pages 2226-2230, XP011698131, ISSN: 1534-4320, DOI: 10.1109/TNSRE.2018.2871947 [retrieved on 2018-11-19] | 1-6,9, 10,12,13 | INV. A61B5/04 A61B5/11 |
| Y | * figures 1-3 * * abstract * * page 2227, column 2, line 18 - page 2229, column 1, line 26 * * page 2230, column 1, line 6 - line 45 * | 7,8,11 | |
| Y | PARKEY CHARNA ET AL: "Time interleaved analog to digital converters: Tutorial 44", IEEE INSTRUMENTATION & MEASUREMENT MAGAZINE, IEEE SERVICE CENTER, PISCATAWAY, NJ, US, vol. 16, no. 6, 31 December 2013 (2013-12-31), pages 42-51, XP011536625, ISSN: 1094-6969, DOI: 10.1109/MIM.2013.6704972 [retrieved on 2014-01-07] * page 42, column 1, line 1 - page 43, column 2, line 15 * * page 45, column 2, line 4 - page 46, column 1, line 17 * | 8 | TECHNICAL FIELDS SEARCHED (IPC) A61B G01R H05K |
| Y | US 3 887 944 A (BAJOREK CHRISTOPHER H ET AL) 3 June 1975 (1975-06-03) * column 2, line 64 - column 3, line 46 * | 7 | |

-/--

The present search report has been drawn up for all claims

| Place of search | Date of completion of the search | Examiner |
|---|---|---|
| The Hague | 14 February 2020 | Oancea, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 19 20 1512

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KEVIN C MCGILL ET AL: "High-Resolution Alignment of Sampled Waveforms", IEEE TRANSACTIONS ON BIOMEDICAL ENGINEERING, IEEE SERVICE CENTER, PISCATAWAY, NJ, USA, vol. BME-19, no. 6, 30 June 1984 (1984-06-30), pages 462-468, XP011173640, ISSN: 0018-9294 * page 462, column 1, line 1 - column 2, line 33 * ----- | 8 | |
| Y | EP 1 987 769 A1 (UNIV CORP KANAZAWA I OF TECHN [JP] ET AL.) 5 November 2008 (2008-11-05) | 11 | |
| A | * figures 5-7,18 * * paragraph [0058] - paragraph [0061] * * paragraph [0094] - paragraph [0095] * ----- | 10,13 | |
| X | US 9 451 734 B2 (SEIKO EPSON CORP [JP]) 20 September 2016 (2016-09-20) | 14,15 | |
| A | * figures 1,4,12B,14 * * column 3, line 22 - line 36 * * column 3, line 54 - line 63 * * column 6, line 21 - line 31 * * column 8, line 59 - column 9, line 3 * * column 10, line 66 - column 11, line 30 * * column 11, line 13 - line 30 * ----- | 9 | TECHNICAL FIELDS SEARCHED (IPC) |
| A | WO 2018/025829 A1 (TDK CORP) 8 February 2018 (2018-02-08) & EP 3 494 879 A1 (NATIONAL UNIV CORPORATION TOKYO MEDICAL AND DENTAL UNIV [JP] ET AL.) 12 June 2019 (2019-06-12) * paragraph [0024] - paragraph [0028] * ----- | 10 | |

The present search report has been drawn up for all claims

| Place of search | Date of completion of the search | Examiner |
|---|---|---|
| The Hague | 14 February 2020 | Oancea, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

**Application Number**

EP 19 20 1512

The Search Division considers that the present European patent application does not comply with the
requirements of unity of invention and relates to several inventions or groups of inventions, namely:

1. claims: 1-8

   A biomagnetic field measurement apparatus with means for
   improving measurement accuracy

   ---

2. claims: 9-13

   A biomagnetic field measurement with means for increasing
   the magnetic scanning area

   ---

3. claims: 14, 15

   A magnetic shield box with means for reducing a stray
   magnetic field within

   ---

## EP 3 646 782 A1

### ANNEX TO THE EUROPEAN SEARCH REPORT
### ON EUROPEAN PATENT APPLICATION NO.

EP 19 20 1512

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2020

| Patent document cited in search report | | Publication date | Patent family member(s) | | Publication date |
|---|---|---|---|---|---|
| US 3887944 | A | 03-06-1975 | CA | 1056502 A | 12-06-1979 |
| | | | DE | 2422927 A1 | 23-01-1975 |
| | | | FR | 2235452 A1 | 24-01-1975 |
| | | | GB | 1428298 A | 17-03-1976 |
| | | | IT | 1022028 B | 20-03-1978 |
| | | | JP | S547448 B2 | 06-04-1979 |
| | | | JP | S5023868 A | 14-03-1975 |
| | | | US | 3887944 A | 03-06-1975 |
| EP 1987769 | A1 | 05-11-2008 | EP | 1987769 A1 | 05-11-2008 |
| | | | ES | 2728794 T3 | 28-10-2019 |
| | | | JP | 4834076 B2 | 07-12-2011 |
| | | | JP | 5137149 B2 | 06-02-2013 |
| | | | JP | 2012020143 A | 02-02-2012 |
| | | | JP | WO2007099697 A1 | 16-07-2009 |
| | | | US | 2009012384 A1 | 08-01-2009 |
| | | | WO | 2007099697 A1 | 07-09-2007 |
| US 9451734 | B2 | 20-09-2016 | NONE | | |
| WO 2018025829 | A1 | 08-02-2018 | CN | 109561848 A | 02-04-2019 |
| | | | EP | 3494879 A1 | 12-06-2019 |
| | | | JP | WO2018025829 A1 | 11-07-2019 |
| | | | US | 2019167136 A1 | 06-06-2019 |
| | | | WO | 2018025829 A1 | 08-02-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015083242 A
- WO 2015129756 A

- JP 2017152573 A

**Non-patent literature cited in the description**

- **BOTO, E. ; MEYER, S. S. ; SHAH, V. ; ALEM, O. ; KNAPPE, S. ; KRUGER, P. ; FROMHOLD, T. M. ; LIM, M. ; GLOVER, P. M. ; MORRIS, P. G.** Anew generation of magnetoencephalography: Room temperature measurements using optically-pumped magnetometers. *Neuroimage,* 2017, vol. 149, 404-414